# EUROPEAN PATENT APPLICATION

(11) **EP 3 623 009 A1**
(43) Date of publication of application: **18.03.2020**
(21) Application number: 18382658.5
(22) Date of filing: 13.09.2018
(51) Int. Cl.: A61P 35/00, A61K 31/4985, A61K 45/06

(54) **MODULATION OF TRF1 FOR BRAIN CANCER TREATMENT**

(71) Applicant: Fundación del Sector Público Estatal Centro Nacional de Investigaciones Oncológicas Carlos III (F.S.P. CNIO), 28029 Madrid (ES)
(72) Inventor: BLASCO MARHUENDA, Maria Antonia, 28029 Madrid (ES); PASTOR FERNANDEZ, Joaquin Angel, 28029 Madrid (ES); BEJARANO BOSQUE, Leire, 28029 Madrid (ES); MENDEZ PERTUZ, Marinela, 28016 Madrid (ES); MARTINEZ RODRIGUEZ, Paula, 28029 Madrid (ES); BLANCO-APARICIO, Carmen, 28029 Madrid (ES); GOMEZ-CASERO ESTEBAN, Elena, 28029 Madrid (ES); GARCIA-BECCARIA, Maria, 28020 Madrid (ES)
(74) Representative: Elzaburu S.L.P.

(57) **Abstract**

The invention provides TRF1 inhibitors and compositions comprising them for the treatment of a brain cancer, such as a glioblastoma, and particularly a glioblastoma multiforme (GBM). PI3K inhibitors can be among the TRF1 inhibitors used. The compositions can comprise more than one TRF1 inhibitor, being particularly advantageous that at least one of the inhibitors is a PI3K inhibitor and that at least a second possible TRF1 inhibitor present is selected from the group an RTK inhibitor, a MEK inhibitor, an ERK inhibitor, an HSP90 inhibitor, docetaxel and gemcitabine, because such compositions show a synergic effect. The invention also relates to a method for identifying compounds candidates to be used for treating glioblastoma or other cancers, which method is based on the identification of the compound as a TRF1 inhibitor.

SEQUENCE LISTING

<110>  FUNDACIÓN DEL SECTOR PÚBLICO ESTATAL CENTRO NACIONAL DE
       INVESTIGACIONES ONCOLÓGICAS CARLOS III (F.S.P. CNIO)

<120>  MODULATION OF TRF1 FOR BRAIN CANCER TREATMENT

<130>  EP-1928

<160>  48

<170>  PatentIn version 3.5

<210>  1
<211>  27
<212>  DNA
<213>  Artificial sequence

<220>
<223>  Forward primer for RT-qPCR for Cre gene

<400>  1
ttaatccata ttggcagaac gaaaacg                                          27


<210>  2
<211>  22
<212>  DNA
<213>  Artificial sequence

<220>
<223>  Reverse primer for RT-qPCR for Cre gene

<400>  2
caggctaagt gccttctcta ca                                               22


<210>  3
<211>  15
<212>  DNA
<213>  Artificial sequence

<220>
<223>  Taqman probe for Cre gene

<400>  3
cctgcggtgc taacc                                                       15


<210>  4
<211>  26
<212>  DNA
<213>  Artificial sequence

<220>
<223>  Forward primer for RT-qPCR for Terf1-3-MD gene; used to test for
       Trf1lox; targeted to a sequence unique to the Trf1 recombined
       allele

<400>  4
gctatacgaa gttattcgag gtcgat                                           26

<210>  5
<211>  15
<212>  DNA
<213>  Artificial sequence

<220>
<223>  Reverse primer for RT-qPCR for Terf1-3-MD gene: used to test for
       Trf1lox; targeted to a sequence unique to the Trf1 recombined
       allele

<400>  5
ggtggcggcc gaagt                                                        15


<210>  6
<211>  22
<212>  DNA
<213>  Artificial sequence

<220>
<223>  Taqman probe for Terf1-3-MD gene: used to test for Trf1lox;
       targeted to a sequence unique to the Trf1 recombined allele

<400>  6
ctctagaaag tataggaact tc                                                22


<210>  7
<211>  16
<212>  DNA
<213>  Artificial sequence

<220>
<223>  Forward primer for RT-qPCR for Terf1-3-WT gene; used to test for
       Trf1+; targeted to sequence at the 3' loxP insertion site

<400>  7
gagacggcgc gaaacc                                                       16


<210>  8
<211>  17
<212>  DNA
<213>  Artificial sequence

<220>
<223>  Reverse primer for RT-PCT for Terf1-3-WT gene: used to test for
       Trf1+; targeted to sequence at the 3' loxP insertion site

<400>  8
gcgggagcca ggacttc                                                      17


<210>  9
<211>  17
<212>  DNA
<213>  Artificial sequence

<220>
<223>  Taqman probe for Terf1-3-WT gene; used to test for Trf1+;
       targeted to sequence at the 3' loxP insertion site

<400>  9

ccgcttcctg tttgctg                                                    17


<210>  10
<211>  18
<212>  DNA
<213>  Artificial sequence

<220>
<223>  Forward primer for RT-qPCR for Tva gene: used to test the
       presence of the Tva transgene

<400>  10
cacagaggct cccactgt                                                   18


<210>  11
<211>  17
<212>  DNA
<213>  Artificial sequence

<220>
<223>  Reverse primer for RT-qPCR for Tva gene: used to test the
       presence of the Tva transgene

<400>  11
atgcggccgt gattcct                                                    17


<210>  12
<211>  16
<212>  DNA
<213>  Artificial sequence

<220>
<223>  Taqman probe for Tva gene: used to test the presence of the Tva
       transgene

<400>  12
ctggacgtgc tctgcc                                                     16


<210>  13
<211>  20
<212>  DNA
<213>  Artificial sequence

<220>
<223>  Forward primer for RT-qPCR for p16-WT gene: used to test the
       presence of the p16 allele

<400>  13
cgaggacccc actaccttct                                                 20


<210>  14
<211>  17
<212>  DNA
<213>  Artificial sequence

<220>
<223>  Reverse primer for RT-qPCR for p16-WT gene: used to test the
       presence of the p16 allele

<400>  14
ccgctcttgg gccaagt                                                    17


<210>  15
<211>  16
<212>  DNA
<213>  Artificial sequence

<220>
<223>  Taqman probe for p16-WT gene: used to test the presence of the
       p16 allele

<400>  15
caggcatcgc gcacat                                                     16


<210>  16
<211>  29
<212>  DNA
<213>  Artificial sequence

<220>
<223>  Forward primer for RT-qPCR for p16-KO: used to test the absence
       of the p16 allele

<400>  16
ctctactttt tcttctgact tttcaggtg                                       29


<210>  17
<211>  21
<212>  DNA
<213>  Artificial sequence

<220>
<223>  Reverse primer for RT-qPCR for p16-KO: used to test the absence
       of the p16 allele

<400>  17
cccctacccg gtagaattga c                                               21


<210>  18
<211>  19
<212>  DNA
<213>  Artificial sequence

<220>
<223>  Taqman probe to test the absence of the p16 allele

<400>  18
atgatgatgg gcccccgtc                                                  19


<210>  19
<211>  25
<212>  DNA
<213>  Artificial sequence

<220>
<223>  Forward primer to assess Cre mediated Trf1 deletion

<400>  19
atagtgatca aaatgtggtc ctggg                                              25


<210>  20
<211>  19
<212>  DNA
<213>  Artificial sequence

<220>
<223>  Reverse primer (SA-R1) to assess Cre mediated Trf1 deletion

<400>  20
gcttgccaaa ttgggttgg                                                     19


<210>  21
<211>  19
<212>  DNA
<213>  Artificial sequence

<220>
<223>  forward primer for qRT-PCR for mouse TRF1 gene

<400>  21
gtctctgtgc cgagccttc                                                     19


<210>  22
<211>  25
<212>  DNA
<213>  Artificial sequence

<220>
<223>  Reverse primer for qRT-PCR for mouse TRF1 gene

<400>  22
tcaattggta agctgtaagt ctgtg                                              25


<210>  23
<211>  23
<212>  DNA
<213>  Artificial sequence

<220>
<223>  Forward primer for qRT-PCR for mouse TBP1 gene

<400>  23
acccttcacc aatgactcct atg                                                23


<210>  24
<211>  21
<212>  DNA
<213>  Artificial sequence

<220>
<223>  Reverse primer for qRT-PCR for mouse TBP1 gene

<400>  24
tgactgcagc aaatcgcttg g                                                  21

<210>  25
<211>  20
<212>  DNA
<213>  Artificial sequence

<220>
<223>  forward primer for qRT-PCR for mouse TRF2 gene

<400>  25
agagccagtg gaaaaaccac                                                   20


<210>  26
<211>  20
<212>  DNA
<213>  Artificial sequence

<220>
<223>  Reverse primer for qRT-PCR for mouse TRF2 gene

<400>  26
atgatgggga tgccagatta                                                   20


<210>  27
<211>  20
<212>  DNA
<213>  Artificial sequence

<220>
<223>  forward primer for qRT-PCR for mouse POT1A gene

<400>  27
aaactatgaa gccctcccca                                                   20


<210>  28
<211>  20
<212>  DNA
<213>  Artificial sequence

<220>
<223>  Reverse primer for qRT-PCR for mouse POT1A gene

<400>  28
cgaagccaga gcagttgatt                                                   20


<210>  29
<211>  20
<212>  DNA
<213>  Artificial sequence

<220>
<223>  forward primer for qRT-PCR for mouse RAP1 gene

<400>  29
aaggaccgct acctcaagca                                                   20


<210>  30

<211>  20
<212>  DNA
<213>  Artificial sequence

<220>
<223>  Reverse primer for qRT-PCR for mouse RAP1 gene

<400>  30
tgttgtctgc ctctccattc                                                   20


<210>  31
<211>  20
<212>  DNA
<213>  Artificial sequence

<220>
<223>  Forward primer for qRT-PCR for mouse TPP1 gene

<400>  31
acttgtgtca gacggaaccc                                                   20


<210>  32
<211>  19
<212>  DNA
<213>  Artificial sequence

<220>
<223>  Reverse primer for qRT-PCR for mouse TPP1 gene

<400>  32
caaccagtca cctgtatcc                                                    19


<210>  33
<211>  21
<212>  DNA
<213>  Artificial sequence

<220>
<223>  forward primer for qRT-PCR for mouse TIN2 gene

<400>  33
tcggttgctt tgcaccagta t                                                 21


<210>  34
<211>  21
<212>  DNA
<213>  Artificial sequence

<220>
<223>  Reverse primer for qRT-PCR for mouse  TIN2

<400>  34
gcttagcttt aggcagagga c                                                 21


<210>  35
<211>  20
<212>  DNA
<213>  Artificial sequence

<220>
<223>  forward primer for qRT-PCR for human TRF1 gene

<400>  35
ttctaatgaa ggcagcggca                                                   20


<210>  36
<211>  20
<212>  DNA
<213>  Artificial sequence

<220>
<223>  Reverse primer for qRT-PCR for human TRF1 gene

<400>  36
gttgctgggt tccatgttgc                                                   20


<210>  37
<211>  23
<212>  DNA
<213>  Artificial sequence

<220>
<223>  forward primer for qRT-PCR for human a-TUB gene

<400>  37
agtgaaaaca atctaaccag aaa                                               23


<210>  38
<211>  17
<212>  DNA
<213>  Artificial sequence

<220>
<223>  Reverse primer for qRT-PCR for human a-TUB gene

<400>  38
aggcccgtga agatatg                                                      17


<210>  39
<211>  21
<212>  DNA
<213>  Artificial sequence

<220>
<223>  forward primer for qRT-PCR for human TRF2 gene

<400>  39
gaccttccag cagaagatgc t                                                 21


<210>  40
<211>  20
<212>  DNA
<213>  Artificial sequence

<220>
<223>  reverse primer for qRT-PCR for human TRF2 gene

<400>  40
gttggaggat tccgtagctg                                                   20


<210>  41
<211>  20
<212>  DNA
<213>  Artificial sequence

<220>
<223>  forward primer for qRT-PCR for human POT1 gene

<400>  41
   tgggtattgt acccctccaa                                                  20


<210>  42
<211>  21
<212>  DNA
<213>  Artificial sequence

<220>
<223>  reverse primer for qRT-PCR for human POT1 gene

<400>  42
gatgaagcat tccaaccacg g                                                 21


<210>  43
<211>  20
<212>  DNA
<213>  Artificial sequence

<220>
<223>  forward primer for qRT-PCR for human RAP1 gene

<400>  43
cggggaacca cagaataaga                                                   20


<210>  44
<211>  20
<212>  DNA
<213>  Artificial sequence

<220>
<223>  reverse primer for qRT-PCR for human RAP1 gene

<400>  44
ctcaggtgtg ggtggatcat                                                   20


<210>  45
<211>  20
<212>  DNA
<213>  Artificial sequence

<220>
<223>  forward primer for qRT-PCR for human TPP1 gene

<400>  45
cccgcagagt tctatctcca                                                   20

<210>  46
<211>  20
<212>  DNA
<213>  Artificial sequence

<220>
<223>  reverse primer for qRT-PCR for human TPP1 gene

<400>  46
ggacagtgat aggcctgcat                                                   20


<210>  47
<211>  20
<212>  DNA
<213>  Artificial sequence

<220>
<223>  forward primer for qRT-PCR for human TIN2 gene

<400>  47
ggagtttctg cgatctctgc                                                   20


<210>  48
<211>  20
<212>  DNA
<213>  Artificial sequence

<220>
<223>  reverse primer for qRT-PCR for human TIN2 gene

<400>  48
gatcccgcac tataggtcca                                                   20

## Description

### FIELD OF THE INVENTION

The invention relates to the treatment of cancer and the compounds that can be used for it. More particularly, the invention relates to TRF1 inhibitors and compositions comprising them for the treatment of a brain cancer, particularly glioblastoma, as well as to a method for identifying compounds candidates to be used for glioblastoma treatment which is based on the identification as the compound as a TRF1 inhibitor.

### BACKGROUND OF THE INVENTION

Telomeres are considered potential anticancer targets due to the fact that more than 90% of human tumors aberrantly over-express telomerase (Joseph et al. 2010; Kim et al. 1994; Shay and Bacchetti 1997), while the remaining telomerase-negative tumors activate ALT. In this regard, most studies have focused in telomerase inhibition. The best example are the studies with the telomerase inhibitor GRN163L, also called Imetelstat. However, mouse models of telomerase-based therapeutic strategies have shown some limitations, as the anti-tumorigenic effect is only achieved when telomeres reach a critically short length (Gonzalez-Suarez et al. 2000; Perera et al. 2008) and this effect is lost in the absence of the *p53* tumor suppressor gene, which is commonly mutated in cancer (Chin et al. 1999; Greenberg et al. 1999). In agreement with these findings in mice, human clinical trials with telomerase inhibitors have only shown therapeutic benefits in few myeloid malignancies but have largely failed in solid tumors (Baerlocher et al. 2015; Daniel El Fassi et al. 2015; Middleton et al. 2014; Parkhurst et al. 2004; Tefferi et al. 2015), maybe as a consequence of telomere length heterogeneity within tumors, which may hamper the effective killing of all tumor cells.

Mammalian telomeres are bound by the so-called shelterin complex formed by the telomere repeat factors 1 and 2 (TRF1 and TRF2), the TRF1-interacting factor 2 (TIN2), the Protection of Telomeres 1 (POT1), the POT1-TIN2 organizing protein TPP1 (also known as TINT1, PTOP or PIP1) and the repressor/activator protein 1 or RAP1 (De Lange 2002, 2005; Liu et al. 2004). TRF1 and TRF2 are bound to double stranded DNA repeats and interact with each other through TIN2 (Houghtaling et al. 2004; Jeffrey Zheng Sheng Ye et al. 2004).The shelterin complex has an indispensable role protecting telomeres from activating DDR and triggering apoptosis and senescence. Interestingly, not only telomerase but also shelterins are often mutated in cancer. Indeed, our group and others have identified POT1 as the first member of telomeric proteins to be mutated in several types of human cancer, both sporadic and familial, including chronic lymphocytic leukaemia (CLL) (Ramsay et al. 2013), familial melanoma (Robles-Espinoza et al. 2014; Shi et al. 2014), Li-Fraumeni like-families (LFL) with cardiac angiosarcomas (CAS) (Calvete et al. 2015), glioma (Bainbridge et al. 2015), mantle cell lymphoma (Zhang et al. 2014), and parathyroid adenoma (Newey et al. 2012).

The fact that shelterins are frequently mutated in cancer supports the notion that targeting shelterins may be a novel and promising strategy to target telomeres in cancer, which would lead to a rapid telomere dysfunction independently of telomere length.

Regarding this matter, previous works of the group of the present inventors have led to the following findings:
1. TRF1 genetic deletion *in vivo* induces a persistent DNA damage response at telomeres, which is sufficient to block cell division and induce senescence and/or apoptosis in different tissues of healthy mice (Martinez et al., 2009)
2. TRF1 is over-expressed in adult stem cell compartments as well as in pluripotent stem cells, where it is essential to maintain tissue homeostasis and pluripotency, respectively (Boué et al. 2010; Schneider et al. 2013)

Additionally, they have also previously shown that induction of telomere uncapping by *Trf1* genetic depletion or chemical inhibition can effectively block the growth of rapidly growing lung tumors, in a manner that is independent of telomere length (García-Beccaria et al. 2015). The assays described in said article were carried out in *K*-*Ras^{G12V}*-induced lung tumors, in a p53-deficient background, and showed that *Trf1* downregulation by a *Trf1*-shRNA resulted in a markedly delayed tumor onset and growth, while Trf1 chemical inhibition, in turn, effectively impair the growth of already established lung adenocarcinomas without affecting mouse and tissue viability. Chemical inhibition was carried out with two compounds, named ETP-47228 and ETP-47037 in the article, which were previously known as inhibitors of the kinase PI3K and that are included as examples in international patent applications WO2010119264 and WO2011089400, respectively. Although the authors commented that the results obtained with TRF1 chemical inhibition opened a therapeutic window for targeting TRF1 in cancer that merited further work, no assays with other types of tumors have been reported until recently.

Malignant gliomas represent the majority of all primary central nervous system (CNS) neoplasms. Based on the cell type of origin, gliomas were first categorized into 4 different groups: astrocytomas (astrocytes), ependymomas (ependymal cells), oligodendrogliomas (oligodendrocytes) and mixed gliomas. Also, the World Health Organization (WHO) classified the central nervous system tumors into four different grades (grade I to grade IV) according to the histological characteristics and tumor aggressiveness (Louis et al. 2007).

The most frequent and aggressive glioma is glioblastoma multiforme (GBM), a grade IV astrocytoma (Louis et al. 2007). GBM is well known for his highly heterogeneous nature and cancer-initiating capacities (Molina *et al.,* 2010). According to Medscape, it accounts for 12-15% of intracranial neoplasm and 50-60% of astrocytic tumors, with an incidence of 1-3 new cases per 100.000 people every year. GBM is a common but deadly brain tumor, with a very low mean survival. The current treatments for GBM consist in surgical resection combined with radiotherapy and adjuvant chemotherapy (Furnari et al. 2007; Hegi et al. 2005).. Despite all the advances in the molecular characterization of glioblastoma, the median survival has not improved in the last 50 years, remaining to be only about 14-16 months (Wen and Kesari 2008).

Poor prognosis is linked to high proliferation and cell heterogeneity, including glioma stem cells (GSCs). GSCs are not affected by the current treatments and are able to recapitulate the whole tumor, showing a strong recurrence. Thus, current treatments do not improve the overall survival in patients for that reason and, despite new advances in therapeutic interventions, GBM is still considered an incurable human tumor. This situation makes of GBM an urgent matter of cancer research, which needs new targets and further solutions for its treatment.

The present invention provides a solution to said problem.

### SUMMARY OF THE INVENTION

The present invention is based on the findings of the group of the inventors related to TRF1 and its relationship with GBM. The inventors have found that TRF1 is upregulated in mouse and human GBM, as well as in astrocytomas. They have always found that brain-specific Trf1 genetic deletion in GBM mouse models inhibited GBM initiation and progression, increasing survival. Trf1 deletion increased telomeric DNA damage and reduced proliferation and stemness. TRF1 chemical inhibitors mimicked these effects in human GBM cells and also blocked tumor sphere formation and tumor growth in xenografts from patient-derived primary GSCs. Thus, targeting telomeres throughout TRF1 inhibition has appeared as an effective therapeutic strategy for GBM and other brain tumors, such as other astrocytomas.

Based on these findings, it is herein provided an alternative therapy to target GBM which consists of TRF1 inhibition. This means an alternative solution for the treatment of a brain tumor which is considered incurable and that, as previously commented, is well known for his highly heterogeneous nature and cancer-initiating capacities.

Thus, one aspect of the present invention is a composition which comprises at least a TRF1 inhibitor for use in the treatment or prevention of a brain tumor, which composition, preferably, also comprises one or more pharmaceutically acceptable excipients, diluents or vehicles. More particularly, the brain tumor is a glioblastoma multiforme tumor. In any of the embodiments, the composition may additionally comprise one or more pharmaceutically acceptable excipients. Preferably, the composition comprises at least a first and a second TRF1 inhibitor and at least of the first and the second TRF1 inhibitor is a TRF1 inhibitor which decreases TRF1 protein levels. The second TRF1 inhibitor can be selected from the group of: a RTK inhibitor, a MEK inhibitor, an ERK inhibitor, an mTOR inhibitor, a CDK inhibitor, an HSP90 inhibitor, a PLK inhibitor, an Aurora inhibitor, docetaxel and gemcitabine. It is particularly preferred that the first TRF1 inhibitor is selected from the group of: a) a compound which acts through the Akt/PI3K pathway, and b) a compound of Formula I:

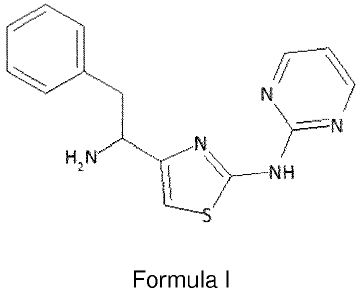

The TRF1 inhibitor which acts through the Akt/PI3K pathway can be selected of the groups of compounds claimed in international applications WO2010119264 and WO2011089400, with a particular preference for the compounds named ETP-47228 and ETP-47037. With regard to the second TRF1, a possible embodiment is that it is selected from the group of the compounds that have been found to be TRF1 inhibitors by the assays described herein, that is: Geldanamycin, Docetaxel, Gemcitabine, Alisertib, Dasatinib, GSK461364, KU-0063794, SCH772984, Selumetinib, Flavopiridol. The compositions for use in the treatment of glioblastoma multiforme wherein the first TRF1 inhibitor is ETP-47037 and the second TRF1 inhibitor is gemcitabine are particularly preferred.

Another possible embodiment for the compositions of the present invention is a composition which comprises at least a TRF1 inhibitor for use in the treatment or prevention of a brain tumor, preferably glioblastoma multiforme, which additionally comprises another anti-tumoral compound, preferably a compound that is used in the treatment of glioblastoma multiforme, which compound can be temozolomide. The TRF1 inhibitor, as above, can be selected from the group of a) a compound which acts through the Akt/PI3K pathway, such as the compounds claimed in international applications WO2010119264 and WO2011089400, and b) a compound of Formula I.

Another aspect of the invention is a compound which is a TRF1 inhibitor for use in the treatment or prevention of a brain tumor. Preferably, the compound which is a TRF1 inhibitor, and/or the composition which comprises such compound, is for use in blocking, diminishing or slowing the progression or the recurrence of a glioblastoma tumor.

Yet another aspect of the invention is a method for identifying a compound as a compound for use in the treatment of glioblastoma, which comprises a step wherein it is determined that the compound inhibits or decreases TRF1 activity.

### BRIEF DESCRIPTION OF THE FIGURES

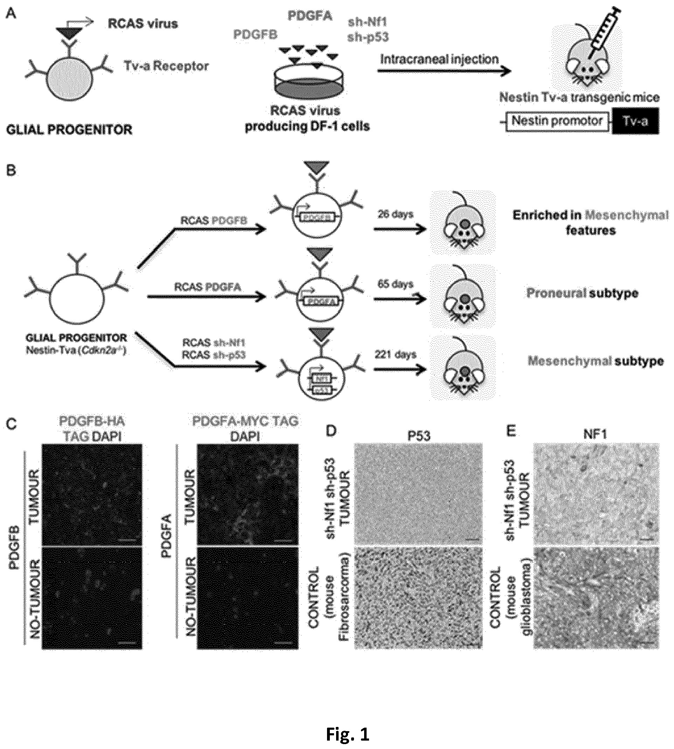

**Fig. 1****. Modeling GBM using the RCAS-Tva System.** (**A**) RCAS-Tva is a gene transfer system in which RCAS virus will specifically target Tva expressing cells. In our system Tva will be expressed under the promotor of Nestin. RCAS virus-producing DF-1 cells are intracraneally injected into Nestin-Tva mice. (**B**) GBM is induced after overexpression of PDGFB/PDGFA or knockdown of Nf1 and p53 in Nestin positive cells. (**C**) Immunofluorescence against HA and MYC tag to confirm overexpression of PDGFB and PDGFA respectively. Scale bar 20 µm. (**D**) IHC against p53 to confirm knockdown in the tumors. A mouse fibrosarcoma expressing p53 is used as positive control. Scale bar 200 µm (**E**) IHC against Nf1 to confirm knockdown in the tumors. A mouse glioblastoma expressing Nf1 is used as positive control. Scale bar 50 µm.

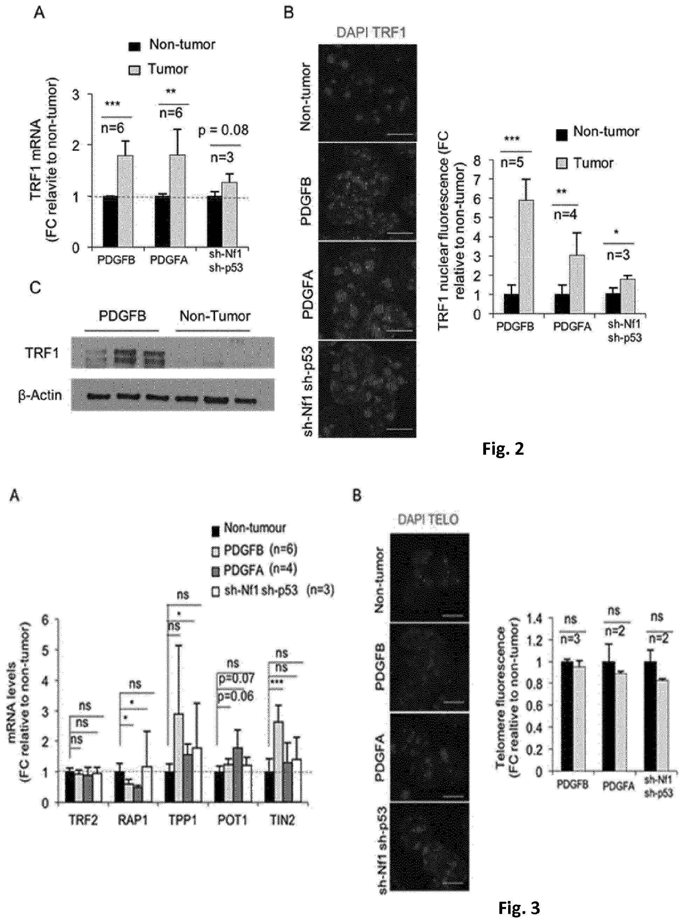

**Fig. 2****. TRF1 is overexpressed in different mouse GBM subtypes (A)** *Trf1* expression levels measured by RT-qPCR in the different GBM subtypes compared to non-tumor areas. **(B)** Quantification of nuclear TRF1 fluorescence in tumor and non-tumor areas (right) and representative images (left). Scale bar 5 µm. **(C)** Western blot for TRF1 protein levels in PDGFB tumors and non-tumor areas. Data are represented as mean ± SD. n represents the number of mice. Statistical analysis: unpaired *t-test.*
**Fig. 3****. Shelterin and telomere quantification in the different GBM subtypes.** (**A**) TRF2, RAP1, TPP1, POT1 AND TIN2 mRNA expression levels measured by RT-qPCR in the different GBM subtypes compared to non-tumor areas. **(B)** Telomere Q-FISH analysis of tumor and non-tumor areas. Data are represented as mean ± SD. n represents the number of mice. Statistical analysis: unpaired *t-test.*

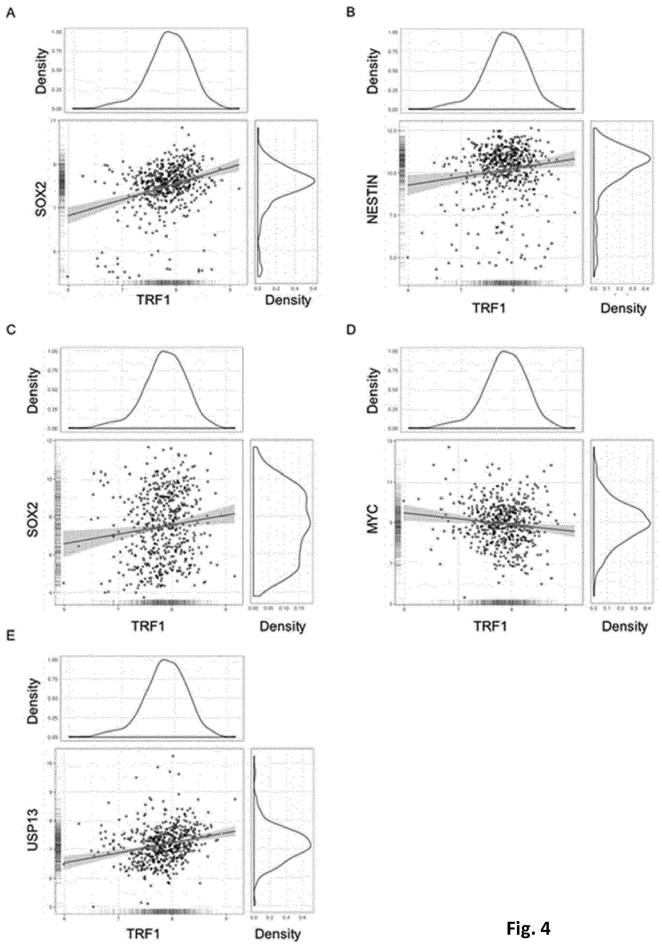

**Fig. 4****. High TRF1 expression correlates with GBM stem cell markers. (A)** Correlation between TRF1 and SOX2 analyzed by the Gliovis platform. p<0.01 **(B)** Correlation between TRF1 and NESTIN analyzed by the Gliovis platform. p<0.01 **(C)** Correlation between TRF1 and CD133 analyzed by the Gliovis platform. p=0.01 **(D)** Correlation between TRF1 and MYC analyzed by the Gliovis platform. p<0.01 **(E)** Correlation between TRF1 and USP13 analyzed by the Gliovis platform. p<0.01. Correlation method: Pearson's product-moment correlation.

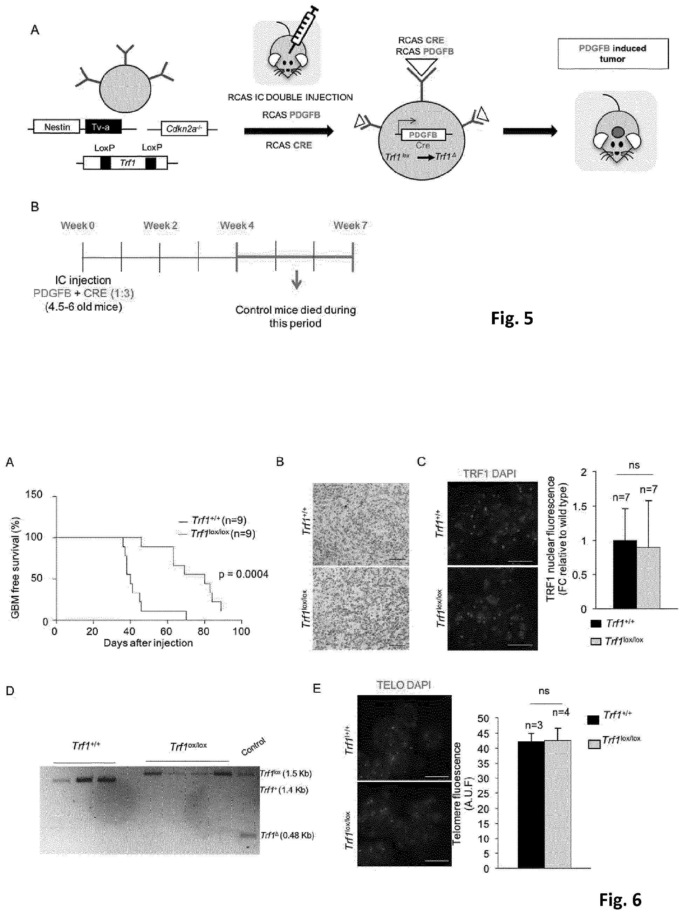

**Fig. 5****. Experimental procedure. (A)** GBM is induced by PDGFB overexpression simultaneously with Cre expression to delete the *Trf1^{lox}* allele. These events happen specifically in Nestin-positive cells. **(B)** PDGFB and Cre producing cells are injected in 1:3 ratio. Mice start dying from GBM tumors at week 4 after induction.
**Fig. 6****. *Trf1* deletion impairs tumor initiation in PDGFB induced GBM. (A)** Survival curves of mice with the indicated phenotypes. **(B)** Representative image of *Trf1*^{*+*/*+*} and *Trf1*^{*lox*/*lox*} tumor histology. Scale bar 50 µm. **(C)** TRF1 protein nuclear intensity of *Trf1*^{*+*/*+*} and *Trf1*^{/*ox*/*lox*} tumors (right panel) as determined by TRF1 immunofluorescence. Representative images (left panel). Scale bar 5 µm **(D)** Analysis of *Trf1* excision by PCR. **(E)** Telomere Q-FISH analysis of *Trf1*^{*+*/*+*} and *Trf1*^{*lox*/*lox*} tumors. Scale bar 50 µm. Data are represented as mean ± SD. n represents the number of mice. Statistical analysis: *Log-rank test* and unpaired *t-test.*

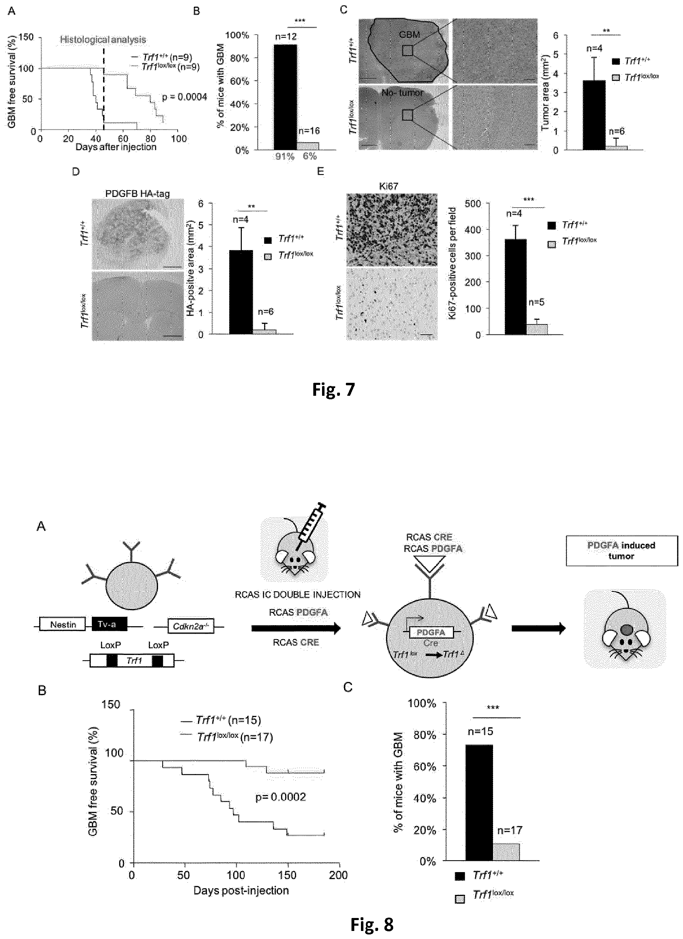

**Fig. 7****. Analysis of the tumors at the same time-point (A)** Survival curves of mice with the indicated phenotypes. Histological analysis is performed at day 45 after tumor induction. **(B)** Percentage of *Trf1*^{*+*/*+*} and *Trf1*^{*lox*/*lox*} mice affected by GBM at 45 days after tumor induction. **(C)** Quantification of tumor area by H&E in *Trf1*^{*+*/*+*} and *Trf1*^{*lox*/*lox*} mice (right panel). Representative images of H&E (left panel). **(D)** Quantification of HA-tag positive areas as a PDGFB expression in *Trf1*^{*+*/*+*} and *Trf1*^{*lox*/*lox*} mice (right panel). Representative images of HA-tag immunohistochemistry images (left panel). **(E)** Number of Ki67-positive cells per field in *Trf1*^{*+*/*+*} and *Trf1*^{*lox*/*lox*} mice (right panel). Representative images of Ki67 immunohistochemistry (left panel). Data are represented as mean ± SD. n represents the number of mice. Statistical analysis: *Log-rank test, Chi-Square* and unpaired *t-test.*
**Fig. 8****. *Trf1* deletion impairs tumor initiation in PDGFA induced GBM (A)** Tumors are induced by PDGFA overexpression simultaneously with Cre expression to delete the *Trf1^{lox}* allele. These events happen specifically in Nestin-positive cells. (**B)** Survival curves of the indicated genotypes. **(C)** Percentage of *Trf1*^{*+*/*+*} and *Trf1*^{*lox*/*lox*} mice affected with GBM 150 days after tumor induction. n represents the number of mice. Statistical analysis: *Log-rank test* and *Chi-Square.*

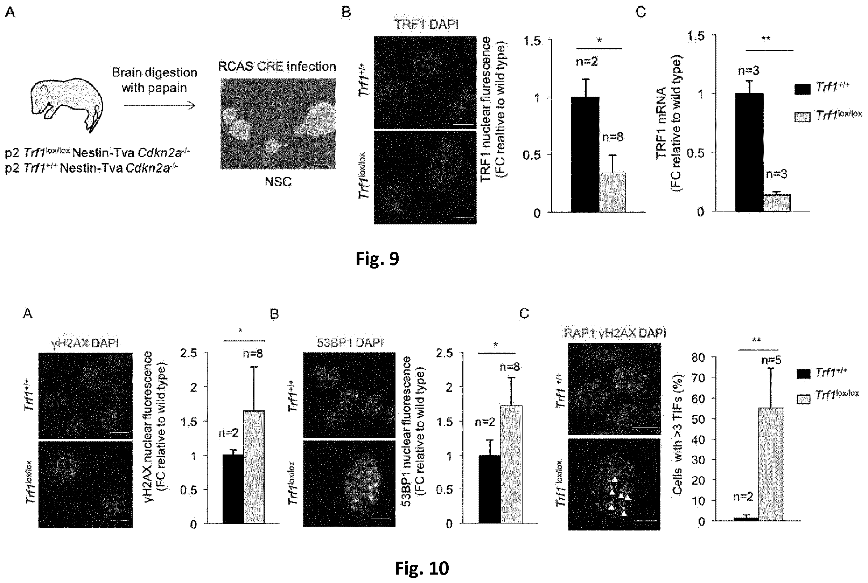

**Fig. 9****. *Trf1* deletion in mouse-derived NSCs (A)** NSCs are obtained by brain digestion with papain in 2 days old pups from the indicated genotypes. *Trf1* allele is depicted by Cre-mediated excision. **(B)** TRF1 nuclear intensity as determined by TRF1 protein immunofluorescence in *Trf1*^{*+*/*+*} and *Trf1*^{*lox*/*lox*} NSCs (right panel). Representative images of TRF1 immunofluorescence (left panel). Scale bar 5 µm (up) **(C)** *Trf1* mRNA expression levels measured by RT-qPCR in *Trf1*^{*+*/*+*} and *Trf1*^{*lox*/*lox*} NSCs. Data are represented as mean ± SD. n represents independent NSC lines. Statistical analysis: unpaired *t-test.*
**Fig. 10****. *Trf1* abrogation induces DNA damage in NSCs. (A)** γH2AX nuclear intensity in *Trf1*^{*+*/*+*} and *Trf1*^{*lox*/*lox*} NSCs (right panel). Representative images of γH2AX immunofluorescence (left panel). **(B)** 53BP1 nuclear intensity in *Trf1*^{*+*/*+*} and *Trf1*^{*lox*/*lox*} NSCs (right panel). Representative images of 53BP1 immunofluorescence (left panel). **(C)** Percentage of cells presenting 3 or more γH2AX and RAP1 colocalizing foci (TIFs) (right panel). Representative images of γH2AX and RAP1 double immunofluorescence (left panel). White arrowheads: co-localization of γH2AX and RAP1. Data are represented as mean ± SD. *n* represents independent NSC lines. Statistical analysis: unpaired *t-test.* Scale bar 5 µm.

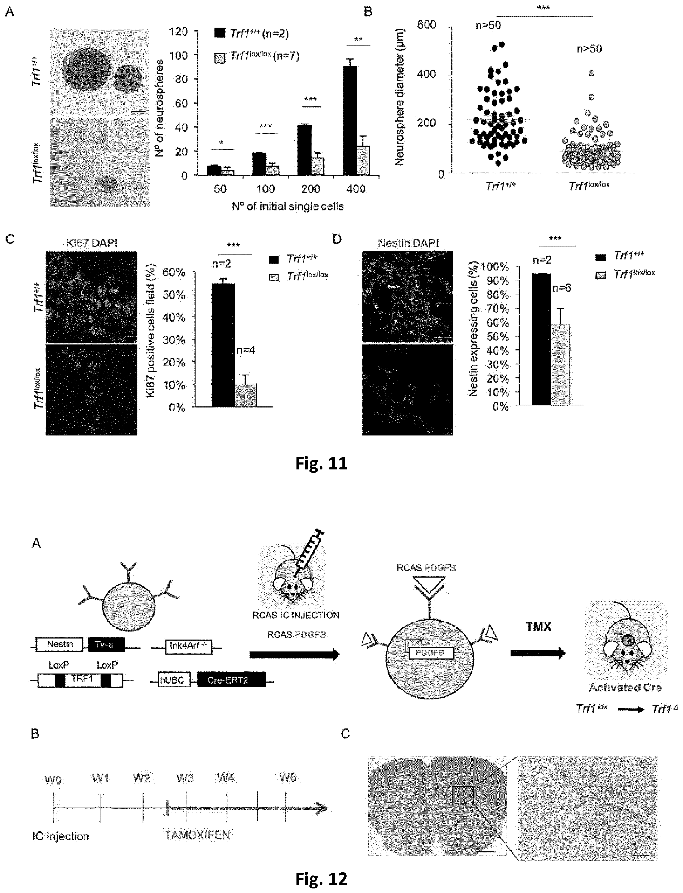

**Fig. 11****. *Trf1* deletion reduces sternness and proliferation in NSCs. (A)** Quantification of the number of neurospheres formed by *Trf1*^{*+*/*+*} and *Trf1*^{*lox*/*lox*} NSCs (right panel). Representative images of the neurospheres, bright field (left panel). Scale bar 100 µm **(B)** Quantification of the neurosphere diameter formed by *Trf1*^{*+*/*+*} and *Trf1*^{*lox*/*lox*} NSCs. **(C)** Percentage of cells positive for Ki67 (right panel). Representative image of Ki67 immunofluorescence (left panel). Scale bar 20 µm **(D)** Percentage of cells positive for Nestin (right panel). Representative image of Nestin immunofluorescence (left panel). Scale bar 50 µm Data are represented as mean ± SD with the exception of 10B which is represented as mean ± SEM. *n* represents independent NSC lines with the exception of 10B in which *n* represents the number of neurospheres. Statistical analysis: unpaired t-*test.*
**Fig. 12****. Experimental procedure. (A)** Tumors are induced by PDGFB overexpression, and *Trf1^{lox}* allele is generated by tamoxifen treatment after the tumors are formed. **(B)** PDGFB producing cells are injected to induce the tumors. 2.5 weeks after tumor induction mice are treated with tamoxifen. Mice start dying from GBM die at week 4 after treatment. **(C)** Representative image of tumor histology 2.5 weeks after tumor induction with PDGFB. Scale bar 500 µm (left) and 100 µm (right).

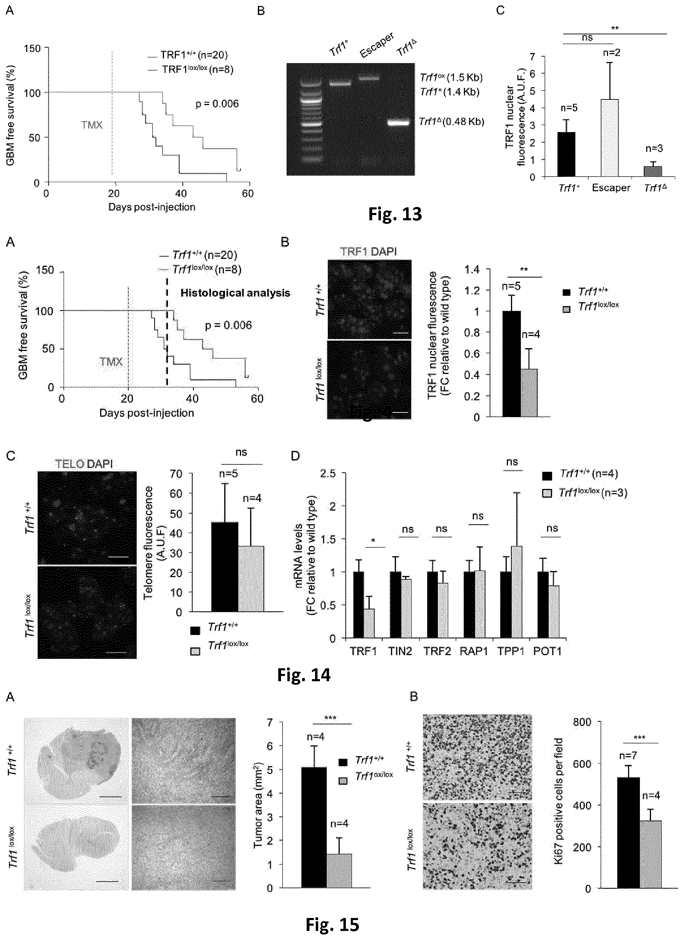

**Fig. 13****. *Trf1* deletion delays tumor progression in PDGFB driven GBM. (A)** Survival curve analysis of the indicated genotypes. **(B)** Analysis of *Trf1* excision by PCR. **(C)** TRF1 nuclear intensity of *Trf1⁺, Trf1^{lox}* tumors and escapers. Data are represented as mean ± SD. *n* represents the number of mice. Statistical analysis: *Log-rank test* and unpaired *t-test.*
**Fig. 14****. Telomere length and shelterin analysis in** *Trf1* **deleted tumors (A)** Histological analysis is performed 32 days after tumor induction. **(B)** TRF1 nuclear intensity of *Trf1*^{*+*/*+*} and *Trf1*^{/*ox*/*lox*} tumors as determined by immunofluorescence (right panel). Representative images of TRF1 immunofluorescence (left panel). Scale bar 5 µm. **(C)** Telomere Q-FISH analysis of *Trf1*^{*+*/*+*} and *Trf1*^{*lox*/*lox*} tumors. Representative images (left panel). Scale bar 5 µm **(D)** TRF2, RAP1, POT1, TIN2 and TPP1 mRNA levels by RT-qPCR in *Trf1*^{+/+}, *Trf1*^{lox/lox} tumors. Data are represented as mean ± SD. *n* represents the number of mice. Statistical analysis: *Log-rank test* and unpaired *t-test.*
**Fig. 15****.** *Trf1* **deletion causes a reduction in the tumor area and proliferation (A)** Quantification of tumor areas by H&E in *Trf1*^{*+*/*+*} and *Trf1*^{*lox*/*lox*} tumors 32 days after tumor induction (right panel). Representative images of H&E (left panel). Scale bar 1 mm (left) and 100 µm (right). **(B)** Number of Ki67-positive cells per field in *Trf1*^{*+*/*+*} and *Trf1*^{*lox*/*lox*}tumors 32 days after tumor induction (right panel). Representative images of Ki67 immunohistochemistry (left panel). Scale bar 100 µm. Data are represented as mean ± SD. *n* represents the number of mice. Statistical analysis: unpaired *t-test.*

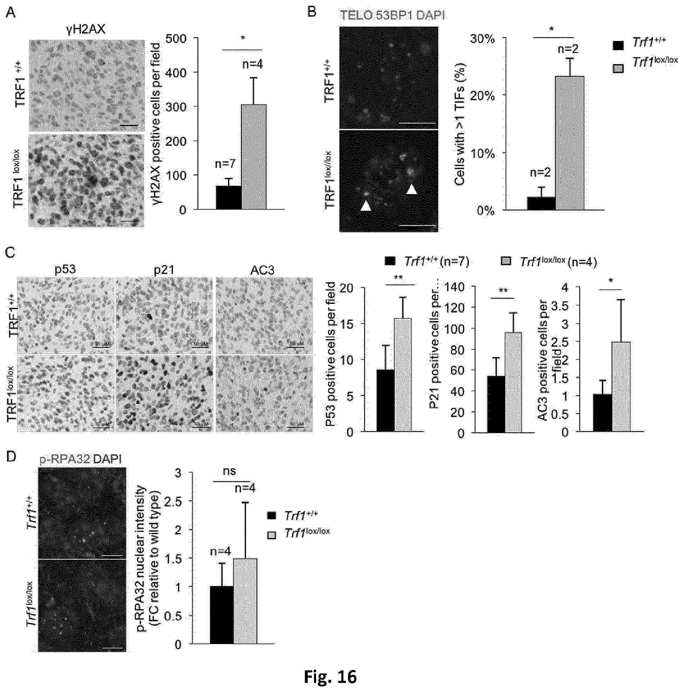

**Fig. 16****. *Trf1* deficient tumors show an increased DDR (A)** Number of γH2AX - positive cells per field in *Trf1*^{*+*/*+*} and *Trf1*^{*lox*/*lox*} tumors 32 days after tumor induction (right panel). Representative images of γH2AX immunohistochemistry (left panel). Scale bar 20 µm. **(B)** Percentage of cells presenting 1 or more 53BP1 and telomere colocalizing foci (TIFs) (right panel). Representative images (left panel). White arrowheads: colocalization of 53BP1 and telomeres. Scale bar 5 µm. **(C)** Representative images (left) and percentage (right) of p53, p21 and AC3-positive cells. Scale bar 50 µm. **(D)** p-RPA32 nuclear intensity of *Trf1*^{*+*/*+*} and *Trf1*^{*lox*/*lox*} tumors. Representative images (left panel). Scale bar 5 µm. Data are represented as mean ± SD. *n* represents the number of mice. Statistical analysis: unpaired *t-test.*

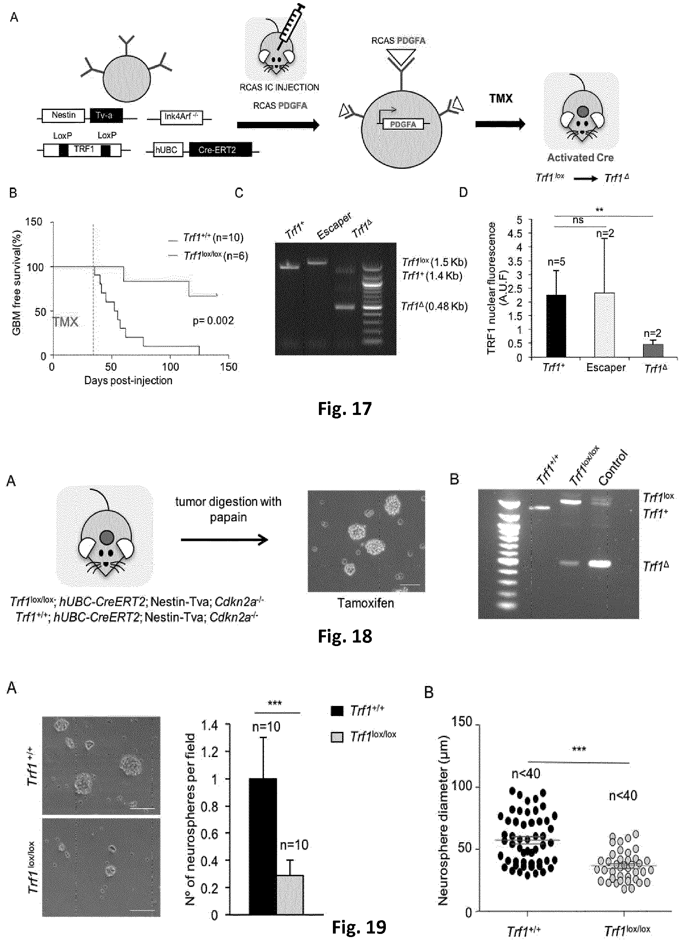

**Fig. 17****. *Trf1* deletion delays tumor progression in PDGFA driven GBM. (A)** Tumors are induced by PDGFA overexpression, and *Trf1^{lox}* allele is depleted by tamoxifen treatment after the tumors are formed. **(B)** Survival curves of the indicated genotypes. **(C)** Analysis of *Trf1* excision by PCR. **(D)** TRF1 nuclear intensity of *Trf1⁺*, *Trf1^{lox}* tumors and escapers. Data are represented as mean ± SD. *n* represents the number of mice. Statistical analysis: *Log-rank test* and unpaired *t-test.*
**Fig. 18****. Experimental procedure to study the effects of *Trf1* abrogation specifically in GSCs. (A)** *Trf1*^{*+*/*+*} and *Trf1*^{*lox*/*lox*} GSCs are obtained by tumor digestion with papain. The *Trf1^{lox}* allele is generated by tamoxifen treatment. Scale bar 100 µm (**B)** Analysis of *Trf1* excision by PCR.
**Fig. 19****. *Trf1* deletion reduces sternness in GSCs. (A)** Quantification of number of neurospheres formed by *Trf1*^{*+*/*+*} and *Trf1*^{*lox*/*lox*} GSCs (right panel). Representative images of the neurospheres, bright field (left panel). Scale bar 100 µm **(B)** Quantification of the neurosphere diameter formed by *Trf1*^{*+*/*+*} and *Trf1*^{*lox*/*lox*} GSCs. Data are represented as mean ± SD in 19A and mean ± SEM in 19B. n represents number of fields in 19A and the number of spheres in 19B. Statistical analysis: unpaired *t-test.*

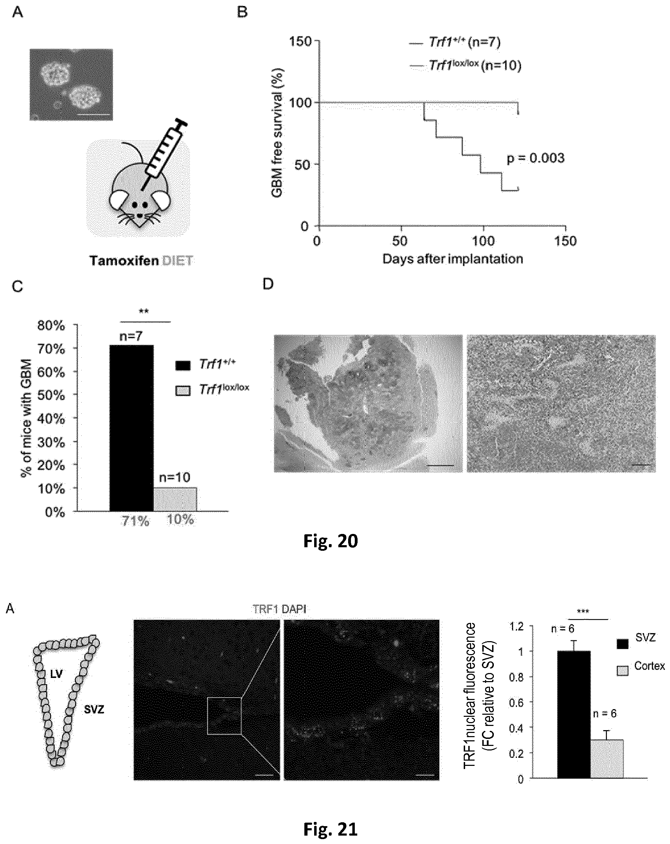

**Fig. 20****. *Trf1* deletion reduces the tumorigenic potential in GSCs. (A)** GSCs are orthotopically injected in syngeneic mice fed with tamoxifen. Scale bar 100 µm **(B)** Survival curves of mice injected with *Trf1*^{*+*/*+*} and *Trf1*^{*lox*/*lox*} GSCs. **(C)** Percentage of mice affected by the injection of *Trf1*^{*+*/*+*} and *Trf1*^{/*ox*/*lox*} GSCs. **(D)** Representative image of *Trf1*^{*+*/*+*} tumor histology. Scale bar 500 µm (left) and 100 µm (right). Data are represented as mean ± SD. n represents the number of mice.. Statistical analysis: unpaired *t-test* and *Log-rank test.*
**Fig. 21****. Trf1 is upregulated in the subventricular zone. (A)** Representative images (left) and quantification (right) of TRF1 nuclear fluorescence in the SVZ compared with the surrounding cerebral cortex. Scale bar 50 µm (left) and 10 µm (right).

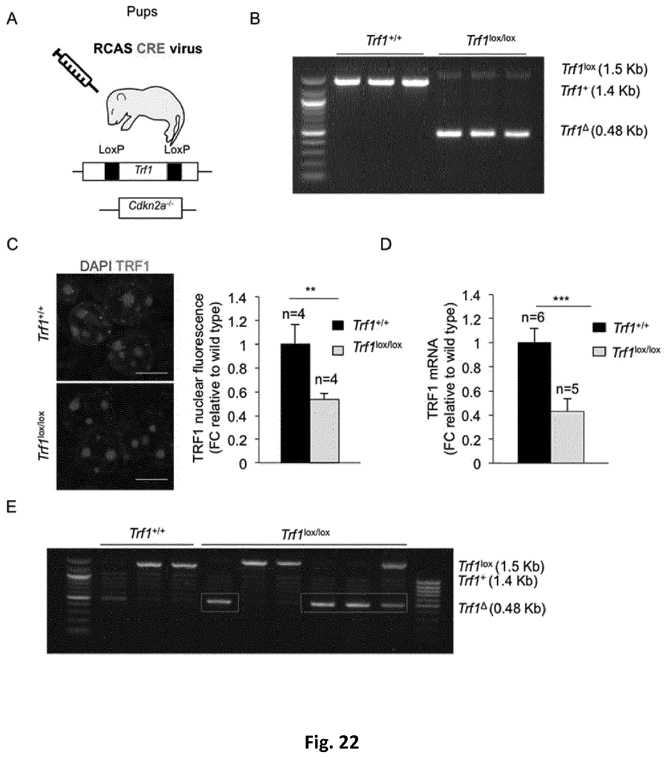

**Fig. 22****. *Trf1* brain-specific deletion in healthy pups (A)** *Trf1* deletion is induced by Cre-mediated recombination in 2 day-old newborns. (**B)** Analysis of *Trf1* excision by PCR. **(C)** TRF1 nuclear intensity of *Trf1*^{*+*/*+*} and *Trf1*^{*lox*/*lox*} brains (right panel). Representative images of TRF1 immunofluorescence (left panel). Scale bar 5 µm **(D)** TRF1 expression levels measured by RT-qPCR in *Trf1*^{*+*/*+*} and *Trf1*^{*lox*/*lox*} brains. **(E)** Analysis of *Trf1* excision by PCR in adults. Data are represented as mean ± SD. n represents the number of mice. Statistical analysis: unpaired *t-test.*

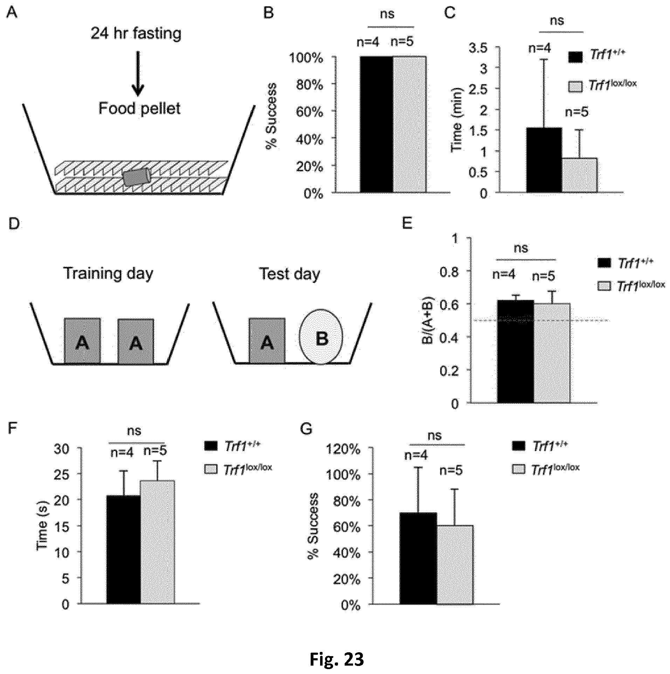

**Fig. 23****. *Trf1* brain specific deletion in healthy mice does not compromise brain function. (A**) After 24 h fasting, mice are moved to a cage with a buried food pellet and both the success and the time to find the pellet are measured. **(B, C)** Percentage of success finding the pellet (B) and time needed to find the pellet (C). **(D)** Mice were trained in a box with two identical objects (A). The test day one of the object was changed (B). **(E)** Quantification of time spent with B/(A+B). **(F)** Time spend in the rotarod. **(G)** Percentage success in the tightrope. Data are represented as mean ± SD. n represents the number of mice. Statistical analysis: unpaired *t-test.*

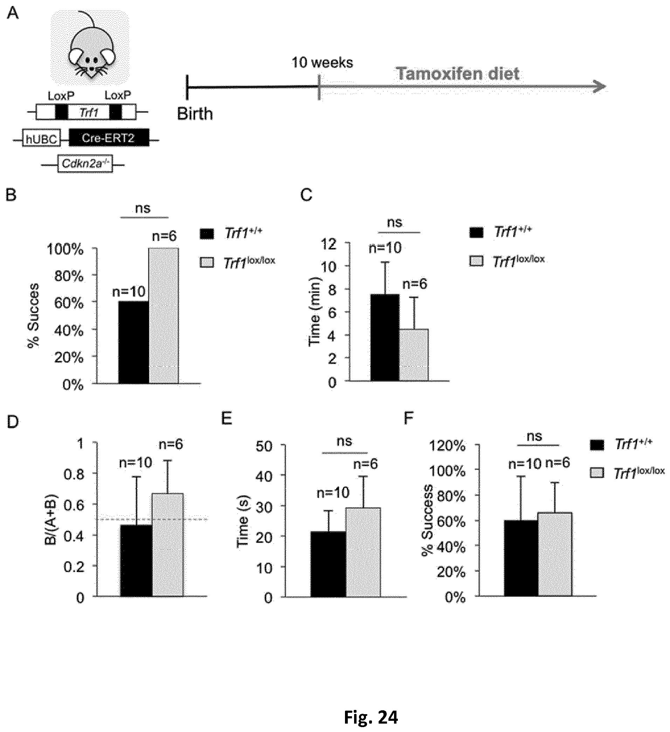

**Fig. 24****. *Trf1* whole-body deletion in healthy mice does not compromise brain function. (A**) *Trf1* whole body deletion is induced by tamoxifen diet from the age of 10 weeks. **(B, C)** Percentage of success finding the pellet (B) and time needed to find the pellet (C). **(D)** Quantification of time spent with B/(A+B). **(E)** Time spend in the rotarod. **(F)** Percentage success in the tightrope. Data are represented as mean ± SD. n represents number of mice. Statistical analysis: unpaired *t-test.*

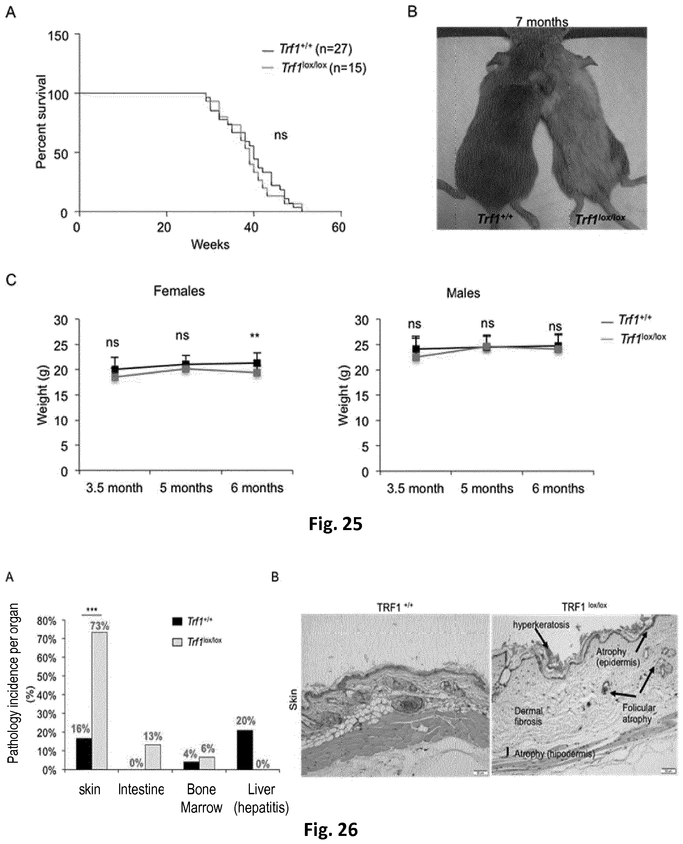

**Fig. 25****. *Trf1* whole-body deletion does not compromise mice viability in *Cdkn2a* deficient background. (A**) Survival curves of the indicated genotypes **(B)** Representative pictures of *Trf1*^{*+*/*+*} and *Trf1*^{*lox*/*lox*} mice at 5 months of age. **(C)** Weight follow-up in mice and females of the indicated genotypes. Data are represented as mean ± SD. n represents number of mice. Statistical analysis: *Log-rank test* and unpaired *t-test.*
**Fig. 26****. *Trf1* deficient mice show a higher incidence of skin pathologies (A)** Pathology incidence in the different organs of the indicated genotypes **(B)** Representative pictures of *Trf1*^{*+*/*+*} and *Trf1*^{*lox*/*lox*} skin at the human end-point. Arrows indicate the main pathologies. Scale bar 50 µm. n represents number of mice. Statistical analysis: *Chi-square.*

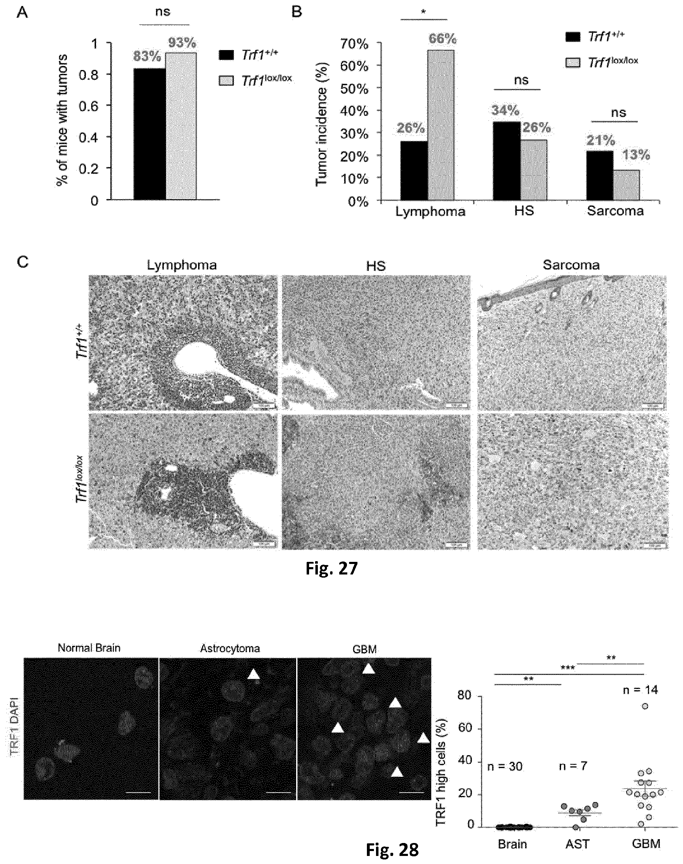

**Fig. 27****. Tumor incidence upon *Trf1* deletion. (A)** Percentage of mice with tumors in *Trf1*^{*+*/*+*} and *Trf1*^{/*ox*/*lox*} mice. **(B)** Incidence of lymphomas, histiocytic sarcomas and sarcomas in the indicated genotypes. **(C)** Representative images of the tumors. Scale bar 100 µm. n represents number of mice. Statistical analysis: *Chi-square.*
**Fig. 28****. TRF1 is upregulated in human GBM tissue. (A**) Representative images (left) and quantification (right) of percentage of cells with high TRF1 expression determined by immunofluorescence. Scale bar 10 µm. Data are represented as mean ± SEM. n represents number of independent human samples. Statistical analysis: unpaired *t-test.*

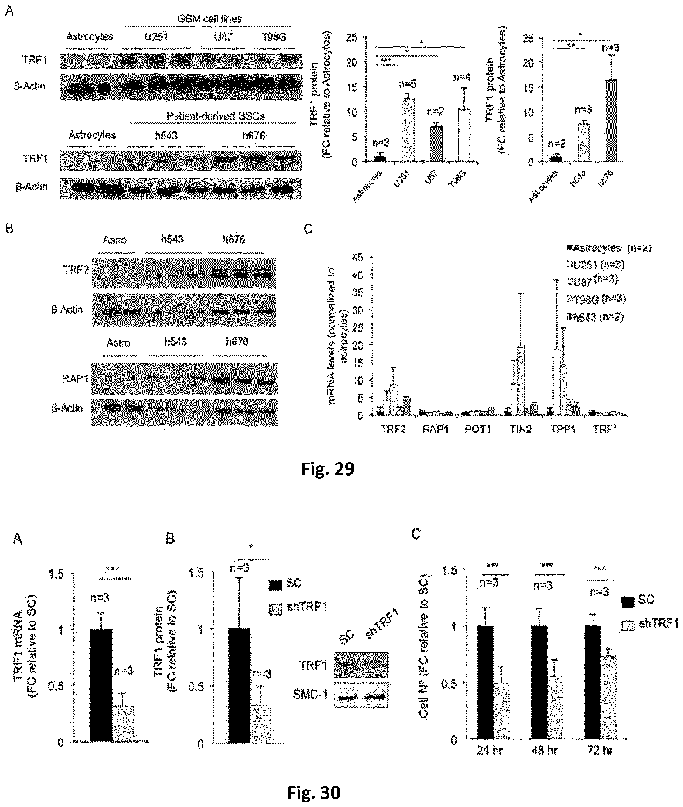

**Fig. 29****. Shelterin quantification in human GBM cells. (A)** Western blot images (left) and quantification (right) of TRF1 protein levels in the indicated cells. **(B)** TRF2 and RAP1 protein levels in the indicated cells. Western blot images (left). **(C)** TRF2, RAP1, POT1, TIN2, TPP1 and TRF1 mRNA levels by RT-qPCR in the indicated cell types. All the cases are not significant. Data are represented as mean ± SEM. n represents number of independent human samples. Statistical analysis: unpaired *t-test.*
**Fig. 30****. TRF1 downregulation in the U251 cell line. (A)** *Trf1* expression levels measured by RT-qPCR in control- and *Trf1* knockdown-U251 cells. **(B)** TRF1 protein levels in control- and *Trf1* knockdown-U251 cells. Western blot images (left panel). (**B)** Cell number assessed at 24 and 48h in control- and *Trf1* knockdown-U251 cells. Data are represented as mean ± SD. n represents number of biological replicates. Statistical analysis: unpaired *t-test.*

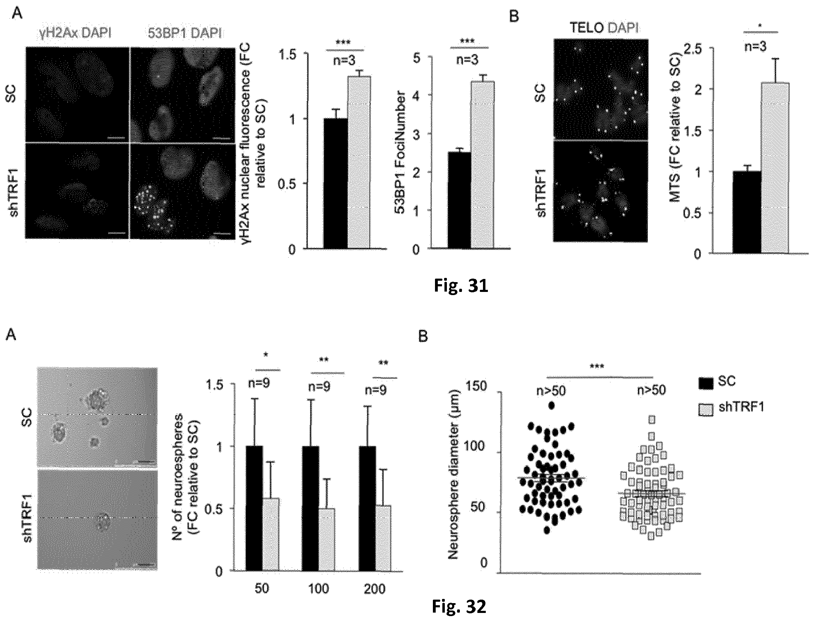

**Fig. 31****. TRF1 downregulation induces DNA damage in the U251 cell line (A**) γH2AX mean intensity and 53BP1 foci number in control- and *Trf1* knockdown-U251 cells. Representative images of 53BP1 and γH2AX immunofluorescence (left panel). Scale bar 5 µm. **(B)** Quantification of Multitelomeric signals in metaphases in control- and *Trf1* knockdown-U251 cells. Representative images of the qFISH in the metaphases (left panel). Scale bar 5 µm. Data are represented as mean ± SD. n represents number of biological replicates. Statistical analysis: unpaired *t-test.*
**Fig. 32****. TRF1 downregulation reduces sternness in the U251 cell line. (A)** Quantification of the number of neurospheres in control- and *Trf1* knockdown-U251 cells. Representative images of the neurospheres, bright field (left). Scale bar 100 µm. **(B)** Quantification of the neurosphere diameter formed by control- and *Trf1* knockdown-U251 cells. Data are represented as mean ± SD in 32A and mean ± SEM in 32B. n represents biological replicates in 32A and the number of spheres in 32B. Statistical analysis: unpaired *t-test.*

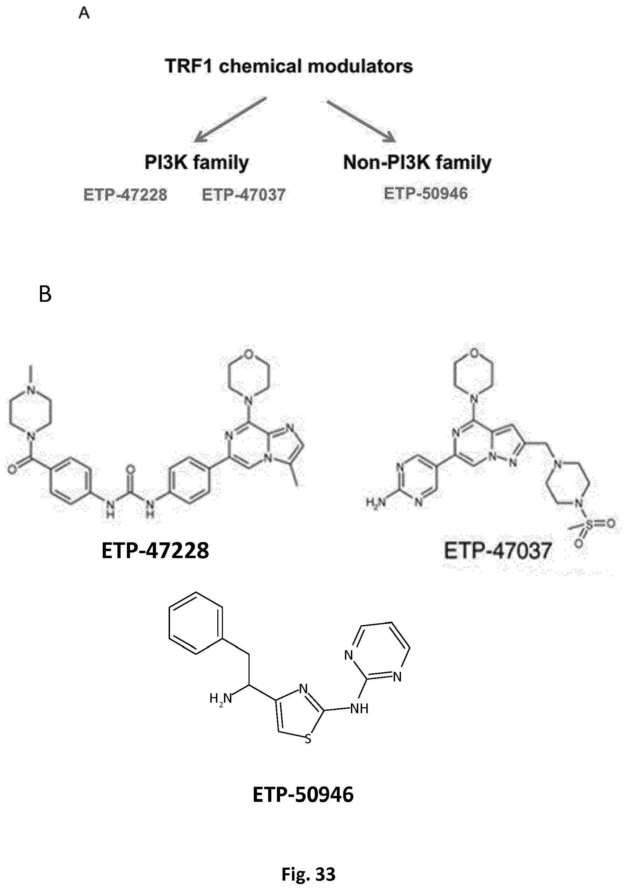

**Fig. 33****. TRF1 chemical modulators. (A)** Representation of the main hits obtained in the screening (B) Structure of the chemical compounds ETP-47228, ETP-47037 and ETP-50946.

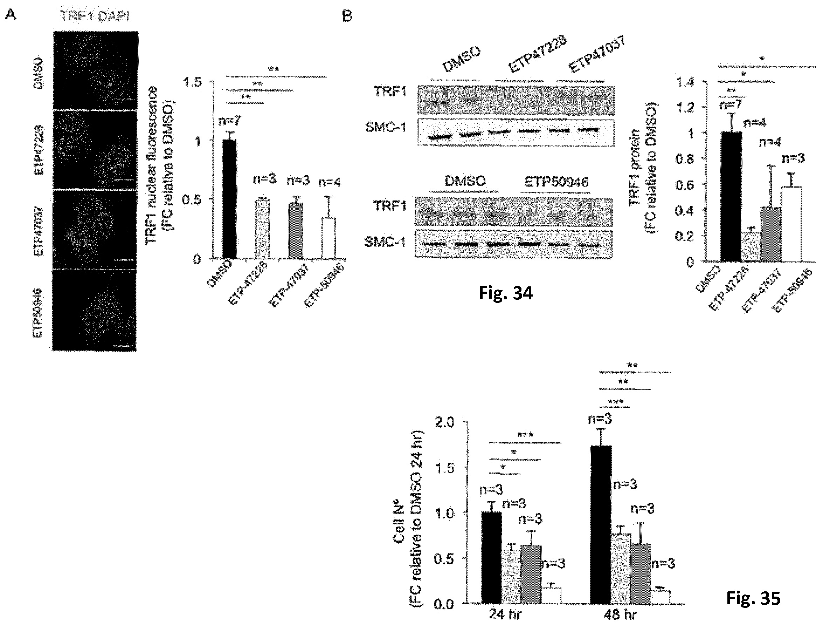

**Fig. 34****. TRF1 protein downregulation after treatment with the compounds. (A)** Representative images (left) and quantification (right) of TRF1 nuclear fluorescence of U251 cells treated with the indicated compounds. Scale bar 5 µm. **(B)** Western blot images (left) and TRF1 protein levels (right) of U251 cells treated with the indicated compounds. Data are represented as mean ± SD. n represents number of biological replicates. Statistical analysis: unpaired *t-test.*
**Fig. 35****. TRF1 chemical modulators reduce proliferation in the U251 GBM cell line. (A)** Cell number assessed at 24 and 48h of U251 cells treated with ETP-47228, ETP-47037, ETP-50946 or DMSO. Data are represented as mean ± SD. n represents number of biological replicates. Statistical analysis: unpaired *t-test*

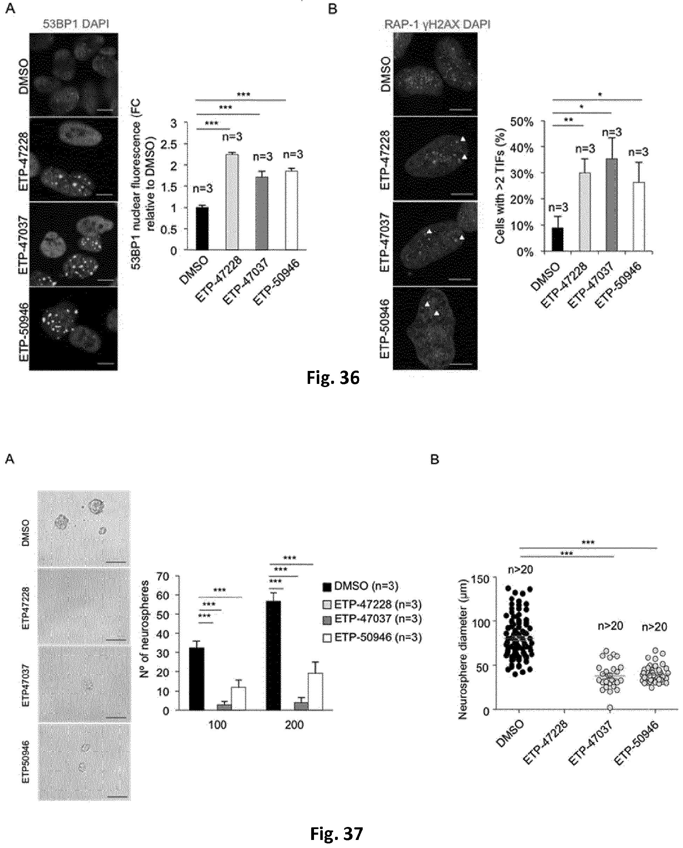

**Fig. 36****. TRF1 chemical modulators induce DNA damage in the U251 GBM cell line. (A)** 53BP1 mean intensity of U251 cells treated with the indicated compounds (right panel) and representative images (left panel). Scale bar 5 µm. **(B)** Representative images (left) and percentage (right) of cells presenting 2 or more γH2AX and RAP1 colocalizing foci (TIFs). White arrowheads: colocalization of γH2AX and RAP1. Scale bar 10 µm. Data are represented as mean ± SD. n represents number of biological replicates. Statistical analysis: unpaired *t-test.*
**Fig. 37****. TRF1 chemical modulators reduce sternness in the U251 GBM cell lines. (A)** Representative images (left) and quantification (right) of the number of neurospheres formed by U251 cells treated with the indicated compounds. Scale bar 100 µm. **(B)** Quantification of the neurosphere diameter after treatment with the indicated compounds. Data are represented as mean ± SD in 37A and mean ± SEM in 37B. n represents number of biological replicates in 37A and the number of spheres in 37B. Statistical analysis: unpaired *t-test.*

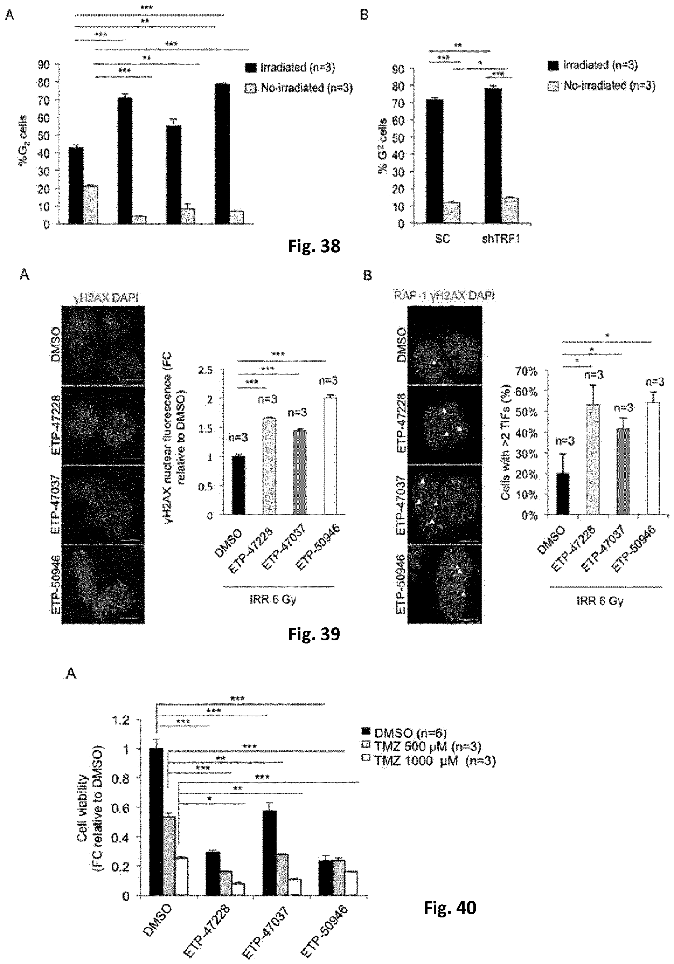

**Fig. 38****. TRF1 inhibition synergizes with γ-irradiation to induce cell cycle arrest. (A)** Percentage of U251 cells in G₂ phase upon 6 Gy irradiation and treated with the indicated compounds. **(B)** Percentage of U251 cells in G₂ phase upon 6 Gy irradiation in control- and *Trf1*-knocdown cells. Data are represented as mean ± SD. n represents number of biological replicates. Statistical analysis: unpaired *t-test.*
**Fig. 39****. TRF1 inhibition synergizes with γ-irradiation to induce DNA damage. (A)** γH2AX nuclear intensity in U251 cells and treated with the indicated compounds after 6Gy irradiation and representative images (left). DMSO represents IRR alone. Scale bar 5 µm. **(B)** Representative images (left) and percentage (right) of cells presenting 2 or more γH2AX and RAP1 colocalizing foci (TIFs) upon 6 Gy irradiation and treated with the indicated compounds. DMSO represents IRR alone. White arrowheads: colocalization of γH2AX and RAP1. Scale bar 10 µm. **(B)** Data are represented as mean ± SD. n represents number of biological replicates. Statistical analysis: unpaired *t-test.*
**Fig. 40****. TRF1 inhibition synergizes with temozolomide to reduce cell viability. (A)** Cell viability measured by an MTT assay in the U251 human cell line treated with the indicated compounds and no-temozolomide, temozolomide 500 µM or temozolomide 1000 µM for three days. Data are represented as mean ± SD. n represents biological replicates. Statistical analysis: unpaired *t-test.*

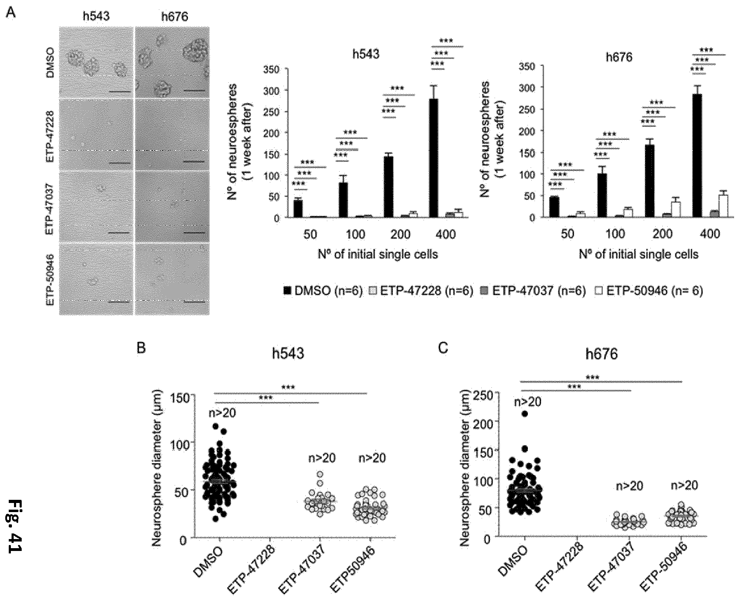

**Fig. 41****. TRF1 chemical downregulation reduces stemness in GSCs. (A)** Representative images (left) and quantification (right) of number of neurospheres formed by h543 and h676 GSCs treated with the indicated compounds. Scale bar 100 µm. **(B)** Quantification of the sphere diameters in the h543 and h676 patient-derived GSCs treated with ETP-47228, ETP-47037, ETP-50946 or DMSO. Data are represented as mean ± SD in 41A and mean ± SEM in 41B. n represents biological replicates in 41A and number of spheres in 41B. Statistical analysis: unpaired *t-test.*

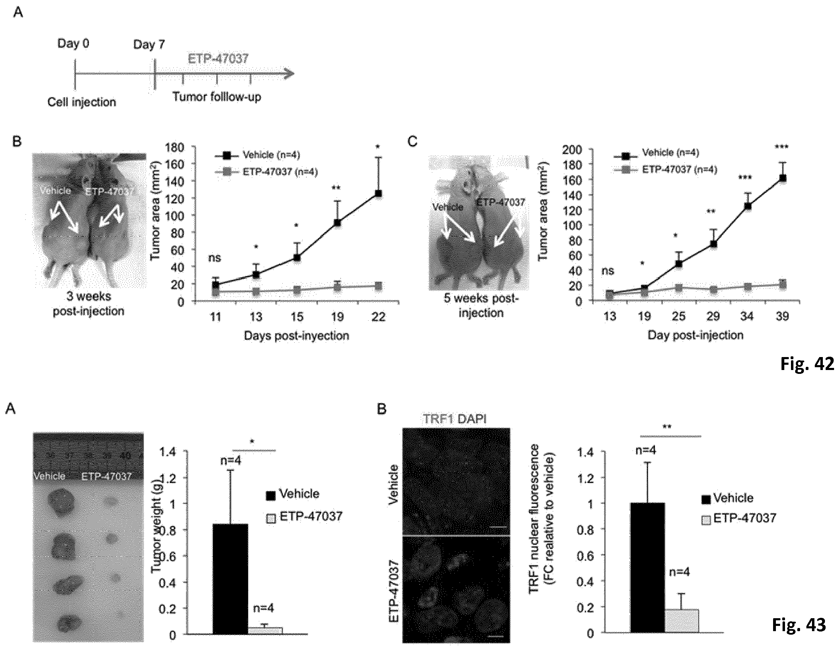

**Fig. 42****. Treatment with ETP-47037 chemical compounds reduces tumor growth in GSCs derived xenografts. (A**) Xenograft mouse models from patient-derived primary GSCs are generated by subcutaneous injection of GSCs into nude mice. One week after injection, mice are treated either with ETP-47037 or with vehicle as placebo. **(B**) Representative image of tumors (left) and longitudinal tumor growth follow-up (right) in ETP-47037-treated or vehicle-treated xenograft models with h676 GSCs (right). (**C**) Representative image of tumors (left) and longitudinal tumor growth follow-up (right) in ETP-47037 or vehicle-treated mice injected with h543 GSCs and representative image of tumors. Data are represented as mean ± SD. n represents the number of tumors. Statistical analysis: unpaired *t-test.*
**Fig. 43****. ETP-47037 treated tumors show smaller areas and a significant reduction in TRF1 protein levels. (A)** Representative image of tumors (left) and tumor weight (right) in ETP-47037 or vehicle-treated mice injected with h676 GSCs at post-mortem. (**B**) TRF1 nuclear fluorescence in ETP-47037 or vehicle-treated tumors. Scale bar 5 µm. Data are represented as mean ± SD. n represents the number of tumors. Statistical analysis: unpaired *t-test*

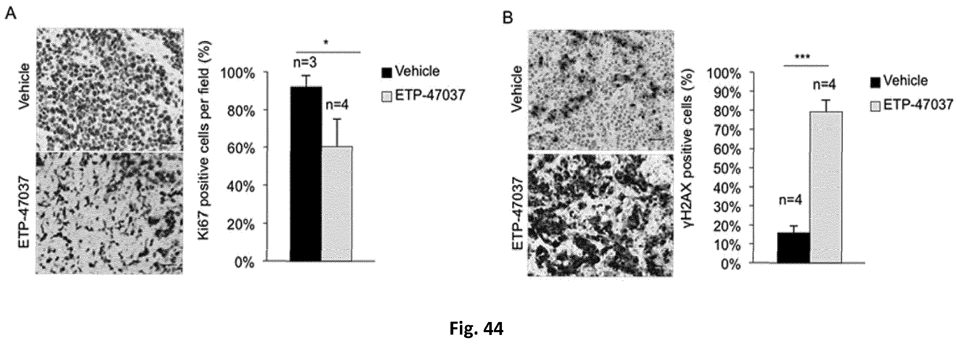

**Fig. 44****. ETP-47037 treatment reduces proliferation and induces DNA damage in GSCs derived xenografts. (A)** Representative images (left) and percentage (right) of Ki67-positive cells per field in ETP-47037 or vehicle-treated tumors. Scale bar 50 µm. (**B**) Representative images (left) and percentage (right) of yH2AX-positive cells per field in in ETP-47037 or vehicle-treated tumors. Scale bar 50 µm. Data are represented as mean ± SD. n represents the number of tumors. Statistical analysis: unpaired *t-test.*

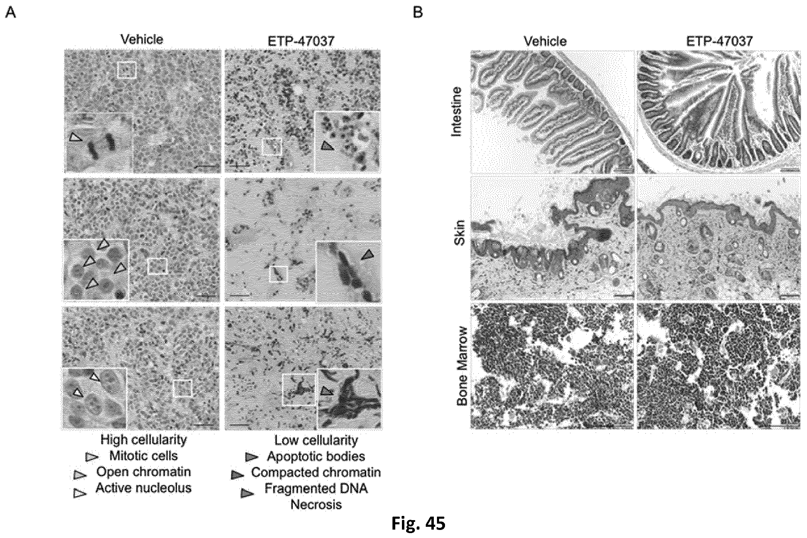

**Fig. 45****. Histological analysis of tumors and normal tissue after treatment with vehicle or ETP-47037.** (**A)** Histological analysis of the xenografts after treatment with ETP-47037 or vehicle. Scale bar 50 µm. **(B)** Histological analysis of the intestine, skin and bone marrow after treatment with ETP-47037 or vehicle. Scale bar 100 µm.

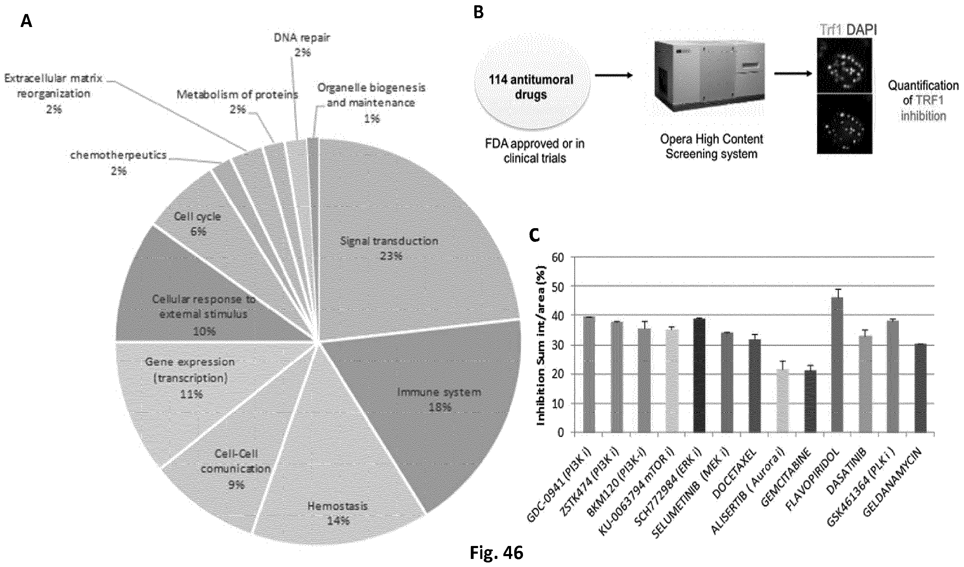

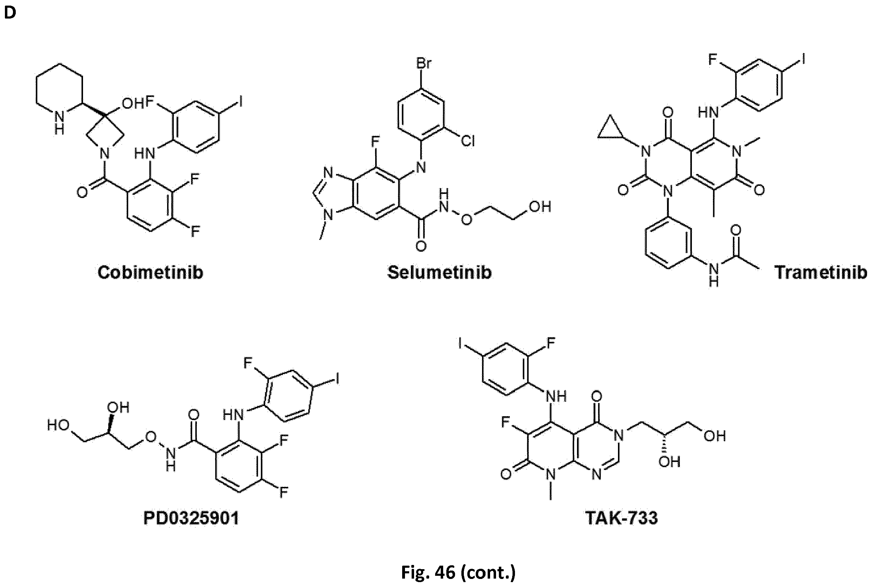

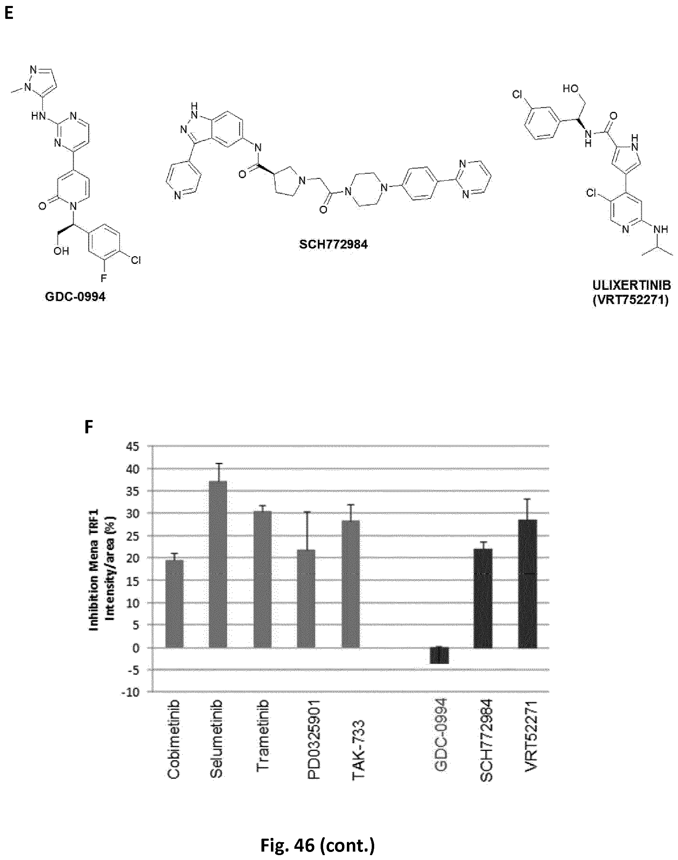

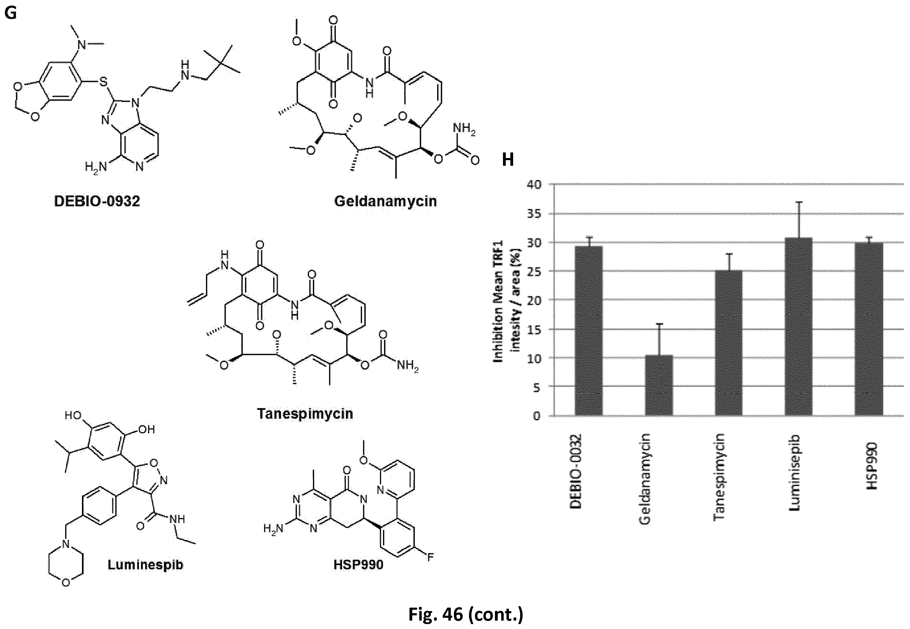

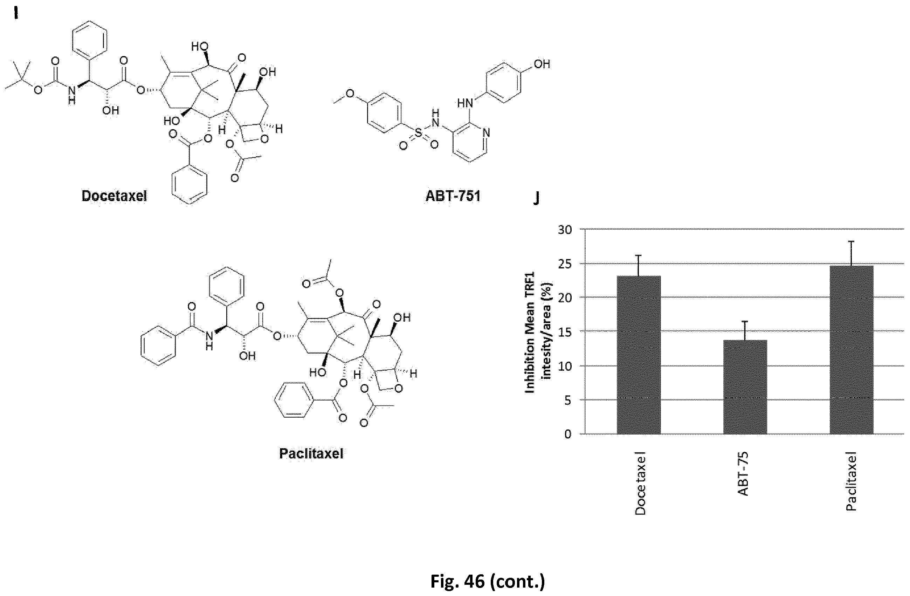

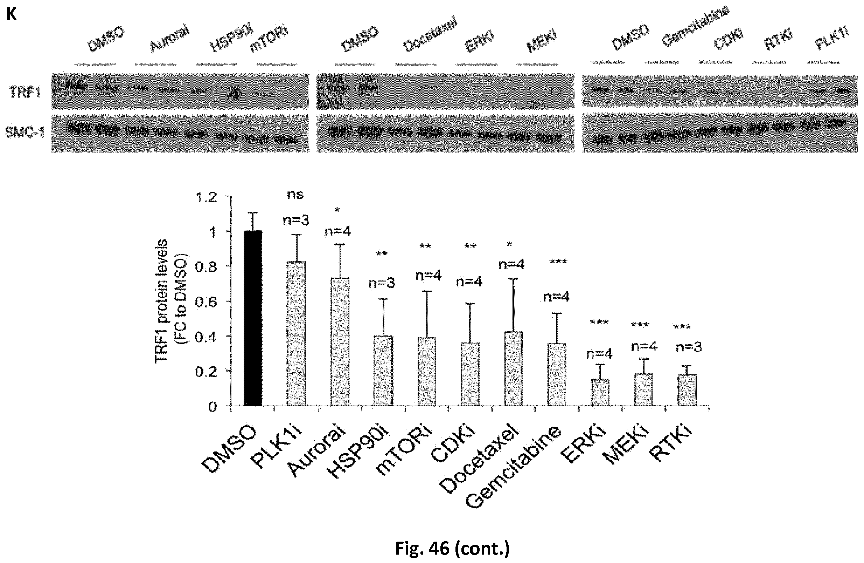

**Fig. 46****. Screening of ETP-antitumoral library: Identification of TRF1 modulators in compounds approved by FDA or in clinical trials. (A)** Drugs in ETP-antitumoral library distribution in Reactome Pathways. **(B)** Opera High ContentScreening system was used to identify compounds with the ability to inhibit TRF1, from representative images of confocal validation obtained from cells treated with DMSO (upper photograph of the right side of the panel and b) 1 µM of the tested compound, and determining the signal corresponding to TRF1 foci; TRF1 foci (green signal) can be appreciated clearly in the upper on the nuclear staining with DAPI (blue signal), but they have decreased in the lower photograph. **(C)** Representation of the inhibition of TRF1 levels by drugs belonging to different signaling pathways that modulate TRF1 at telomeres. Bar fillings vary depending on the signaling pathway. The results corresponding to three PI3K inhibitors inside the ETP-antitumoral library are represented by the three first bars from the right, validating the screening. **(D)** Structurally diverse MEK inhibitors tested in the chemical biology validation of the MEK/ERK pathway as modulator of TRF1 levels. **(E)** Structurally diverse ERK inhibitors tested in the chemical biology validation of the MEK/ERK pathway as modulator of TRF1 levels. **(F)** Inhibition of TRF1 levels by the MEK and ERK inhibitors represented in (D) and (E), respectively, measured by immunofluorescence. **(G)** Structurally diverse HSP90 inhibitors tested in the chemical biology validation of HSP90 pathway as modulator of TRF1 levels. **(H)** Inhibition of TRF1 levels by the HSP90 inhibitors represented in (G), measured by immunofluorescence. **(I)** Structurally diverse tubulin agent tested in the chemical biology validation of tubulin agents as modulator of TRF1 levels. **(J)** Inhibition of TRF1 levels by the tubulin agents represented in (I), measured by immunofluorescence. **(K)** Western Blot (up) and quantification (down) of TRF1 protein levels in the h676 GSCs after 24 h treatment with 1 µM of the indicated compounds. Data are represented as mean ± SD. n represents biological replicates. Statistical analysis: unpaired *t-test.*

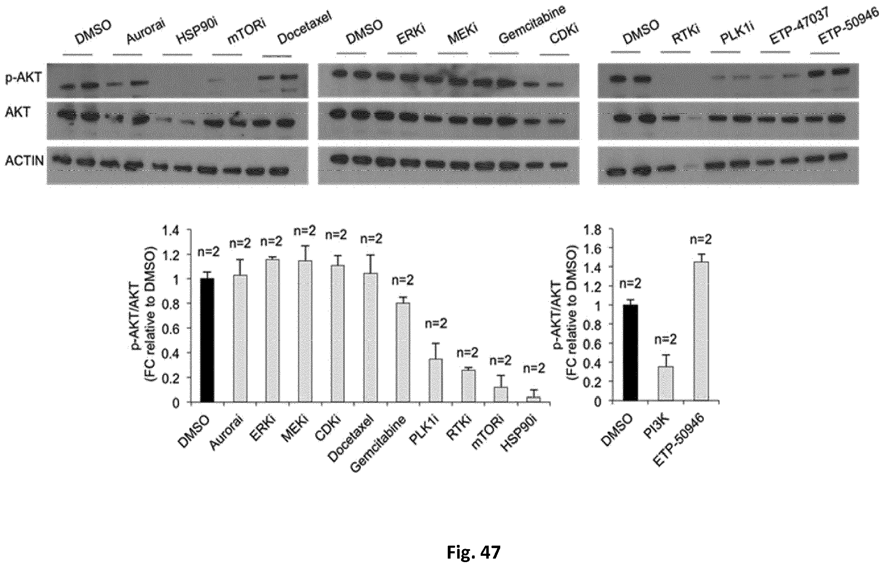

**Fig. 47****. Characterization of p-AKT activation after treatment with the novel TRF1 modulators.** Western Blot (up) and quantification (down) of p-AKT/AKT ratio in the h676 GSCs after 24 h treatment with 1 µM of the indicated compounds. Data are represented as mean ± SD. n represents biological replicates.

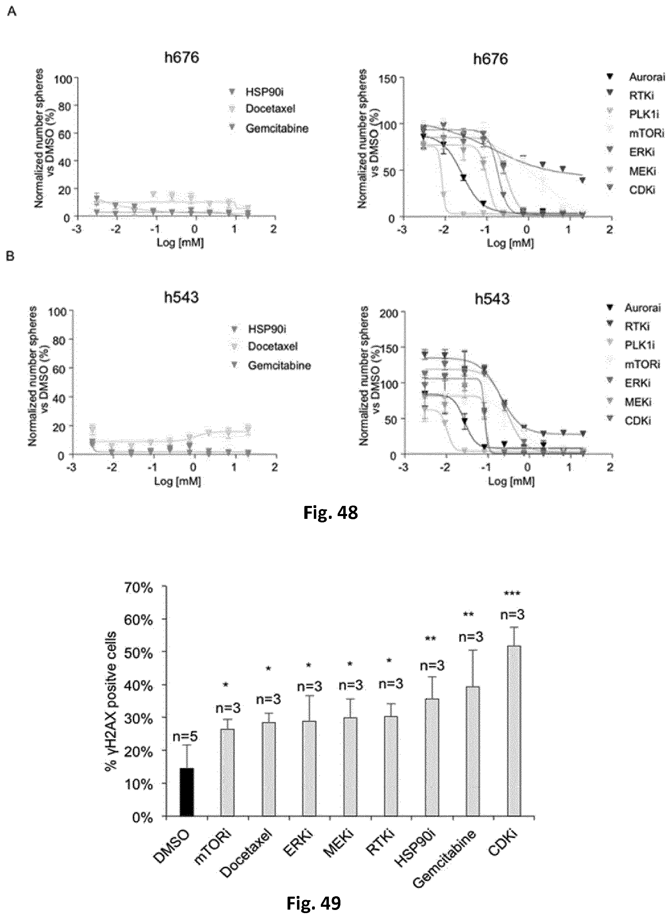

**Fig. 48****. Novel TRF1 modulators impair sphere formation in GSCs.** (**A)** Dose-response curves of the h676 GSCs treated 7 days with the indicated compounds. **(B)** Dose-response curves of the h543 GSCs treated 7 days with the indicated compounds. Data are represented as mean ± SD.
**Fig. 49****. Novel TRF1 modulators induce DNA damage.** γH2AX quantification in h676 GSCs after treatment with the indicated compounds for 24 hr. Data are represented as mean ± SD. n represents biological replicates. Statistical analysis: unpaired *t-test.*

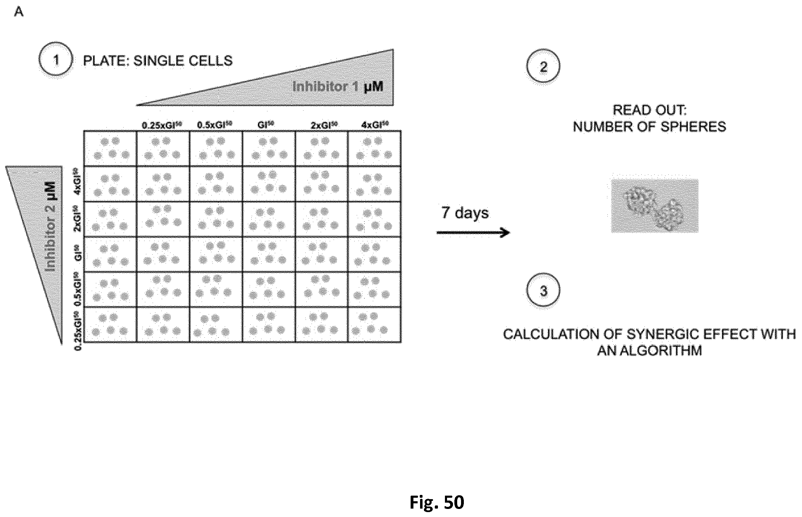

**Fig. 50****. Combination studies (A)** Steps for the characterization of synergic effects: (1) Disaggregated GSCs are plated into a matrix of different concentrations of 2 independent compounds; (2) After 7 days the number of spheres are counted in each well; and (3) The synergic effect is calculated using an algorithm.

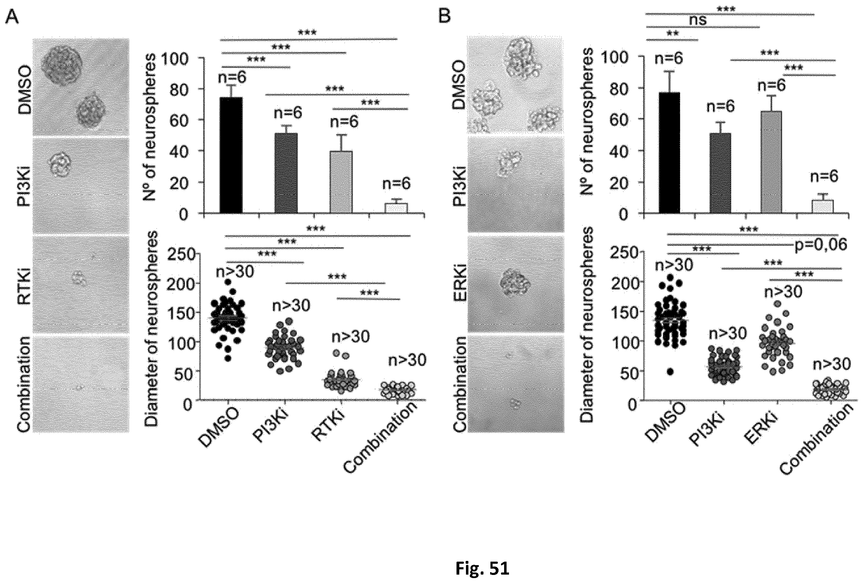

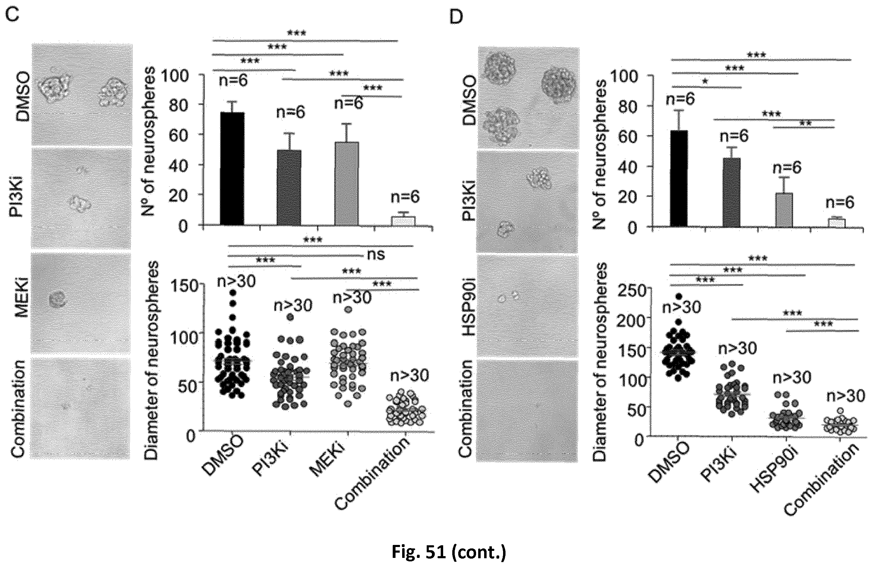

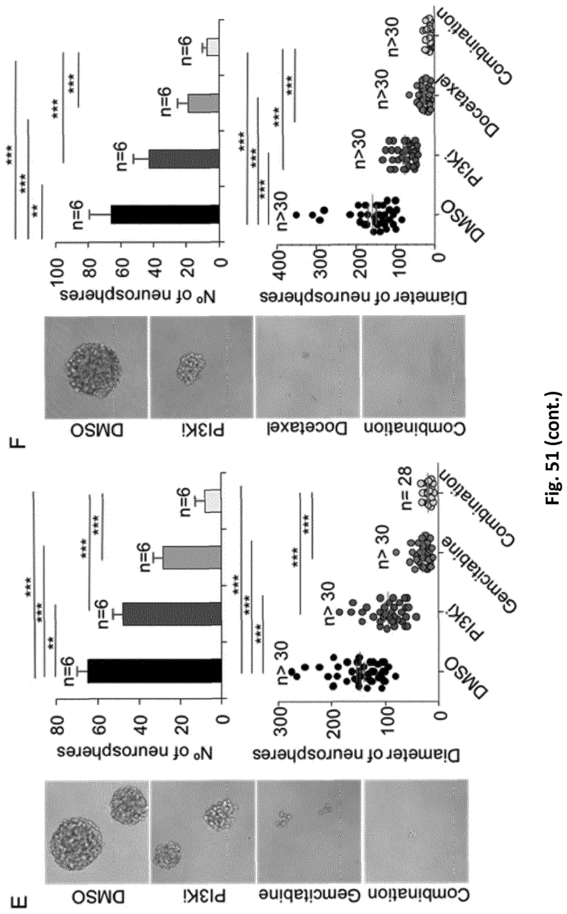

**Fig. 51****. ETP-47037 (PI3Ki) shows synergic effect with various novel TRF1 modulators. (A-F)** Quantification of number of spheres (up) and diameter (down) in h676 GSCs treated with the indicated compounds. Representative image of the spheres (left). Data are represented as mean ± SD (up) or mean ± SD (down). n represents biological replicates (up) or number of spheres (down). Statistical analysis: unpaired *t-test.*

### DETAILED DESCRIPTION OF THE INVENTION

As described above, the present invention relates to the therapeutic use of TRF1 inhibitors for the prevention and/or treatment of glioblastoma multiforme (GBM) and other brain tumors. The subject to be treated will be an animal suffering or having suffered from GBM or another brain tumor, preferably a mammal, and more preferably a human being, and the therapy will be intended to block, diminish or slow the progression of a brain tumor such a glioblastoma tumor and/or to delay or prevent recurrence of the tumor. Then, the present invention relates to TRF1 inhibitors for use in the treatment of glioblastoma multiforme, and other brain tumors, as well as to a method for the treatment of a glioblastoma tumor or another brain tumor, wherein the subject is animal suffering or having suffered from GBM or another brain tumor, which animal will be preferably a mammal, and more preferably a human being.

The TRF1 inhibitor can be administered in a composition, which composition can be administered, for instance, intravenously, as it is demonstrated in the Examples of the present application, so that a composition which comprises at least a TRF1 inhibitor for use in the treatment or prevention of glioblastoma or another brain tumor is also comprised within the present invention. The composition may have more than one TRF1 inhibitor, that is, at least a first TRF1 inhibitor and a second TRF1 inhibitor. The compositions with at least a TRF1 inhibitor which acts through the AKT/PI3K pathway and at least a TRF1 inhibitor selected from the group of RTK inhibitors, ERK inhibitors, MEK inhibitors, HSP90 inhibitors, Docetaxel and Gemcitabine are particularly, preferred, because said combinations of TRF1 inhibitors shows synergistic effects.

The invention is based on the assays described in detailed in the "Examples" section of the present application, which were carried out with different GBM mouse models and patient-derived GSCs-based models, and where the researchers use both genetic ablation mouse models as well as chemical inhibition to validate direct targeting of telomere protection, instead of telomerase activity, as an effective target in GBM. The following results and findings were obtained:
1. TRF1 is upregulated in three different mouse GBM models (comparing tumor areas with non-tumor areas) and in different human GBM cells compared to astrocytes. Also, TRF1 immunofluorescence analysis in tissue samples from normal brain, astrocytomas and GBM revealed that GBM exhibit the highest percentage of TRF1 high cells, followed by astrocytomas, while in normal brain TRF1 was almost undetectable.
2. TRF1 overexpression is independent of telomere length in mouse GBM. It is remarkable that the therapeutic effect of TRF1 inhibition occurs in a telomere length independent manner, because it overcomes the potential problem of telomere length heterogeneity within tumors and the inability to kill all tumor cells including the tumor-initiating populations
3. TRF1 inhibition blocks both tumor initiation and progression in two independent mouse models of GBM, by a mechanism which involves DNA damage induction, reduced stemness and reduced proliferation
4. TRF1-defficient NSCs (neural stem cells) and GSCs (glioma stem-like cells) present a reduced stemness and tumorigenicity, at the same time that induces a DNA damage response at telomeres both in isolated populations of these cells as well as in the context of tumors
5. As a consequence of the effects set forth in points 2 and 3, tumors appear much later, again pointing the importance of *Trf1* for the stem cell characteristics of this kind of tumors
6. *Trf1* deletion is also effective once the tumors are established, demonstrating its potential as a cancer target, which could be translated into human patients and other animals suffering from GBM or other brain tumors
7. *Trf1* brain-specific or whole-body deletion in healthy mice does not impair mice viability or cognitive functions. This result is consistent with previous results of the same research group (Garcia-Beccaria *et al.,* 2015) which showed that TRF1 full body deletion does not impair organism viability and only affects slightly to high proliferative tissues. Thus, TRF1, anti-cancer target, fulfill the important requisite of not showing deleterious effects in healthy tissues or compromising organism viability, even the cancer to treat is a tumor of the central nervous system such as a brain tumor like GBM
8. Several compounds, most of them already approved by the FDA or in clinical trials, have the ability to downregulate TRF1 levels and reduce stemness in GSCs. Among these compounds there are: PI3K inhibitors, mTOR inhibitors, RTK inhibitors, ERK inhibitors, MEK inhibitors, Docetaxel, Gemcitabine, CDK inhibitors and HSP90 inhibitors. Also, an additional compound, ETP-50946, effectively downregulate TRF1 levels and reduces stemness in GSCs.
9. Different combinations of the compounds stated in point 5 show synergic effects in vitro in GSCs. Specifically, the present inventors have found synergistic effects among compounds which are PI3K inhibitors (TRF1 inhibitors acting through the AKT/PI3K pathway) and RTK inhibitors, ERK inhibitors, MEK inhibitors, HSP90 inhibitors, Docetaxel and Gemcitabine.

Thus, TRF1 appears as a potentially interesting target for targeting both GBM telomere length heterogeneity and tumor-initiating capabilities and for blocking the development of already initiated GBM tumors.

Based on such findings, it is herein provided to use TRF1 as a novel target in the treatment of GBM and other brain tumors. It can be achieved by: (1) using different compounds with the ability to target TRF1 and decrease its activity, which compounds give rise to a decrease of TRF1 protein level through their action at different pathways; (2) by using combinations of the compounds mentioned in point (1) or (3) by using the compound ETP-50946. The high levels of TRF1 found in astrocytomas is a support for the applicability of such approach for other brain tumors, such as astrocytomas. On the other hand, the effects found in GSCs, the main cause of GBM recurrence, indicate that targeting TRF1 could be also useful to delay or prevent the recurrence of GBM tumors.

The findings of the present inventors are consistent with previous knowledge about telomere maintenance and GBM, which reinforce the possibility of achieving therapeutic effects in GBM by targeting the telomeres. Thus, the promoter of the catalytic subunit of telomerase (TERT) is mutated in 58-84% of human primary GBMs (Arita et al. 2013; Boldrini et al. 2006; Brennan et al. 2013; Koelsche et al. 2013; Nonoguchi et al. 2013), while paedriatic GBMs frequently display an ALT phenotype (alternative lengthening of telomeres) associated with ATRX mutations (Heaphy et al. 2011; Schwartzentruber et al. 2012). Also, a component of the shelterin complex, POT1, has been found to be mutated in familiar glioblastoma cases (Bainbridge et al. 2015; Calvete et al. 2015; Newey et al. 2012; Ramsay et al. 2013; Robles-Espinoza et al. 2014; Shi et al. 2014; Zhang et al. 2014). These facts highlight the importance of telomere maintenance in glioblastoma, but left unsolved the problem of how to deal with the strong recurrence observed in GBM due to the fact that current GBM treatments do not affect GSCs, which are able to recapitulate the whole tumor.

As can be seen in the assays included in the Examples section below, the present inventors have extensively demonstrated that targeting TRF1 affects every cell in the tumor, including GSCs. Based on this fact, TRF1 inhibition shows a great advantage in comparison with the current treatments in GBM.

In addition, the findings of the present inventors demonstrate that targeting TRF1 in cancer could also be apply to other tumor types, specifically those with a high stem cell nature, something that was not obvious from the prior art.

Even though the group of the present inventors had previously reported that *Trf1* inhibition was effectively blocking tumor growth in lung cancer mouse models, it was not obvious that the same results could be obtained in Glioblastoma. As has been explained above, GBM is a tumor with a mean survival of 14-16 months. The bad prognosis of GBM is mainly due to the heterogeneity of the disease, as there is co-existence of both tumor cells and GSCs. The GSCs are known to show radio-resistance and chemo-resistance properties, and this causes tumor relapse after the standard treatments. This *stem* nature of GBM is what makes it different to other tumors like lung tumors, and also makes it more difficult to find effective therapies. The present inventors have been the first ones to demonstrate that *Trf1* genetic and chemical inhibition drastically reduces stemness in glioma stem cells. In summary, the fact that it had been already demonstrated that *Trf1* inhibition worked in lung tumors did not make it obvious that it would also work in GBM.

The results obtained in GBM were also not obvious or expectable even knowing that *Trf1* is enriched and necessary for adult and pluripotent stem cells, because the fact that *Trf1* is essential for adult and pluripotent stem cells does not imply that it will also be important for cancer stem cells. Cancer stem cells are genetically very different to adult and pluripotent stem cells, what makes unexpectable the behavior of cancer stem cells from the knowledge of adult and pluripotent stem cells. The present inventors have been the ones who have described, for the first time, the effects of *Trf1* deletion in cancer stem cells, in particular in glioma-stem cells. The have been also the first ones to demonstrate the *Trf1* genetic and chemical inhibition reduced stemness in glioma-stem cells, which results in a significant impact in mice survival, which effect was previously not expectable.

As it is used herein, the abbreviation TRF1 (Telomeric Repeat Binding Factor 1, also abbreviated as TERF1) encompasses TRF1 proteins of all animals, including mammals and, among them, human beings. When TRF1 refers specifically to the human protein (UnitProtKB P54274, encoded by gene 11728 of HGNC database, mRNA Genbank access NM_017489, version NM017489.2 of 22 July 2018), it is specified. Trf1, (written with only one capital letter), as it is used herein, refers to the mouse protein (UnitProtKB P70371, encoded by gene ID 21749 of NCBI database, updated 12 August 2018), while *Trf1* refers to the mouse gene which encodes Trf1 mouse protein.

As used herein, "targeting TRF1" both means modulating the expression of TRF1 gene and modulating TRF1 protein activity, either by directly provoking an increase or decrease of the protein activity or by affecting TRF1 protein levels. "Modulating" means provoking a change in expression (gene) or activity (protein), either an increase or a decrease. As used herein, "inhibiting" means having a negative effect on TRF1 activity, either by provoking a download in TRF1 gene expression which results in a decrease of TRF1 activity due to a decrease in TRF1 protein level, or by provoking a decrease in the activity of the previously expressed proteins as such, or combinations thereof. Thus, the decrease of TRF1 activity might result from a direct interaction of a compound with TRF1 protein, from an increase in the levels of one or more compounds that directly interact with TRF1 protein and hinder its activity, or from the action of a compound that is a modulator of a pathway and that has an influence in TRF1 expression, or might occur by other means, provided that a decrease in the activity of TRF1 protein can be observed.

Consequently, a "TRF1 inhibitor", as used herein, is an environmental factor or a compound that gives rise to a decrease of TRF1 activity, whatever the means by which it acts. The term "compounds that are TRF1 inhibitors" encompasses both nucleotide sequences or analogues thereof that decrease TRF1 gene expression (such as oligodeoxyribonucleotides, oligoribonucleotides or siRNA), and other compounds, usually referred herein as "chemical compounds", which are not comprised by a sequence of nucleotides or analogues thereof and, often, have been obtained by a chemical synthetic process in a laboratory.

Among the compounds that can be used as TRF1 inhibitors, a possible embodiment is the use of compounds that are PI3K inhibitors or TRF1 inhibitors that act through the AKT/PI3K pathway and, among them, the compounds claimed in international patent applications WO2010119264 and WO2011089400. Some of the inventors of the present invention recently found that the compounds claimed in said applications belong to a PI3K inhibitors family (Méndez-Pertuz et al. 2017). Interestingly, characterization of these PI3K inhibitors uncovered an important functional connection between the PI3K pathway and TRF1 regulation, thus connecting two of the major pathways in cancer and aging, namely telomeres and the PI3K pathway (Méndez-Pertuz et al. 2017). In particular, the authors of said scientific article found that PI3K chemical inhibitors, as well as inhibitors of the PI3K downstream target AKT, significantly reduce TRF1 telomeric foci and lead to increased telomeric DNA damage and fragility. By using both chemical and genetic ablation of different PI3K catalytic subunits, PI3Kα, but not the other Pi3K isoforms, was identified as responsible for this TRF1 inhibition. It is also described in the same article that TRF1 is phosphorylated at different residues by AKT and that these modifications regulate TRF1 foci formation *in vivo* (Mendez-Pertuz et al. 2017).

Thus, in a possible embodiment of the present invention, the TRF1 inhibitor can be a PI3K inhibitor (preferably, a PI3Kα inhibitor) or a TRF1 inhibitor that acts through the AKT/PI3K pathway. In a possible preferred embodiment, the TRF1 inhibitor is a compound claimed in international patent application WO2010119264 or WO2011089400, which are herein incorporated by reference. Thus, the TRF1 inhibitor can be a compound of formula 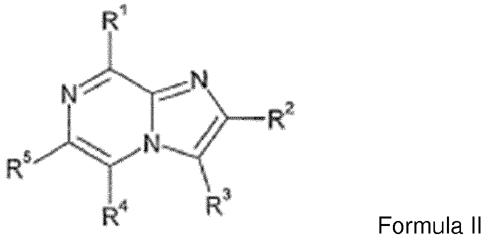 wherein:
R¹ represents:
   (a) -N(R^{1a})R^{1b}, in which R^{1a} and R^{1b} are linked together to form, together with the nitrogen atom to which they are necessarily attached, a 5- to 7-membered ring optionally containing a further one or two heteroatoms, optionally containing one or two double bonds, and which ring is optionally substituted by one or more substituents selected from =O and B¹;
   (b) a heterocycloalkyl group (attached to the requisite imidazopyrazine via a carbon atom), optionally substituted by one or more substituents selected from =O and B²;
   (c) a monocyclic heteroaryl group optionally substituted by one or more substituents selected from B³;
R² and R³ independently represent:
   (i) hydrogen;
   (ii) (ii) Q¹;
   (iii) C₁₋-_{I2} alkyl optionally substituted by one or more substituents selected from =O, =S, =N(R^{10a}) and Q²; or
R² or R³ may represent a fragment of Formula IIR 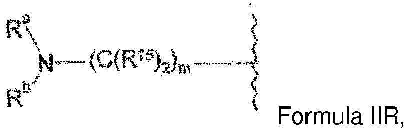 wherein
   m represents 0, 1, 2, 3, 4, 5 or 6;
   each R¹⁵ represents hydrogen, halo or C₁₋₆ alkyl optionally substituted by one or more substituents selected from E¹; or
   the two R¹⁵ groups may linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E²;
   R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
      (a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring;
      (b) comprises a linker group -(C(R^{X})₂)_{P}- and/or -(C(R^{x})₂)r-O-(C(R^{x})₂)s- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
      (c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
   all of which cyclic groups, defined by the linkage of R^{a} and R^{b}, are optionally substituted by one or more substituents selected from =O and E³;
R⁴ represents hydrogen or a substituent selected from halo, -CN, -OR^{10b}, -N(R^{10b})R^{11b},-C(O)N(R^{10b})R^{11b}, -C(O)R^{10b}, C₁₋₆ alkyl and heterocycloalkyl, which latter two groups are optionally substituted by one or more substituents selected from E⁴ and =O;
   but wherein at least one of R², R³ and R⁴ represents a substituent other than hydrogen; R⁵ represents aryl or heteroaryl (both of which are optionally substituted by one or more substituents selected from E⁵);
each Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -OC(=Y)-R^{10a}, -OC(=Y)-OR^{10a}, -OC(=Y)N(R^{10a})R^{11a}, -OS(O)₂OR¹⁰³, -OP(=Y)(OR^{10a})(OR^{11a}), -OP(OR^{10a})(OR^{11a}), -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R¹⁰³, -SR^{10a}, -S(O)R^{10a}, C_{1-I2} alkyl, heterocycloalkyl (which latter two groups are optionally substituted by one or more substituents selected from =O, =S, =N(R^{10a}) and E⁶), aryl or heteroaryl (which latter two groups are optionally substituted by one or more substituents selected from E⁷);
each B¹, B² and B³ independently represent halo, -NO₂, -CN, -N(R^{10a})R^{11a}, -OR^{10a}, -C(=Y)-R^{10a}, -C(=Y)-OR^{10a}, -C(=Y)N(R^{10a})R^{11a}, -N(R^{12a})C(=Y)R^{11a}, -N(R^{12a})C(=Y)OR^{11a}, -N(R^{12a})C(=N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -SC(=Y)OR^{10a}, -S(O)₂R^{10a}, C₁₋₁₂ alkyl, heterocycloalkyl (which latter two groups are optionally substituted by one or more substituents selected from =O and E⁸), aryl or heteroaryl (which latter two groups are optionally substituted by one or more substituents selected from E⁹);
or, any two B¹ substituents, when attached to the same carbon atom (thereby forming a spiro-cycle), may be linked together to form, a 3- to 12- membered ring, optionally containing one or more heteroatoms, which ring optionally contains one or more double bonds, and which ring is itself optionally substituted by one or more substituents selected from halo, =O and Ci₋₃ alkyl optionally substituted by one or more fluoro atoms;
each R^{10a}, R^{11a}, R^{12a}, R^{10b} and R^{11b} independently represent, on each occasion when used herein, hydrogen, C^₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a}, R^{11a} and R^{12a} and/or any pair of R^{10b} and R^{11b} may be linked together to form a 3- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁴, E⁵, E⁶, E⁷, E⁸, E⁹, E¹⁰, E¹¹ and E¹² independently represents, on each occasion when used herein:
   (i) Q⁴;
   (ii) C_{1-I2} alkyl optionally substituted by one or more substituents selected from =O and Q⁵;
   or
any two E¹, E², E³, E⁴, E⁵, E⁶, E⁷, E⁸, E⁹, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), -OP(OR²⁰XOR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or any relevant pair of R²⁰, R²¹ and R²², may be linked together to form a 3- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
   (i) Q⁷;
   (ii) C₁₋₆ alkyl or heterocycloalkyl, both of which are optionally substituted by one or more substituents selected from =O and Q⁸;
each Q⁷ and Q⁸ independently represents, on each occasion when used herein: -CN, halo, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂R⁵⁰, -SR⁵⁰, -S(O)R⁵⁰ or C₁₋₆ alkyl optionally substituted by one or more fluoro atoms;
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms;
or a pharmaceutically acceptable ester, amide, solvate or salt thereof.

Among the possible compounds of Formula II, a possible preferred group is the group of compounds wherein
i) R¹ is a heterocycloalkyl group, preferably a 6-membered heterocycloalkyl group, and more preferably a morpholine ring,
ii) R⁴ is H,
iii) R⁵ is a substituted aryl group, wherein the substituent, R, is preferably in the *p*-position with regard to the position whereby R⁵ it is linked to Formula II.

Thus, a group of preferred compounds among those of Formula II are the compounds of Formula Ila 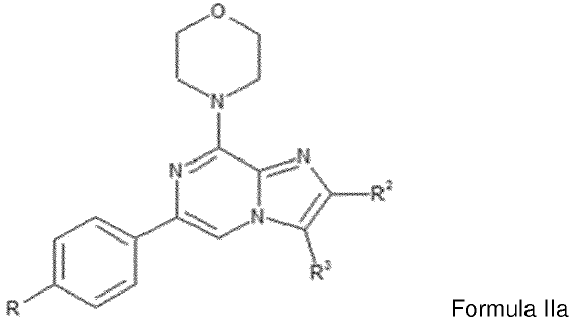 and particularly those compounds wherein
R represents NH-CO-NH-Ph, where the later Ph (phenyl) group is substituted in the p-position with a group CO-piperazinyl additionally substituted in the 4-piperazinyl N with methyl;
R² and R³ independently represent:
   (i) hydrogen;
   (ii) Q¹;
   (iii) C₁₋₁₂ alkyl optionally substituted by one or more substituents selected from =O, =S, =N(R^{10a}) and Q²; or
R² or R³ may represent a fragment of Formula IIR 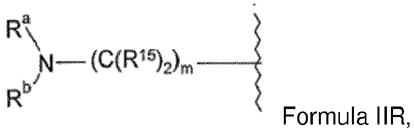 wherein
   m represents 0, 1, 2, 3, 4, 5 or 6;
   each R¹⁵ represents hydrogen, halo or C₁₋₆ alkyl optionally substituted by one or more substituents selected from E¹; or the two R¹⁵ groups may linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E²;
   R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
      (a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring;
      (b) comprises a linker group -(C(R^{X})₂)_{P}- and/or -(C(R^{x})₂)r-O-(C(R^{x})₂)s- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
      (c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle), all of which cyclic groups, defined by the linkage of R^{a} and R^{b}, are optionally substituted by one or more substituents selected from =O and E³;
each Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -OC(=Y)-R^{10a}, -OC(=Y)-OR^{10a}, -OC(=Y)N(R^{10a})R^{11a}, -OS(O)₂OR¹⁰³, -OP(=Y)(OR^{10a})(OR^{11a}), -OP(OR^{10a})(OR^{11a}), -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R^{10a}, -SR^{10a}, -S(O)R^{10a}, C_{1-I2} alkyl, heterocycloalkyl (which latter two groups are optionally substituted by one or more substituents selected from =O, =S, =N(R^{10a}) and E⁶), aryl or heteroaryl (which latter two groups are optionally substituted by one or more substituents selected from E⁷); each R^{10a}, R^{11a}, R^{12a}, R^{10b} and R^{11b} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a}, R^{11a} and R^{12a} and/or any pair of R^{10b} and R^{11b} may be linked together to form a 3- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁴, E⁵, E⁶, E⁷, E⁸, E⁹, E¹⁰, E¹¹ and E¹² independently represents, on each occasion when used herein:
   (i) Q⁴;
   (ii) C_{1-I2} alkyl optionally substituted by one or more substituents selected from =O and Q⁵;
   or
any two E¹, E², E³, E⁴, E⁵, E⁶, E⁷, E⁸, E⁹, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰,-OC(=Y)-OR²⁰, -OC(=Y)N(R²⁰)R²¹, -OS(O)₂OR²⁰, -OP(=Y)(OR²⁰)(OR²¹), -OP(OR²⁰XOR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹ and R²², may be linked together to form a 3- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
   (i) Q⁷;
   (ii) C₁₋₆ alkyl or heterocycloalkyl, both of which are optionally substituted by one or more substituents selected from =O and Q⁸;
each Q⁷ and Q⁸ independently represents, on each occasion when used herein: -CN, halo, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂R⁵⁰, -SR⁵⁰, -S(O)R⁵⁰ or C₁₋₆ alkyl optionally substituted by one or more fluoro atoms;
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms;
or a pharmaceutically acceptable ester, amide, solvate or salt thereof.

Among the compounds of Formula Ila above defined, a possible embodiment is that R² is H and R³ is -CH3 (methyl), such as the compound of the following formula:

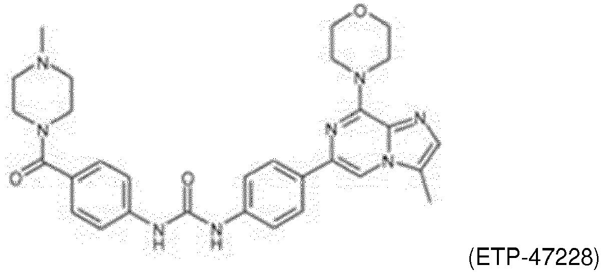

This compound is named ETP-47228 below, as in previous works of the group of the present inventors (Garcia-Beccaria *et al.,* 2015).

The TRF1 inhibitor can be also one of the compounds claimed in WO2011089400, which compounds are also PI3K inhibitors, that is, a compound of Formula III 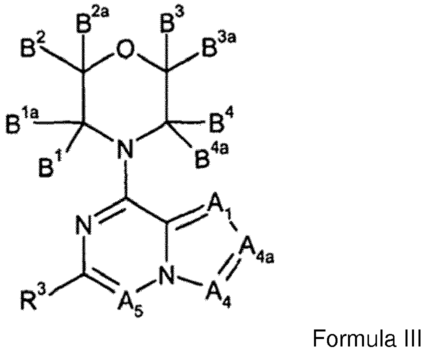 wherein
A₁ represents N or C(R¹);
A₄ represents N or C(R^{1a});
A4a represents N or C(R^{1b});
wherein at least one of A₄ and A₄ₐ does not represent N;
A5 represents N or C(R²);
each B¹, B^{1a}, B², B^{2a}, B³, B^{3a}, B⁴ and B^{4a} independently represent hydrogen or a substituent selected from halo, -C(=Y)-R^{10a}, -C(=Y)-OR^{10a}, -C(=Y)N(R^{10a})R^{11a}, -S(O)₂N(R^{10a})R^{11a}, C₁₋₁₂ alkyl, heterocycloalkyl (which latter two groups are optionally substituted by one or more substituents selected from =O and E¹), aryl and/or heteroaryl (which latter two groups are optionally substituted by one or more substituents selected from E²); or
any two B¹, B^{1a}, B², B^{2a}, B³, B^{3a}, B⁴ and B^{4a} substituents that are attached to the same carbon atom (i.e. B¹ and B^{1a}; B² and B^{2a}; B³ and B^{3a}; and/or B⁴ and B^{4a}) may together form a =O group;
or, any two B¹, B^{1a}, B², B^{2a}, B³, B^{3a}, B⁴ and B^{4a} substituents may be linked together to form a further 3- to 12- membered ring, optionally containing (in addition to the atom(s) of the morpholine ring) one or more heteroatom(s), which ring optionally contains one or more double bonds, and which ring is itself optionally substituted by one or more substituents selected from halo, =O and C₁₋₃ alkyl optionally substituted by one or more fluoro atoms; R¹ and R² independently represents hydrogen or a substituent selected from halo, -CN,-OR^{10b}, -N(R^{10b})R^{11b}, -C(O)N(R^{10b})R^{11b}, C₁₋₁₂ (e.g. C₁₋₆) alkyl and heterocycloalkyl, which latter two groups are optionally substituted by one or more substituents selected from E³ and =O;
R^{1b} (when present) represents:
   (i) C₁₋₁₂ alkyl optionally substituted by one or more substituents selected from Q^{1a};
   (ii) heterocycloalkyl (linked via a carbon atom) optionally substituted by one or more substituents selected from =O and Q^{1b}; or
   (iii) a fragment of formula IIIR;
R^{1a} (when present) represents:
   (i) hydrogen;
   (ii) Q¹;
   (iii) C₁₋₁₂ alkyl optionally substituted by one or more substituents selected from =O, =S, =N(R^{10a}) and Q²; or
   (iv) a fragment of formula IIIR;
   the fragment of formula IIIR represents: 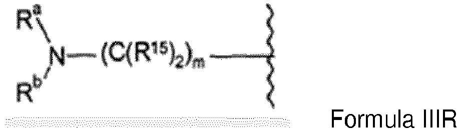 wherein:
   m represents 1, 2, 3, 4, 5 or 6;
   each R¹⁵ represents hydrogen, halo or C₁₋₆ alkyl optionally substituted by more substituents selected from E⁴; or
   the two R¹⁵ groups may be linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E⁵;
   R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
      (a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring (in which the heteroatoms are preferably selected from sulfur and, especially, nitrogen and oxygen);
      (b) comprises a linker group -(C(R^{x})₂)ₚ- and/or -(C(R^{x})₂)ᵣ-O-(C(R^{x})2)ₛ- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
      (c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
   all of which cyclic groups, defined by the linkage of R^{a} and R^{b}, are optionally substituted by one or more substituents selected from =O, =NOR^{10a} and E⁶;
R³ represents aryl or heteroaryl (both of which are optionally substituted by one or more substituents selected from E⁷);
each Q^{1a}, Q^{1b}, Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a},-C(=Y)N(R^{10a})-OR^{11c}, -OC(=Y)-R^{10a}, -OC(=Y)-OR^{10a}, -OC(=Y)N(R^{10a})R^{11a}, -OS(O)₂OR^{10a},-OP(=Y)(OR^{10a})(OR^{11a}), -OP(OR^{10a})(OR^{11a}), -N(R^{12a})C(=Y)R^{11a}, -N(R^{12a})C(=Y)OR^{11a},-N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a},-SC(=Y)R^{10a}, -S(O)₂R^{10a}, -SR^{10a}, -S(O)R^{10a}, C₁₋₁₂ alkyl, heterocycloalkyl (which latter two groups are optionally substituted by one or more substituents selected from =O, =S, =N(R^{10a}) and E⁸), aryl or heteroaryl (which latter two groups are optionally substituted by one or more substituents selected from E⁹);
each R^{11c} independently represents C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹);
each R^{10a}, R^{11a}, R^{10b}, R^{11b} and R^{12a} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a} and R^{11a} or R^{10b} and R^{11b} may be linked together to form a 4- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁴, E⁵, E⁶, E⁷, E⁸, E¹⁰, E¹¹ and E¹² independently represents, on each occasion when used herein:
   (i) Q⁴;
   (ii) C₁₋₁₂ alkyl optionally substituted by one or more substituents selected from =O and Q⁵; or
      any two E¹, E², E³, E⁴, E⁵, E⁶, E⁷, E⁸, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -C(=Y)N(R²⁰)-O-R^{21a}, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹),-OP(OR²⁰)(OR²¹), -N(R²²)C(=Y)R²¹, -N(R²²)C(=Y)OR²¹, -N(R²²)C(=Y)N(R²⁰)R²¹,-NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰,-S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R^{21a} represents C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹ and R²², may be linked together to form a 4- to 20-membered ring, optionally containing one or more heteroatoms, optionally containing one or more unsaturations, and which ring is optionally substituted by one or more substituents selected from J⁶ and =O;
   each J¹, J², J³, J⁴, J⁵ and J⁶ independently represents, on each occasion when used herein:
   (i) Q⁷;
   (ii) C₁₋₆ alkyl or heterocycloalkyl, both of which are optionally substituted by one or more substituents selected from =O and Q⁸;
each Q⁷ and Q⁸ independently represents, on each occasion when used herein: halo, -CN, -N(R⁵⁰R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂N(R)⁵⁰R⁵¹, -N(R⁵²)-C(=Y^{a})-N(R⁵⁰)R⁵¹, -S(O)₂R⁵⁰, -SR⁵⁰, -S(O)R⁵⁰, C₁₋₆ alkyl (optionally substituted by one or more fluoro atoms), heterocycloalkyl, aryl or heteroaryl (which latter three groups are optionally substituted by one or more substituents selected from halo, -OR⁶⁰ and -N(R⁶¹)R⁶²);
each Y³ independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
each R⁶⁰, R⁶¹ and R⁶² independently represent hydrogen or C₁₋₆ alkyl optionally substituted by one or more fluoro atoms,
or a pharmaceutically acceptable ester, amide, solvate or salt thereof.

Among the possible compounds of Formula II, a possible preferred group is the group of compounds wherein
i) B¹, B^{1a}, B², B^{2a}, B³, B^{3a}, B⁴, B^{4a} are all of them hydrogen,
ii) R³ represents a substituted heteroaryl, preferably a substituted pyrimidinyl group, more preferably 2'-aminopyrimidinyl which is specially preferred that is linked by the 5' position of the ring to the rest of the molecule, and/or
iii) A₄ₐ represents C(R^{1b}), wherein R1^{b} represents a fragment of Formula IIIR 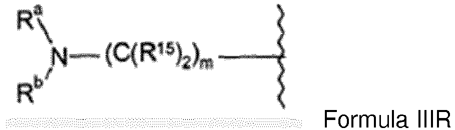 wherein m is 1 and each R¹⁵ represents hydrogen

Thus, a group of preferred compounds among those of Formula III are the compounds of Formula IIIa 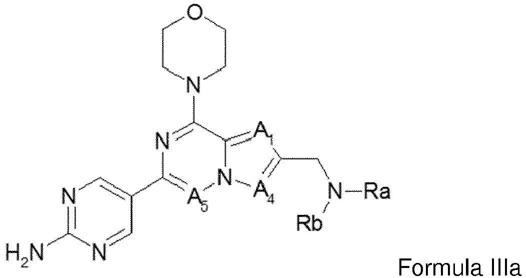 wherein
A₁ represents N or C(R¹);
A₄ represents N or C(R^{1a});
A5 represents N or C(R²);
R¹ and R² independently represents hydrogen or a substituent selected from halo, -CN,-OR^{10b}, -N(R^{10b})R^{11b}, -C(O)N(R^{10b})R^{11b}, C₁₋₁₂ (e.g. C₁₋₆) alkyl and heterocycloalkyl, which latter two groups are optionally substituted by one or more substituents selected from E³ and =O;
R^{1a} (when present) represents:
   (i) hydrogen;
   (Ü) Q¹;
   (iii) C₁₋₁₂ alkyl optionally substituted by one or more substituents selected from =O, =S, =N(R^{10a}) and Q²; or
   (iv) a fragment of formula IIIR;
   the fragment of formula IIIR represents: 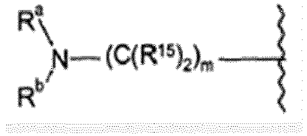
wherein:
m represents 1, 2, 3, 4, 5 or 6;
each R¹⁵ represents hydrogen, halo or C₁₋₆ alkyl optionally substituted by more substituents selected from E⁴; or
the two R¹⁵ groups may be linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E⁵;
R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
   (a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring (in which the heteroatoms are preferably selected from sulfur and, especially, nitrogen and oxygen);
   (b) comprises a linker group -(C(R^{x})₂)ₚ- and/or -(C(R^{x})₂)ᵣ-O-(C(R^{x})2)ₛ- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
   (c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
   all of which cyclic groups, defined by the linkage of R^{a} and R^{b}, are optionally substituted by one or more substituents selected from =O, =NOR^{10a} and E⁶;
each Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -C(=Y)N(R^{10a})-OR^{11c}, -OC(=Y)-R^{10a}, -OC(=Y)-OR^{10a}, -OC(=Y)N(R^{10a})R^{11a}, -OS(O)₂OR^{10a}, -OP(=Y)(OR^{10a})(OR^{11a}), -OP(OR^{10a})(OR^{11a}), -N(R^{12a})C(=Y)R^{11a}, -N(R^{12a})C=(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R^{10a}, -SR^{10a}, -S(O)R^{10a}, C₁₋₁₂ alkyl, heterocycloalkyl (which latter two groups are optionally substituted by one or more substituents selected from =O, =S, =N(R^{10a}) and E⁸), aryl or heteroaryl (which latter two groups are optionally substituted by one or more substituents selected from E⁹);
each R^{11c} independently represents C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹);
each R^{10a}, R^{11a}, R^{10b}, R^{11b} and R^{12a} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a} and R^{11a} or R^{10b} and R^{11b} may be linked together to form a 4- to 20-membered ring, optionally containing one or more heteroatoms, optionally containing one or more unsaturations, and which ring is optionally substituted by one or more substituents selected from =O, =S, =N(R²⁰) and E¹²;
each E³, E⁴, E⁵, E⁶, E⁷, E⁸, E¹⁰, E¹¹ and E¹² independently represents, on each occasion when used herein:
   (i) Q⁴;
   (ii) C₁₋₁₂ alkyl optionally substituted by one or more substituents selected from =O and Q⁵; or
any two E¹, E², E³, E⁴, E⁵, E⁶, E⁷, E⁸, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -C(=Y)N(R²⁰)-O-R^{21a}, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹),-OP(OR²⁰)(OR²¹), -N(R²²)C(=Y)R²¹, -N(R²²)C(=Y)OR²¹, -N(R²²)C(=Y)N(R²⁰)R²¹,-NR²²S(O)₂R²⁰, -NR²²S(O)₂N(R²⁰)R²¹, -S(O)₂N(R²⁰)R²¹, -SC(=Y)R²⁰, -(O)₂R²⁰, -SR²⁰,-S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R^{21a} represents C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹ and R²², may be linked together to form a 4- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
   (i) Q⁷;
   (ii) C₁₋₆ alkyl or heterocycloalkyl, both of which are optionally substituted by one or more substituents selected from =O and Q⁸;
each Q⁷ and Q⁸ independently represents, on each occasion when used herein: halo,-CN, -N(R⁵⁰R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹,-NR⁵²S(O)₂R⁵⁰, -S(O)2N(R⁵⁰)R⁵¹, -N(R⁵²)-C(=Y^{a})-N(R⁵⁰)R⁵¹, -S(O)₂R⁵⁰, -SR⁵⁰, -S(O)R⁵⁰, C₁₋₆ alkyl (optionally substituted by one or more fluoro atoms), heterocycloalkyl, aryl or heteroaryl (which latter three groups are optionally substituted by one or more substituents selected from halo, -OR⁶⁰ and -N(R⁶¹)R⁶²);
each Y³ independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
each R⁶⁰, R⁶¹ and R⁶² independently represent hydrogen or C₁₋₆ alkyl optionally substituted by one or more fluoro atoms,
or a pharmaceutically acceptable ester, amide, solvate or salt thereof.

It is particularly preferred that the TRF1 inhibitor of Formula IIIa is a compound wherein,
A₁ represents C(R¹) and R¹ is hydrogen,
A₄ represents N,
A₅ is CH,
R^{a} and R^{b} are linked together, along with the requisite nitrogen atom to which they are necessarily attached to form a 6-membered group, which 6-membered group is a saturated ring that contains nitrogen as further heteroatom and which is substituted with-S(O)₂CH₃, and
   that is particularly the compound 3-10 of international patent application WO2011089400, that is: 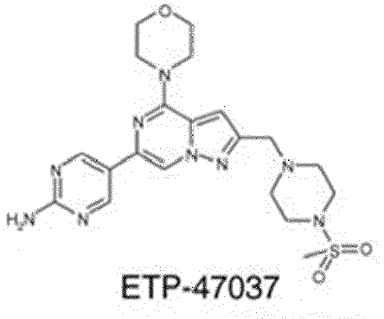 which has been named ETP-47037 herein, as in previous works of the group of the present inventors (Garcia-Beccaria *et al*., 2015).

The assays described in the Examples section of the present application have allowed to identify another compound as a TRF1 inhibitor which had not been previously described as such: the compound for Formula I 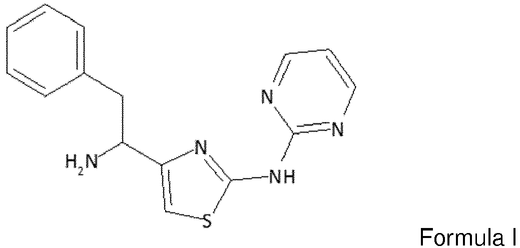 which is named ETP-50946 in the Examples below. This compound has been found to have the ability to decrease TRF1 protein levels, as the PI3K inhibitors ETP-47037 and ETP-47228 mentioned above. Thus, in another possible embodiment of the present invention, the compound which is a TRF1 inhibitor which is use in the treatment of GBM or another brain tumor is the compound of Formula I (ETP-50946).

In the assays described in Examples of the present application, the effects of these three compounds (ETP-50946, ETP-47037 and ETP-47228) in GBM cell lines and human patient-derived GSCs were checked. It was observed that the three independent compounds were able to decrease TRF1 total protein levels. Also, the present inventors demonstrated that the compounds recapitulate the findings observed with TRF1 genetic deletion, including decreased TRF1 protein levels, induction of telomere DNA damage foci or TIFs, and decreased proliferation and stemness of glioma cell lines. More importantly, these small molecules present a striking effect in blocking sphere formation (size and diameter) also in human patient-derived primary glioma stem cells, thus demonstrating the ability of these inhibitors to reduce their stem potential. The importance of this finding must be highlighted, giving the fact that the recurrence of GBM after the current treatments its due to the resistance of the glioma stem cells and their capability to recapitulate the original tumor.

Moreover, it is demonstrated in Examples included below that oral administration of TRF1 chemical modulators drastically reduces tumor growth *in vivo* in xenograft mouse models from patient-derived primary glioma stem cells. Interestingly, no signs of sickness or morbidity were detected in the xenograft models treated with TRF1 chemical inhibitors compared to the placebo group, which together with the lack of brain function phenotypes upon TRF1 genetic deletion in the brains, supports a therapeutic window for TRF1 inhibition.

Thus, it is another aspect of the present invention a composition which comprises at least a TRF1 inhibitor for use in the treatment or prevention of glioblastoma multiforme (GBM) or another brain tumor, which composition preferably also comprises one or more pharmaceutically acceptable excipient, diluent, vehicle or carrier. It can be also said that the invention refers to a method for the treatment of a glioblastoma tumor or another brain tumor by the administration of a composition which comprises at least a TRF1 inhibitor, wherein the subject is an animal suffering or having suffered from GBM or another brain tumor, which animal will be preferably a mammal, and more preferably a human being. Oral administration can be one of the possible routes of the composition; depending on the TRF1 inhibitor and its characteristics, also comprised within the present invention are other possible administration routes, such as intraperitoneal, intravenous, intracranial, and other known routes which may be suitable depending on the compound or compounds present in the composition.

The assay described herein show that the administration of a TRF1 chemical inhibitor and temozolomide, a drug currently used in the treatment of GBM, increases the effects. Therefore, it is a possible preferred embodiment of the present invention a composition which comprises at least a TRF1 inhibitor for use in the treatment or prevention of glioblastoma multiforme (GBM) or another brain tumor, which composition preferably also comprises one or more pharmaceutically acceptable excipient, diluent, vehicle or carrier, and that additionally comprises another anti-tumoral compound, preferably an anti-tumoral compound used in the treatment of glioblastoma multiforme, which compound temozolomide. The compositions which comprises a TRF1 inhibitor which acts through the Akt/PI3K pathway (such as the compounds claimed in international patent applications WO2010119264 and WO2011089400) and the compound of Formula I, for used in the treatment of glioblastoma multiforme or another brain tumor, are also comprised within the scope of the present invention.

The assays described herein also show that the combined administration of a TRF1 chemical inhibitor with radiotherapy has a synergistic effect. Therefore, it is also a preferred embodiment of the method of treatment of the invention, that is, the method for the treatment of a glioblastoma tumor or another brain tumor by the administration of a composition which comprises at least a TRF1 inhibitor, wherein the subject is an animal suffering or having suffered from GBM or another brain tumor, a method wherein the TRF1 inhibitor is administered in combination with radiotherapy.

As can be also seen in the Examples section, in order to identify additional signaling pathways that modulate TRF1 binding to telomeres, the present inventors performed a screening with a collection of antitumoral drugs, FDA approved or in clinical trials, covering several pathways. The results show that several FDA approved drugs can inhibit TRF1, also showing a decrease of TRF1 protein levels, which drugs belong to several independent families: RTK inhibitors, MEK inhibitors, ERK inhibitors, mTOR inhibitors, CDK inhibitors, HSP90 inhibitors, docetaxel and gemcitabine. Therefore, RTK inhibitors, MEK inhibitors, ERK inhibitors, mTOR inhibitors, CDK inhibitors, HSP90 inhibitors, PLK inhibitors, docetaxel and gemcitabine can be also possible compounds that can be used as TRF1 inhibitors for the treatment of GBM or other brain tumors and are among the TRF1 inhibitors that can be comprised in the compositions of the present invention inhibitor for use in the treatment or prevention of glioblastoma multiforme or another brain tumor. HSP90i (and, particularly, geldanamycin), docetaxel and gemcitabine were identified as the most potent compounds, so that they can be considered preferred embodiments of TRF1 inhibitors for the treatment of GBM and/or other brain tumors, as well as other compounds which have been identified as TRF1 inhibitors by the present inventors, such as alisertib (Aurorai), dasatinib (RTKi), GSK461364 (PLKi), KU-0063794 (mTORi), SCH772984 (MEKi) and flavopiridol (CDKi).

As it is known that the bad prognosis of glioblastoma is mainly due to the existence of a group of cells with stem like properties, also known as glioma-stem like cells (GSCs), the present inventors decided to perform drug combination studies in glioblastoma stem cells in order to design new combinatory treatments based on TRF1 inhibition, which could effectively block resistance of individual drugs. In particular, it was checked if PI3K inhibitors (already known to modulate TRF1) could show synergic effects with any of the groups of compounds which were found to also inhibit TRF1 in the assays of the present inventors, namely MEK inhibitors, ERK inhibitors, mTOR inhibitors, CDK inhibitors, HSP90 inhibitors docetaxel and gemcitabine. All combinations could be considered effective, so that it can be considered comprised within the present invention a composition which comprises at least a TRF1 inhibitor for use in the treatment or prevention of glioblastoma multiforme or another brain tumor, wherein the composition comprises at least a first and a second TRF1 inhibitor and at least one of the TRF1 inhibitor is an inhibitor of TRF1 which decreases TRF1 protein levels, possible embodiments being those one where at least one TRF1 inhibitor is selected of the group of an RTK inhibitor, a MEK inhibitor, an ERK inhibitor, an mTOR inhibitor, a CDK inhibitor, an HSP90 inhibitor, docetaxel and gemcitabine, with preference for MEK inhibitors, ERK inhibitors, mTOR inhibitors, CDK inhibitors, HSP90 inhibitors docetaxel and gemcitabine, being preferred that the other TRF1 inhibitor is a PI3K inhibitor, which PI3K inhibitor can be selected, among others, from the group of PI3K inhibitors claimed in international patent applications WO2010119264 and WO2011089400.

Interestingly PI3K inhibitors (and, specifically, ETP-47037, the one used in the assays) showed a significant synergic effect with MEK inhibitors, ERK inhibitors, mTOR inhibitors, CDK inhibitors, HSP90 inhibitors docetaxel and gemcitabine, opening new therapeutic opportunities to further apply these combinations in human patients. The combination of PI3K inhibitors, particularly PI3Kα inhibitors, and very specially ETP-47037, with an HSP90 inhibitor, docetaxel or gemcitabine is herein provided for the treatment of any cancer type.

As it is described in the Examples section, some compounds were already known in the field of TRF1 modulators, but others not. It can be also considered that RTK inhibitors, MEK inhibitors, ERK inhibitors, mTOR inhibitors, CDK inhibitors, HSP90 inhibitors, docetaxel and gemcitabine has been identified as TRF1 inhibitors thanks to the studies of the present inventors. This shows the potentiality of the methodology used in the present application to identify compounds as potential drugs to be used or further tested for GBM or other brain tumors. Therefore, it can be considered that it is also an aspect of the present invention a method for identifying a compound as a candidate for use in the prevention or treatment of glioblastoma, which comprises a step wherein it is determined that the compound inhibits or decreases TRF1 activity. As in the assays of the present application, the decrease of TRF1 activity can be assessed by verifying that the compound downregulates TRF1 protein levels, what can be done, for instance, by:
a) adding the compound to a culture of cells,
b) quantifying protein levels in the cells subjected to the effect of the compound by a method which is selected from the group of:
   a. determining total TRF1 protein levels,
   b. quantifying TRF1 foci by immunoassays, or
   c. combinations thereof;
c) comparing the data obtained with the cells subjected to the effect of the compound with the data obtained with control cells not having contacted the compound, and
d) concluding that the compound downregulates TRF1 protein levels after verifying that the TRF1 protein levels obtained with the cells subjected to the effect of the compound with the data obtained with control cells not having contacted the compound.

The determination of TRF1 downregulation can be done determining protein levels in a culture of cells of a previously established glioblastoma cell line or cells extracted from a glioblastoma patient, what allows, as possible additional confirmation, verifying that the compound reduces proliferation of the cells having contacted the compound. Additional confirmatory evidences can also be sought, such as assessing that the compound induces DNA damage, as found by the present inventors for TRF1 compound inhibitors, and/or additionally verifying that the compound is able to reduce stemness in a in a culture of cells of a previously established glioblastoma cell line or cells extracted from a glioblastoma patient. The present invention will be explained in more detail through the following Examples and Figures.

### EXAMPLES

The assays described in the following Examples were carried out with the following materials, compounds and methodologies:

### - Mice experimentation

### Mice generation

For the GBM experiments, Nestin-Tva (Holland *et al.,* 1998; Hambardzumyan *et al.,* 2011), *Cdkn2a*^{*-*/*-*} (Serrano *et al.,* 1996) and *Trf1*^{*lox*/*lox*} (Martinez *et al.,* 2009) mice were crossed to obtain the *Trf1*^{*lox*/*lox*}; Nestin-Tva; *Cdkn2a*^{*-*/*-*}; or *Trf1*^{*+*/*+*}; Nestin-Tva; *Cdkn2a^{-l-}* mouse models. These mouse models were further crossed with a mouse strain carrying ubiquitously expressed, tamoxifen-activated recombinase, hUBC-CreERT2 (Ruzankina *et al.,* 2007) to generate *Trf1*^{*lox*/*lox*}; hUBC-CreERT2; Nestin-Tva; *Cdkn2a*^{-/-} and *Trf1*^{*+*/*+*}; hUBC-CreERT2 Nestin-Tva; *Cdkn2a^{-l-}* mice. For xenograft experiments, athymic nude females were obtained from Harlan (Foxn1^{nu/nu}).

### Mice maintenance

All mice were maintained at the Spanish National Cancer Centre (CNIO) under specific pathogen-free conditions in accordance with the recommendations of the Federation of European Laboratory Animal Science Associations (FELASA). All animal experiments were approved by the Ethical Committee (CEIyBA) from the CNIO and performed in accordance with the guidelines stated in the International Guiding Principles for Biomedical Research Involving Animals, developed by the Council for International Organizations of Medical Sciences (CIOMS). Along with those guidelines, mice were monitored in a daily or weekly basis and they were sacrificed in CO₂ chambers when the human endpoint was considered.

Mice were maintained on a 12-hour light/12-hour dark cycle. During light cycle, white light was provided by fluorescent lamps (TLD 36W/840 and TLD58W/840, Philips). Mice had free access to water and standard chow diet (18% of fat-based calorie content, Harlan Teckland 2018). *Trf1*^{*lox*/}*^{lox} or Trf1*^{*+*/*+*}; hUBC*-*CreERT2 mice received intraperitoneal injections of tamoxifen (2 mg/injection, 4-6 injections) for short-term experiments or they were fed *ad libitum* with tamoxifen containing diet for long-term experiments.

### Mice genotyping

Mice genotyping was performed by Transnetyx private company (Cordova, TN 38016), except for tamoxifen treated mice. In these mice, *Trf1* deletion was assessed by standard PCR.

### Transnetyx genotyping

Transnetyx uses a RT-qPCR based system and Taqman probe technology to measure the presence or absence of a desire sequence. The following probes were used to assess the different mouse genotypes:
**CRE** - used to test for Cre; targeted to sequence within the Cre gene coding region.
   Forward Primer Sequence: TTAATCCATATTGGCAGAACGAAAACG (SEQ ID NO:1)
   Reverse Primer Sequence: CAGGCTAAGTGCCTTCTCTACA (SEQ ID NO:2)
   Probe Sequence: CCTGCGGTGCTAACC (SEQ ID NO:3)
**Terf1-3 MD** - used to test for *Trf1^{lox}*; targeted to a sequence unique to the *Trf1* recombined allele.
   Forward Primer Sequence: GCTATACGAAGTTATTCGAGGTCGAT (SEQ ID NO:4)
   Reverse Primer Sequence: GGTGGCGGCCGAAGT (SEQ ID NO:5)
   Probe Sequence: CTCTAGAAAGTATAGGAACTTC (SEQ ID NO:6)
**Terf1-3 WT** - used to test for *Trf1+;* targeted to sequence at the 3' loxP insertion site.
   Forward Primer Sequence: GAGACGGCGCGAAACC (SEQ ID NO:7)
   Reverse Primer Sequence: GCGGGAGCCAGGACTTC (SEQ ID NO:8)
   Probe Sequence: CCGCTTCCTGTTTGCTG (SEQ ID NO:9)
**Tva-** used to test the presence of the Tva transgene
   Forward Primer Sequence: CACAGAGGCTCCCACTGT (SEQ ID NO:10)
   Reverse Primer Sequence: ATGCGGCCGTGATTCCT (SEQ ID NO:11)
   Probe Sequence: CTGGACGTGCTCTGCC (SEQ ID NO:12)
**p16 WT-** used to test the presence of the p16 allele
   Forward Primer Sequence: CGAGGACCCCACTACCTTCT (SEQ ID NO:13)
   Reverse Primer Sequence: CCGCTCTTGGGCCAAGT (SEQ ID NO:14)
   Probe Sequence: CAGGCATCGCGCACAT (SEQ ID NO:15)
**p16 KO-** used to test the absence of the p16 allele
   Forward Primer Sequence: CTCTACTTTTTCTTCTGACTTTTCAGGTG (SEQ ID NO:16)
   Reverse Primer Sequence: CCCCTACCCGGTAGAATTGAC (SEQ ID NO:17)
   Probe Sequence: ATGATGATGGGCCCCCGTC (SEQ ID NO:18) *PCR genotyping*

After tamoxifen treatment, Cre mediated *Trf1* deletion was assessed by the following primers:
Forward Primer (E1-popout): 5' ATAGTGATCAAAATGTGGTCCTGGG 3' (SEQ ID NO:19)
Reverse Primer (SA-R1): 5' GCTTGCCAAATTGGGTTGG 3' (SEQ ID NO:20)

With this pair of primers, the excised *Trf1*^{Δ} allele gives an amplified band of 0.48 kb whereas the unexcised *Trf1*^{lox} allele gives an amplified band of 1.5 kb. *Trf1*⁺ allele gives a band of 1.4 Kb.

### Generation of mouse models with brain tumors

The RCASATv-a system used in this work has been previously described (Holland *et al.,* 1998; Hambardzumyan *et al.,* 2009). Adult mice (4.5-6 weeks old) were injected in the SVZ with 1 µl of DF-1 chicken fibroblasts producing RCAS-Cre, RCAS-PDGFB-HA, RCAS-PDGFA-MYC, RCAS-GFP-shNf1 or RCAS-RFP-shp53 as described at a concentration of 200.000 cells/µl, with the exception of RCAS-Cre producing cells that were injected at a concentration of 600000 cell/µl. All mice were monitored and killed whenever they presented symptoms of brain tumor development. For all studies the present inventors used both male and female mice.

### Intracranial cell transplantation into syngeneic mice

Spheres were dissociated using a 200 µl pipette and were resuspended in a concentration of 100.000 cells/µl. From these aliquots, 1 µl was injected into the brain of adult syngeneic mice. All mice were monitored and killed whenever they presented symptoms of brain tumor development.

### Xenografts experiments

h676 and h543 patient-derived GSCs were dissociated using a 200 µl pipet and resuspended in NeuroCult medium and matrigel in a 1:1 ratio in a concentration of 1000 cell/µl. Nude mice (athymic Nude-Foxn1^{nu/nu} from Harlan) were injected subcutaneously with 100 µl of the cell preparation. ETP-47037 (or vehicle) was orally administrated at a concentration of 75 mg/kg 5 days per week (see also section 8), starting one week after cell injection. The vehicle consisted in 10% N-methyl-2-pyrrolidone (NMP, Sigma Aldrich) and 90% poly ethylene glycol (PEG, Sigma Aldrich). Mice were weighted and tumors were measured every 2-4 days. Tumor area was determined by the following equation: A = π * (a/2) * (b/2), were a and b are tumor length and width respectively.

### Cognitive tests

To evaluate memory skills, mice were tested by the object recognition test (Bernardes de Jesus et al., 2012). A total of two 3 different objects were used for the test, two identical objects (A) and a third different object (B). In day 1, mice were placed in a box with the two identical objects for 10 minutes. In day 2, one of the objects was replaced by object B and the mice were again placed for ten minutes and recorded with a camera. Analysis was made by calculating time spent with object B divided with time spent with (A+B).

To check the ability to smell, mice were tested by the buried food test (Yang & Crawley, 2009). Mice were fasted for 24 h and they were placed in a cage with a buried pellet food. Analysis was made by calculating the percentage of success and the time spent to find the food pellet.

To measure coordination and balance, mice were tested in a Rotarod apparatus (model LE 8200) and with the tightrope test. In the rotarod test the present inventors measured the time mice could stay on the rod. In the tightrope test, the present inventors evaluate the ability of the mice to stay in the rope without falling, and the present inventors considered a "success" if mice were able to stay more than one minute.

### -Cell culture

Human astrocytes (HA) (ScienceCell, Cat#1800), U251 cells (kindly provided by Eric Holland's lab), U87 cells (ATCC Cat#HTB-14), T98G cells (kindly provided by Eric Holland's lab), 293T cells and DF1 chicken fibroblast (ATCC Cat# CRL-12203) were grown at 37°C in 10% FBS (GIBCO) containing DMEM (GIBCO). Mice glioma and neural stem cells and patient-derived glioma stem cells (h543 and h676, described by Ozawa *et al.,* 2014, and Rohle *et al*., 2013) were cultured in neurosphere medium from NeuroCult (Stem Cell Technologies Inc, Vancouver, Canada) supplemented with 10 ng/ml EGF (Gibco), 20 ng/ml basic-FGF (RD Systems) and 1mg/ml Heparin (Stem Cell Technologies).

### Cell transfection

DF1 cells were transfected with the RCAS-Cre, RCAS-PDGFB-HA, RCAS-PDGFA-MYC, RCAS-GFP-shNf1 or RCAS-RFP-shp53 viral plasmids (see Hambardzumyan *et al.,* 2006 for details about its construction) using Fugene 6 Transfection reagent (Roche), accordingly to manufacture protocol. pGIPZ lentiviral TRF1 shRNAs and pGIPZ-scrambled shRNA were introduced in the U251 glioma cell line using standard lentiviral infection procedures.

### Neural Stem Cell (NSC) and Glioma Stem Cell (GSC) isolation

NSCs were obtained by neonatal brain digestion with papain (Worthington). GSCs were extracted from mice tumors using the same procedure. As described above, both mice NSC and GSC were cultured in neurosphere medium from NeuroCult (Stem Cell Technologies Inc, Vancouver, Canada) supplemented with 10 ng/ml EGF (Gibco), 20 ng/ml basic-FGF (RD Systems) and 1mg/ml Heparin (Stem Cell Technologies). Cells were grown in NeuroCult medium suspension or in adhesion in laminin (Life Technologies) coated plates.

### Neurosphere formation assays

Spheres were dissociated into single cells and seeded at a density of 50, 100, 200 and 400 cells/well in a 96 well plate. Neurosphere number was assessed after 7 days. Pictures were taken using Nikon Eclipse Ti-U microscope and neurosphere diameter was measured using NIS Elements BR software.

### Irradiation and temozolomide

Cells were irradiated with 6 Gy using the irradiation apparatus MDS Nordion Gamma Cells 1000. Cells were treated with temozolomide at a concentration of 500 µM or 1000 µM for three days.

### -Histopathology, Immunofluorescence and immunohistochemistry analysis

### Histopathological analysis

Histopathological analysis was performed in paraffin embedded tissue sections stained with hematoxylin and eosin (H&E), with the assistance of the CNIO Histopathology Unit and Juana Maria Flores from the *Universidad Complutense de Madrid* (UCM).

### Immunofluorescence analyses in cells and tissue sections

For immunofluorescence analyses, cells were plated in a proper density in cell culture µCLEAR plates (Greiner) and fixed in 4% formaldehyde in PBS. Cells were permeabilized with 0.25% Triton in PBS and blocked with 5% BSA in PBS. Tissue sections were fixed in 10% buffered formalin (Sigma) and embedded in paraffin. After deparaffinization and citrate antigen retrieval, sections were permeabilized with 0.5% Triton in PBS and blocked with 1%BSA and 10% Australian FBS (GENYCELL) in PBS. The antibodies were applied overnight in antibody diluents with background reducing agents (Invitrogen).

Primary antibodies: anti-Nestin (BD Pharmigen: Cat#556309, RRID AB_396354), anti-Rap1 (BL735, Bethrridyl), rat polyclonal anti-TRF1 (homemade), anti-TRF1 (BED5, Cell Signaling:Cat#3529, RRID: AB_2201452), anti-yH2AX Ser139 (05-636, Millipore), anti-53BP1 (Novus Biologicals Cat#NB100-304, RRID AB_2314619), anti-HA tag (Cell Signaling Technology, 6E2: Cat#2367, RRID:AB_2314619), anti-Myc-tag (9E10, Santa Cruz, CatSC-40)), anti-Ki67 (Master diagnostic Cat#003110QD), anti-p-RPA32 (S4/S8) (Bethyl, Cat# A300-245A, RRID: AB_210547).

Immunofluorescence images were obtained using a confocal ultraspectral microscope (Leica TCS-SP5) or the Opera High Content Screening (HCS) system (Perkin Elmer). Quantifications were performed with Definiens software.

### Immunohistochemistry analyses in tissue sections

Immunohistochemistry stainings were performed by the CNIO Histopathology Unit following standard protocols. Antibodies used for immunohistochemistry included those raised against: γH2AX Ser 139 (Millipore), Ki67 (Master diagnostica), HA tag (Cell Signaling Technology), p53 (POE316A/E9, homemade), p21 (291H/B5, homemade), AC3 (Cell Signaling Technology), NF-1 (Santa Cruz Biotechnology).

Pictures were taken using Olympus AX70 microscope. The percentage of positive cells was identified by eye and the areas were calculated by CellSens Entry software.

### -Western-Blotting

Nuclear protein extracts were obtained using Nuclear Cytosolic Fractionation Kit (Biovision) and protein concentration was determined using the Bio-Rad DC Protein Assay (Bio-Rad). Up to 15 µg of protein per extract were separated in SDS-polyacrylamide gels by electrophoresis. After protein transfer onto nitrocellulose membrane (Whatman), the membranes were incubated with the indicated antibodies. Antibody binding was detected after incubation with a secondary antibody coupled to horseradish peroxidase using chemiluminescence with ECL detection KIT (GE Healthcare)

Primary antibodies: anti-TRF1 (BED5, Cell Signaling: Cat#3529, RRID: AB_2201452), anti-TRF1 (TRF-78, Abcam: Cat# ab10579, RRID: AB_2201461), anti-TRF2 (Novus Biologicals, Cat# NB110-57130, RRID: AB_844199), anti-RAP1 (BL735 Bethyl: Cat#A300-306A, RRID: AB_162721) anti-SMC-1 (Bethyl), anti-βACTIN (Sigma).

Quantifications: Protein-band intensities were measured with ImageJ software and normalized against the loading control.

### -In situ hybridization

### Telomere measure by Quantitative Fluorescence In situ Hybridization (qFISH)

For quantitative telomere fluorescence in situ hybridization (Q-FISH) paraffin-embedded sections were deparaffinized and fixed with 4% formaldehyde, followed by digestion with pepsine/HCI and a second fixation with 4% formaldehyde. Slides were dehydrated with increasing concentrations of EtOH (70%, 90%, 100%) and incubated with the telomeric probe for 3.5 min at 85ºC followed by 2h RT incubation in a wet chamber. In the final steps, the slides were extensively washed with 50% formamide and 0.08% TBS-Tween. Analysis was performed by Definiens software.

### Immuno-FISH

Tissue samples were fixed in 4% formaldehyde and permeabilized with 0.5% Triton in PBS. Telomeric FISH was performed as described in section 5.1 omitting the pepsin digestion step. After washing, immunofluorescence staining was performed and described in section 3.1.

### FISH analysis on metaphase spreads

For metaphase preparation, cells were grown overnight in the presence of 0.1 µg/ml colcemide. Cells were incubated with hypotonic solution (0.4% KCI, 0.4% Sodium citrate) followed by cold methanol/acetic acid (3:1) fixation. On the final steps, cells were spread on glass slides and telomeric FISH was performed as described in 5.1. Analysis of MTS signals was performed by superposing the FISH telomere image and the DAPI image.

### -Real-time qPCR

Total RNA from cells was extracted with the RNeasy kit (QIAGEN) and reverse transcribed was using the iSCRIPT cDNA synthesis kit (BIO-RAD) according to manufacturer's protocols.

Quantitative real-time PCR was performed with the QuantStudio 6 Flex (Applied Biosystems, Life Technologies) using Go-Taq qPCR master mix (Promega) according to the manufacturers protocol. All values were obtained in triplicates. Primers for mouse and human samples are listed below.

Mouse primers:
TRF1-F 5'-GTCTCTGTGCCGAGCCTTC-3' (SEQ ID NO:21)
TRF1-R 5'-TCAATTGGTAAGCTGTAAGTCTGTG-3' (SEQ ID NO:22)
TBP1-F 5'-ACCCTTCACCAATGACTCCTATG-3' (SEQ ID NO:23)
TBP1-R 5'-TGACTGCAGCAAATCGCTTGG-3' (SEQ ID NO:24)
TRF2-F 5'-AGAGCCAGTGGAAAAACCAC-3' (SEQ ID NO:25)
TRF2-R 5'-ATGATGGGGATGCCAGATTA-3' (SEQ ID NO:26)
POT1A-F 5'-AAACTATGAAGCCCTCCCCA-3' (SEQ ID NO:27)
POT1A-R 5'-CGAAGCCAGAGCAGTTGATT-3' (SEQ ID NO:28)
RAP1-F 5'-AAGGACCGCTACCTCAAGCA-3' (SEQ ID NO:29)
RAP1-R 5'-TGTTGTCTGCCTCTCCATTC-3' (SEQ ID NO:30)
TPP1-F 5'-ACTTGTGTCAGACGGAACCC-3' (SEQ ID NO:31)
TPP1-R 5'-CAACCAGTCACCTGTATCC-3' (SEQ ID NO:32)
TIN2-F 5'TCGGTTGCTTTGCACCAGTAT-3' (SEQ ID NO:33)
TIN2-R 5'GCTTAGCTTTAGGCAGAGGAC-3' (SEQ ID NO:34)

Human primers:
TRF1-F 5'-TTCTAATGAAGGCAGCGGCA-3' (SEQ ID NO:35)
TRF1-R 5'-GTTGCTGGGTTCCATGTTGC-3' (SEQ ID NO:36)
α-TUB-F 5'-AGTGAAAACAATCTAACCAGAAA-3' (SEQ ID NO:37)
α-TUB-R 5'-AGGCCCGTGAAGATATG-3' (SEQ ID NO:38)
TRF2-F- 5'-GACCTTCCAGCAGAAGATGCT-3' (SEQ ID NO:39)
TRF2-R- 5'-GTTGGAGGATTCCGTAGCTG-3' (SEQ ID NO:40)
POT1-F- 5'-TGGGTATTGTACCCCTCCAA-3' (SEQ ID NO:41)
POT1-R- 5'- GATGAAGCATTCCAACCACGG-3' (SEQ ID NO:42)
RAP1-F- 5'-CGGGGAACCACAGAATAAGA-3' (SEQ ID NO:43)
RAP1-R- 5'-CTCAGGTGTGGGTGGATCAT-3' (SEQ ID NO:44)
TPP1-F-5'-CCCGCAGAGTTCTATCTCCA-3' (SEQ ID NO:45)
TPP1-R-5'-GGACAGTGATAGGCCTGCAT-3' (SEQ ID NO:46)
TIN2-F-5'-GGAGTTTCTGCGATCTCTGC-3' (SEQ ID NO:47)
TIN2-R-5'-GATCCCGCACTATAGGTCCA-3' (SEQ ID NO:48)

### -PCR

DNA of cells and tissue samples was extracted using Phenol:Chloroform:lsoamyl:Alcohol (Sigma). The present inventors determined Cre-mediated recombination as described above.

### -TRF1 chemical modulators

The different compounds used in these assays described below were obtained from the Experimental Therapeutics Programme (ETP) at CNIO. ETP-47228, ETP-47037 chemical compounds have been previously described (García-Beccaria *et al.,* 2015). ETP-50946 (the compound of Formula I) is the result of the enantiomeric separation of a racemic compound included into a Kinase Inhibitor Library sourced from BioFocus (Galapagos, Belgium).

For in vitro studies, ETP-47228 ETP-47037 and ETP-50946 were dissolved in DMSO at a final concentration of 10 or 5 mM. Cells were treated at a concentration of 10 µM for 24 h or 48 hr.

For oral administration, ETP-47037 was dissolved in 10% N-methyl-2-pyrrolidone (NMP, Sigma Aldrich) and 90% polyethylene glycol (PEG, Sigma Aldrich) at a concentration of 75 mg/kg.

The novel TRF1 modulators identified in the screening of compounds approved by the FDA or in clinical trials are the following:
HSP90i (Geldanamycin: CAS 30562-34-6)
Docetaxel. CAS 114915-14-9
Gemcitabine. CAS 103882-84-4
Aurorai (Alisertib: CAS 1028486-01-2)
RTKi (Dasatinib: CAS 302962-49-8)
PLK1i (GSK461364: CAS 1000873-97-1)
mTor1/2i (KU-0063794: CAS 938440-64-3)
ERKi (SCH772984: CAS 942183-80-4)
MEKi (Selumetinib: CAS 606143-52-6)
CDKi (Flavopiridol: CAS 146426-40-6)

### -Tissue microarray (TMA)

The TMAs TA- 371 and TA-438 where obtained from the CNIO Biobank. The use of this samples has been approved by the Ethical Committee (CEI). TMA acquisition was performed with a TCS SP5 confocal microscope (Leica Microsystems) equipped with Leica HCS-A and the custom-made iMSRC software (Carro *et al.,* 2015). Final images were acquired with a 40x 1.2 N.A. oil immersion Objective.

### -Quantification and statistical analysis

Survival data were analyzed by Kaplan Meier survival curves, and comparisons were performed by Log Rank test. Statistical analysis was performed using GraphPad Prism 5.03. Comparison of the percentage of mice with tumors was performed by Chi-Square test.

Immunofluorescence quantifications were performed with Definiens software and immunohistochemistry quantifications were performed by direct cell counting. Western Blot protein-band intensities were measured with ImageJ software and normalized against the loading control. Unpaired Student's t test (two-tailed) was used to determine statistical significance. P values of less than 0.05 were considered significant. *p<0.05, **p<0.01, ***p<0.001. Statistical analysis was performed using Microsoft® Excel 2011.

### - Example 1. Effects of Trf1 genetic deletion or knockdown: Mouse models of GBM and GBM human cells

### 1.1. TRF1 is overexpressed in different mouse GBM subtypes

In order to determine if TRF1 could be a promising target for the treatment of GBM, the group of the present inventors first generated various mouse models of GBM using the RCAS-Tva system, where RCAS is the viral vector that will specifically infect those cells expressing the Tva receptor. In our model, Tva receptor is expressed under the promotor of Nestin, specific for brain Neural Stem Cells (NSCs). Thus, with this system the group of the present inventors generated different GBM models by specifically targeting Nestin-expressing cells with RCAS vectors carrying different oncogenic insults in Nestin-Tva transgenic mice (Fig. 1A). In particular, the group of the present inventors generated different GBM subtypes by either overexpressing PDGFB or PDGFA in a *Cdkn2a* null background or by knocking down Nf1 and p53 in a wild-type background (Fig. 1B and 1C). PDGFA overexpression results in proneural-like GBMs, while PDGFB and sh-Nf1 sh-p53 induced glioblastomas with a mesenchymal signature (Ozawa *et al.,* 2014) (Fig. 1B)

Interestingly, *Trf1* mRNA levels were significantly upregulated in the three GBM subtypes, with a more prominent upregulation in the case of PDGFA- and PDGFB-induced GBM (Fig. 2A). It was also observed significant overexpression of TRF1 protein levels in all GBM subtypes by using immunofluorescence analysis, and again the PDGFB- and PDGFA-induced tumors were the ones with the highest TRF1 protein over-expression compared to the normal tissue (Fig. 2B). TRF1 protein upregulation was validated by western blot in PDGFB induced tumors compared to normal tissue (Fig. 2C).

Next, the present inventors addressed whether other shelterin components, namely TRF2, RAP1, POT1, TPP1 and TIN2 (Liu *et al.,* 2004), were also upregulated in mouse GBM models. TPP1, POT1 and TIN2 mRNA levels were slightly higher in the three GBM subtypes by RT-qPCR, but the differences did not reach statistical significance with the exception of TIN2 upregulation in PDGFB-induced GBM and TPP1 upregulation in PDGFA-induced tumors (Fig. 3A). RAP1 was significantly downregulated in PDGFB and PDGFA-induced tumors and TRF2 did not change in any of the GBM subtypes (Fig. 3A). To assess whether higher TRF1 levels were the consequence of longer telomeres in the tumors compared to the normal tissue, the present inventors measured telomere length by quantitative telomere FISH (Q-FISH) in both tumor sections and the corresponding normal brain tissue from the different mouse GBM subtypes. No significant differences were found in telomere length between tumors and non-tumoral tissue in any of the GBM subtypes (Fig. 3B). This is in agreement with previous findings showing that high TRF1 levels in pluripotent and adult stem cells are uncoupled from telomere length (Marion *et al.,* 2009; Tejera *et al.,* 2010; Schneider *et al.,* 2013).

It was next studied whether TRF1 levels correlated with the well-known stem cell markers SOX2, NESTIN, CD133 and c-MYC by using the Gliovis data portal for analysis of GBM expression datasets (Bowman *et al.,* 2017). It was found a positive correlation between TRF1 levels and all the stem cell markers with the exception of c-Myc (Fig. 4), although TRF1 was positively correlated with the c-Myc modulator USP13 (Fig. 4) (Fang *et al.,* 2016). These findings suggest that TRF1 overexpression in GBM is not the simple consequence of longer telomeres in these tumors, but instead may reflect on their high cancer stem cell nature, as TRF1 is upregulated in stem cells and pluripotent stem cells (Schneider *et al.,* 2013; Lathia *et al.,* 2015). In summary, all three GBM subtypes preferentially overexpressed TRF1 in a manner that is independent of telomere length, and this over-expression is higher in the PDGFB-induced GBM.

### 1.2. Effects of Trf1 deletion in tumor initiation

### 1.2.1. Trf1 deletion impairs tumor initiation in PDGFB and PDGFA induced GBM

The group of the present inventors next set to genetically validate *Trf1* as a potential anticancer target in the different GBM subtypes studied here. As PDGFB-induced GBM showed the highest TRF1 overexpression, the inventors first studied the impact of *Trf1* abrogation both in tumor initiation and tumor progression in this model. To this end, they crossed Nestin-Tva; *Cdkn2a^{-l-}* mice with *Trf1* inducible knockout mice (Martinez *et al.,* 2009) to obtain Nestin-Tva; *Cdkn2a*^{*-*/*-*}; *Trf1*^{+/+} or Nestin-Tva; *Cdkn2a*^{*-*/*-*}; *Trf1*^{lox/lox} mice (Fig. 5A). Then, they injected intracranially into the subventricular zone (SVZ) of adult mice (4.5-6 weeks) RCAS-PDGFB producing DF-1 cells together with RCAS-Cre producing DF-1 cells in a 1:3 ratio, meaning that the number of RCAS-Cre producing cells was 3 times higher than the RCAS-PDGFB producing cells. This strategy allows the overexpression of PDGFB in glial progenitors simultaneously with Cre-mediated *Trf1* excision specifically in these cells (Fig. 5A and 5B), allowing assessment of the impact of *Trf1* abrogation on tumor initiation.

Mice were monitored every two days for any signs of brain tumor development and were sacrificed at human endpoint. As expected, PDGFB overexpression in the context of *Cdkn2a* deficiency induced tumors in approximately 4-5 weeks after intracranial injection (Ozawa *et al.,* 2014) (Fig. 6A). Strikingly, even in this setting of fast-growing tumors, mice with brain-specific *Trf1* deletion showed an increased survival of 80% compared to the controls (Fig. 6A). Post-mortem GBM analysis revealed that all the tumors were histologically identical (Fig. 6B). More importantly, immunofluorescence and PCR analysis of TRF1 showed that all the tumors in *Trf1*-deleted brains were escapers, as they showed normal TRF1 expression (Fig. 6C and 6D). Telomere Q-FISH analysis also revealed that all the tumors had the same telomere length (Fig. 6E). The fact that no tumors lacking TRF1 expression were found suggests that TRF1 is essential for PDGFB-induced GBM initiation.

In order to study the cellular and molecular effects of *Trf1* deletion in GBM initiation, the present inventors next performed comparative histopathological and molecular analyses at earlier time points, when the control mice started to dye from GBM (i.e., 45 days after tumor induction) (Fig. 7A). At this time point, 91% of *Trf1*^{+/+} mice were affected by brain tumors, while only 6% of *Trf1*^{lox/lox} mice were affected (Fig. 7B). Further histological analysis revealed a significant difference in tumor size between both genotypes, with *Trf1*^{lox/lox} tumors being almost undetectable by H&E staining in most of the cases (Fig. 7C). To further confirm these findings, it was next performed immunohistochemistry analysis of HA-tag, which specifically marks PDGFB expressing cells in this model. Again, HA-tag positive areas were significantly smaller in *Trf1*^{lox/lox} mice compared to the controls (Fig. 7D). Finally, it was observed a high proliferation index in *Trf1*^{+/+} tumors, while *Trf1*^{lox/lox} brains showed very few proliferating cells (Fig. 7E), indicating impaired tumor growth by *Trf1* deletion.

Importantly, similar results were obtained in the PDGFA induced mouse model of GBM. Similarly to the PDGFB model that the group of the present inventors had already described, they injected RCAS-PDGFA producing DF-1 cells together with RCAS-Cre producing DF-1 cells into the SVZ of adult mice (4.5-6 weeks) in a 1:3 ratio (Fig. 8A). In this case, *Trf1*^{lox/lox} mice showed a highly significant 65% increase in survival compared to *Trf1*^{+/+} mice (Fig. 8B). In particular, by day 150 after tumor induction around 75% of *Trf1*^{+/+} mice had already died from GBM, while only 10% of *Trf1*^{lox/lox} mice were affected (Fig. 8C). Taken together, the results obtained with two independent mouse models of glioblastoma suggest that *Trf1* deletion reduces the number of progenitor cells capable of initiating GBM, and thus tumors appear much later.

### 1.2.2. Trf1 abrogation leads to telomere damage and reduced stemness in NSCs

To further study how *Trf1* deletion impairs tumor initiation, the present inventors moved to an *in vitro* system using isolated neural stem cells (NSCs). Of note, NSCs are located in the SVZ and express Nestin. Thus, these cells were the targets of the RCAS vectors when the intracranial injections into *Nestin-Tva* mice were performed. To this end, NSCs were isolated from both *Trf1*^{+/+} and *Trf1*^{*lox*/lox}; Nestin-Tva; *Cdkn2a*^{*-*/*-*} newborns to further infect these cells with the RCAS-Cre vector (Fig. 9A). In particular, the group of the present inventors transduced two independent lines of brain-isolated *Trf1*^{+/+} as well as eight independent lines of *Trf1*^{lox/lox} primary NSCs with the supernatant of DF-1 cells producing RCAS-Cre virus in order to induce *Trf1* deletion. *Trf1* deletion at mRNA and protein levels was confirmed by RT-qPCR and immunofluorescence, respectively (Fig. 9B and 9C).

It has been previously described that *Trf1* genetic deletion induces a persistent DDR response located at telomeres in both fibroblasts and epithelial cells (Martinez et al., 2009). To address whether *Trf1* deletion also leads to DNA damage in NSCs, the group of the present inventors quantified 53BP1 and γH2AX levels by immunofluorescence in *Trf1-*defficient NSCs compared to *Trf1*^{+/+} controls. It was found that the levels of both γH2AX and 53BP1 were significantly higher in *Trf1*^{lox/lox} NSCs compared to *Trf1*^{+/+} controls (Fig. 10A and 10B). In addition, double immunofluorescence staining of γH2AX and the telomeric protein RAP1 showed increased DNA damage specifically located at telomeres (the so-called telomere induced foci or TIFs) in the case of *Trf1*-defficient NSCs compared to *Trf1*^{+/+} controls (Fig. 10C), indicating that telomeres are uncapped as the consequence of *Trf1* deletion, leading to a DDR at telomeres.

Also, it is known that TRF1 expression is upregulated and it is essential for both adult stem cells and pluripotent stem cells (Schneider *et al.,* 2013). Thus, the present inventors next studied the impact of *Trf1* deletion on the stem cell potential of NSCs. To this end, the present inventors performed a neurosphere formation assay by mechanically disaggregating NSCs from both genotypes (*Trf1*^{+/+} and *Trf1*^{lox/lox}) and plating single cells in serial dilutions. One week after plating, the present inventors quantified both the number and the diameter of the neurospheres formed by each genotype, showing that *Trf1-*defficient NSCs formed a decreased number of spheres and with a smaller size, compared to *Trf1*^{+/+} controls (Fig. 11A and 11B). This was accompanied by a significant reduction of the Ki67 proliferation marker (Fig. 11C) and a reduction of percentage of Nestin-positive cells (Fig. 11D). This data indicated that *Trf1* deletion in NSCs induces a DDR located at telomeres, together with a reduce stemness and proliferation, which may reduce the oncogenic potential of these cells upon oncogenic transformation.

### 1.3 Therapeutic effects of Trf1 abrogation in already established GBMs

### 1.3.1. Trf1 deficiency impairs tumor progression in PDGFB and PDGFA induced GBM

To create a system that could mimic the situation in human patients, the present inventors next set to develop new mouse models in which the present inventors could first induce the different GBM subtypes and then delete *Trf1* once the tumors were established. To this end, the present inventors crossed *Trf1*^{+/+} or *Trf1*^{lox/lox}; Nestin-Tva; *Cdkn2a^{-l-}* mice with hUBC-CreERT2 mice to obtain *Trf1*^{+/+} or *Trf1*^{lox/lox}; Nestin-Tva; *Cdkn2a*^{*-*/*-*}; hUBC-CreERT2 mice. This system allowed us to first induce tumors by PDGFB overexpression and 2.5 weeks after the present inventors induced ubiquitous Cre-mediated *Trf1* deletion by tamoxifen administration (Fig. 12A and 12B). At this time point, tumors had already started to form (Fig. 12C).

In this experimental setting, *Trf1*^{lox/lox} deleted mice showed a 33% increase in survival compared to wild-type mice (Fig. 13A), suggesting therapeutic effectiveness of *Trf1* deletion in ceasing GBM progression once the tumors were already established. Furthermore, it was found that 25% of the GBM tumors appearing in *Trf1*-deleted mice were escapers as they showed normal TRF1 expression, and were excluded from further analyses (Fig. 13B and 13C), again highlighting the potent anti-tumorigenic effect of *Trf1* deletion.

To further study the effects of *Trf1* abrogation in already established tumors, the present inventors repeated the experiment sacrificing the mice at an earlier time point, 32 days after tumor induction (Fig. 14A). The present inventors confirmed a 50% decrease in TRF1 protein levels in *Trf1*^{lox/lox} tumors compared to the *Trf1* wild-type controls by using immunofluorescence analysis (Fig. 14B). Interestingly, and similarly to the tumor initiation models, *Trf1* deletion did not cause any significant change in telomere length in these tumors (Fig. 14C), further confirming that the therapeutic effects of *Trf1* deletion are independent of telomere length. Also, the present inventors did not find significant changes in the mRNA levels of any of the other shelterin components by RT-qPCR (Fig. 14D).

Histopathological analysis showed that *Trf1*-deleted tumors were significantly smaller compared to the controls (Fig. 15A). In agreement with the reduced size, *Trf1-*deleted tumors also showed a lower proliferation index as indicated by significantly decreased number of cells with positive Ki67 immunohistochemistry staining (Fig. 15B).

To address whether this decrease in tumor growth was associated with an increase in DNA damage, the present inventors quantified γH2AX positive cells in both genotypes and found significantly increased numbers of cells with DNA damage in the Trf1-deficient tumors compared to the controls (Fig. 16A). Moreover, this damage was located at telomeres as indicated by a significantly increased percentage of cells with more than one telomere-induced foci (TIF) in *Trf1*-deficient tumors compared to the controls (Fig. 16B). Increased telomeric damage was also accompanied by a significant increase in downstream cell cycle inhibitors p21 and p53 and in the apoptosis marker AC3 (Fig. 16C). However, the present inventors did not see significant changes in phospho-RPA32 (Fig. 16D), indicating that DNA damage is probably independent of the ATR/Chk1 pathway. Thus, *Trf1* deletion in already formed GBM tumors leads to decrease in proliferation and to DNA damage induction.

Importantly, similar results were obtained when *Trf1* was deleted in already-established PDGFA-induced tumors. In this case, mice were fed with tamoxifen 5-6 weeks after tumor induction, whenever the first mice started to die from tumors (Fig. 17A). Again, *Trf1* deletion resulted in a significant increase in survival of *Trf1*^{lox/lox} mice compared to control mice (Fig. 17B). As in the PDGFB model, *Trf1* levels were determined by PCR and immunofluorescence in all tumors to eliminate the escapers (Fig. 17C). In summary, *Trf1* deletion effectively blocks tumor progression in two independent (PDGFB and PDGFA) GBM mouse models concomitant with induction of telomere-located DNA damage.

### 1.3.2. Trf1-deficient GSCs show decreased stemness and tumorigenicity

To study the effects of *Trf1* abrogation specifically glioma stem cells (GSCs), the present inventors established an *in vitro* system by isolating GSCs from already-formed PDGFB-tumors in *Trf1*^{+/+} and *Trf1*^{lox/lox}; Nestin-Tva; Cdnkn2a^{-/-}; hUB-CreERT2 mice (Fig. 18A). After 15 days of *in vitro* treatment with tamoxifen, PCR analysis of the *Trf1* locus showed a population of *Trf1*^{lox/lox} GSCs deleted for *Trf1* (Fig. 18B).

The present inventors first assess whether *Trf1* deletion was affecting the stemness of GSCs by measuring sphere formation. Sphere quantification revealed that *Trf1*-deleted cells showed a significant reduction in both number of neurospheres and diameter of the neurospheres compared to the controls (Fig. 19A and 19B).

Next, the present inventors set to study whether *Trf1* deficiency was affecting the tumorigenic potential of these cells. Previous studies have shown that GSCs have the ability to form secondary tumors after orthotopic injection into the brain of syngeneic mice (Jiang et al. 2011). The present inventors decided to inject *Trf1*^{+/+} and *Trf1*^{lox/lox} GSCs into the brain of syngeneic mice fed with tamoxifen in order to induce *Trf1* deletion (Fig. 20A). Mice injected with *Trf1*^{lox/lox} GSCs showed a significant increase in survival compared to the controls (Fig. 20B), indicating that *Trf1* deletion was significantly decreasing the ability of GSCs to form secondary GBM tumors. Moreover, 120 days after GSCs injection, 71% of mice injected with *Trf1*^{+/+} GSCs had died owing to secondary tumors, while only 10% of the mice injected with *Trf1*^{lox/lox} GSCs had died at this point (Fig. 20C). Post-mortem histological analysis showed that these secondary tumors had a similar histology to the parental PDGFB induced tumors (Fig. 20D). In summary, *Trf1* abrogation in GSCs strongly reduces their stemness and their tumor forming potential.

### 1.4. Effects of Trf1 deletion in healthy mice

### 1.4.1 Brain specific Trf1 deletion does not impair cognitive functions in healthy mice

It has been previously demonstrated that whole body *Trf1* deletion in adult mice is compatible with mouse viability, although high proliferative compartments such as the skin and the bone marrow presented a decrease in cellularity (García-Beccaria *et al.,* 2015). Similarly, deletion of *Terf2,* which encodes another essential shelterin component, does not lead to brain dysfunction in adulthood (Lobanova *et al*., 2017). In order to validate *Trf1* as a safe target in the treatment of GBM, the present inventors set to address whether specific *Trf1* deletion in the brain was affecting the brain functions of mice.

The present inventors first checked TRF1 expression in the normal brain. In agreement with increased TRF1 expression in adult stem cells in mice (Schneider et al. 2013), the present inventors found significant TRF1 expression in the subventricular zone (SVZ) compared to cerebral cortex (Fig. 21A). The SVZ is one of the main areas of adult neurogenesis, characterized by the expression of the stem cell marker Nestin (Faiz et al., 2015).

Next, as the percentage of Nestin-positive cells is higher in newborns (Mignone *et al.,* 2004), the present inventors injected RCAS-Cre virus producing DF-1 cells to induce *Trf1* deletion into the brain of *Trf1*^{lox/lox}; *Cdkn2a^{-l-}* and *Trf1*^{+/+}*; Cdkn2a*^{-/-} newborns (2-days old) (Fig. 22A). PCR, RT-qPCR and immunofluorescence analysis of the brain two days after injection confirmed TRF1 downregulation in *Trf1*^{lox/lox} mice compared to controls (Fig. 22B-D). To assess whether *Trf1* deletion was maintained until adulthood, the present inventors injected RCAS-Cre producing DF-1 cells into the brain of a different cohort of mice and sacrificed those mice 2.5 months after injection. PCR analysis confirmed that 4 out of the 6 mice still showed *Trf1* deletion in the brain (Fig. 22E). Thus, decreased TRF1 levels specifically in the brain are maintained to adulthood without resulting in decreased mouse viability.

To address whether *Trf1* depletion in newborn brains affected adult brain function, the present inventors performed different tests to measure cognitive and olfactory capacities, memory, coordination and balance. Olfactory capacities were measured by using the so-called buried food test, in which mice were fasted 24 h and then moved to a new cage with a buried food pellet (Yang and Crawley, 2009) (Fig. 23A). Mice of both genotypes were able to find the food pellet with a 100% success rate (Fig. 23B). The time used to find the pellet was also similar in *Trf1*^{+/+} and *Trf1*^{lox/lox} mice (Fig. 23C). Next, the present inventors evaluated the memory skills by using the object recognition test (Bernardes de Jesus *et al*., 2012). The present inventors first trained the mice by placing them in a box with two identical objects (A and A), and then the present inventors changed one of the objects the day of the test (A and B) (Fig. 23D). By calculating the time spent with the new object B and by dividing this by the time spent with (A+B), which is an indication of the memory skills, the present inventors observed no significant differences between genotypes (Fig. 23E). In order to evaluate coordination and balance the present inventors performed two independent tests, the rotarod and the tight rope (Tomás-Loba *et al.,* 2008; Bernardes de Jesus *et al.,* 2012). In the rotarod test the present inventors measured the time mice could stay on the rod. In the tightrope test, the present inventors evaluate the ability of the mice to stay in the rope without falling, and the present inventors considered a "success" if mice were able to stay more than one minute. No significant differences were found between *Trf1*^{+/+} and *Trf1*^{lox/lox} mice in any of these two tests (Fig. 23F and 23G).

### 1.4.2 Whole-body Trf1 deletion is compatible with mice viability and does not impair cognitive functions

In parallel, in order to address the effects of whole body *Trf1* deletion in adult mice, the present inventors fed 10 weeks old *Trf1*^{lox/lox}; *Cdkn2a*^{*-*/}*⁻;* hUBC-CreERT2 and *Trf1*^{+/+}; *Cdkn2a*^{*-*/*-*}; hUBC-CreERT2 mice with tamoxifen to induce Cre-mediated *Trf1* excision (Fig. 24A). After two months of continuous treatment, the present inventors performed diverse cognitive tests mentioned in section 1.4.1 (i.e. buried food test, object recognition test, rotarod and tightrope) to assess whether whole-body *Trf1* deletion was affection cognitive and neuromuscular capabilities in those mice. *Trf1*-defficient mice did not show significant changes in the performance of any of this test (Fig. 24B-F), demonstrating that whole body *Trf1* deletion does not affect cognitive, olfactory, memory or neuromuscular abilities of the mice.

Next, the present inventors set to address the long-term effects of *Trf1* deletion in whole body *Trf1*-deficient mice. Survival curve analysis revealed no significant changes between both genotypes (Fig. 25A), pointing out that *Trf1* deletion does not affect mouse viability in *Cdkn2a* deficient background. However, *Trf1* deficient mice had a significant hair graying compare to controls (Fig. 25B). Also, weight follow-up showed that *Trf1*^{*lox*/*lox*} females had a significant lower weight at the age of 6 months, while this difference was not affecting males (Fig. 25C).

Full histological analysis at the human end-point confirmed that skin pathologies were significantly higher in the absence of *Trf1,* while the rest of the organs were not significantly affected (Fig. 26A). Among, the observed skin pathologies the most prevalent ones were hyperkeratosis, dermal fibrosis and atrophy in the follicles, epidermis and hypodermis (Fig. 26B).

In addition, the present inventors quantified the number of mice affected with tumors and found no significant differences between both genotypes (Fig. 27A). However, they found significant differences in the tumor spectrum as *Trf1* deficient mice had more lymphomas and less sarcomas (including histiocytic sarcoma and sarcoma) (Fig. 27B and 27C). In summary, *Trf1* whole-body deletion in a *Cdkn2a* deficient background does not cause mayor toxicities in the mice.

### - Example 2. Therapeutic effects of targeting TRF1

### 2.1. TRF1 is overexpressed in human GBM

In order to check whether targeting TRF1 could also be translated into human patients, the present inventors first determined if TRF1 was also overexpressed in human GBM. For this, the present inventors analyzed TRF1 protein levels by immunofluorescence in a total of 30 normal human brains, 7 astrocytomas and 14 GBMs. The percentage of cells presenting high TRF1 levels was highest in GBMs, followed by astrocytomas, while TRF1 was almost undetectable in normal brain tissue (Fig. 28A).

The present inventors further validated these results by results by determining TRF1 total protein levels by Western blot in three independent human GBM cell lines and in two patient-derived primary GSC cultures. Similarly, the inventors found TRF1 overexpression in GBM cell lines and patient-derived GSCs compared to human astrocytes (Fig. 29A). Not only TRF1, but also TRF2 and RAP1 were found upregulated in the patient-derived primary GSCs compared to normal astrocytes (Fig. 29B). This upregulation seems to occur at a posttranscriptional level, as the present inventors did not find significant differences in the mRNA levels of different shelterins determined by RT-qPCR (Fig. 29B).

### 2.2. Effects of Trf1 knockdown in U251 human GBM cell line

Next, the present inventors set to study the effects of *Trf1* downregulation in human cells by knocking-down *Trf1* in the U251 GBM cell line using shRNA producing lentiviral particles. *Trf1* knockdown efficiency was demonstrated by both RT-qPCR and WB (Fig. 30A and 30B). In agreement with the findings in mouse models, *Trf1* downregulation resulted in a decreased proliferation (Fig. 30C).

In addition, *Trf1* knockdown-U251 cells showed an increase in the DNA damage markers γH2AX and 53BP1 (Fig. 31A) and an increase in the so-called multitelomeric signals (Fig. 31 B), a type of telomere aberration previously associated to the loss of TRF1-mediated telomere protection (Martinez *et al*., 2009; Sfeir *et al.,* 2009).

Finally, it was observed that the number and diameter of the neurospheres was also significantly decreased in *Trf1* knocked-down cells compared to the controls (Fig. 32A and 32B). Thus, *Trf1* genetic deletion in human cells mimics the effect observed in mice.

### 2.3. TRF1 protein downregulation using chemical compounds

The present inventors had previously reported the discovery of small molecules with the ability to modulate TRF1 protein levels. These compounds were identified in a screening campaign using a small collection of 640 compounds selected by the CNIO Drug Development Program as representative of the whole collection of 50K (García-Beccaria *et al.,* 2015). In this manner, the present inventors identified compounds belonging to two independent chemical series: Series 1 with ETP-47228 and ETP-47037 as main hits; and Series 2, with ETP-50946 as the main hit (Fig. 33A and 33B). Compounds from Series 1 correspond to a PI3K inhibitor family (Méndez-Pertuz *et al*., 2017), while the mechanism of Series 2 compounds is still unknown.

### 2.3.1. Effect of TRF1 chemical modulators in the U251 GBM human cell line

To address whether these small molecules could represent a new therapeutic strategy for the treatment of GBM, the present inventors first tested the ability of the three independent TRF1 inhibitory molecules (ETP-47228, ETP-47037 and ETP-50946) to downregulate TRF1 protein levels in the U251 human GBM cell line. Immunofluorescence and western blot analysis revealed that the three compounds effectively reduced TRF1 foci and total TRF1 protein levels, respectively (Fig. 34A and 34B).

Similarly to TRF1 genetic depletion, TRF1 chemical inhibitors significantly reduced proliferation (Fig. 35A).

The present inventors also showed a significant upregulation of the DNA damage marker 53BP1 upon treatment with the three independent TRF1 chemical modulators (Fig. 36A). To assess whether the DNA damage was specifically located at telomeres the present inventors determined the presence of the so-called telomere-induced foci or TIFs. To this end, the present inventors performed a double immunofluorescence of γH2AX with the telomeric protein RAP1, which showed that the percentage of cells with 2 or more TIFs was significantly increased upon treatment with the TRF1 chemical inhibitors (Fig. 36B).

Finally, the present inventors checked if the three chemical compounds had the ability to reduce stemness in these cells. They cultured U251 cells with NSC media in order to obtain a suspension culture enriched in stem cells. The inventors performed a sphere formation assay with these cells treated with ETP-47228, ETP-47037 and ETP-50946 or DMSO for seven days. Treated cells showed a strong reduction in both number of neurospheres and diameter of neurospheres, compared to DMSO-treated cells (Fig. 37A and 37B). Of note, all these effects recapitulated what the present inventors observed with *Trf1* genetic downregulation (See 2.2).

### 2.3.2 Synergic effect of TRF1 downregulation with γ-irradiation and temozolomide

The standard treatment for GBM patients consists on surgical resection combined with radiation and chemotherapy, as well as adjuvant chemotherapy. Unfortunately, there is a strong recurrence of these tumors after treatment owing to their radioresistant and chemoresistant properties (Bao *et al.,* 2006; Bhat *et al.,* 2013; Segerman *et al.,* 2016). Interestingly, dysfunctional telomeres have been previously shown to lead to increased radiosensitivity, most likely as the consequence of telomere uncapping (Alt *et al.,* 2000; Goytisolo *et al.,* 2000). In addition, low levels of telomerase expression have been shown to correlate with a higher sensitivity to TMZ indicating that telomeres may also play an important role in TMZ resistance (Kanzawa *et al.,* 2003). On the other hand, as shown here, both genetic and chemical TRF1 inhibition significantly impaired GBM proliferation and stemness concomitant with induction of a DNA damage response at telomeres. Thus, the present inventors decided to study the combined effects of simultaneous TRF1 inhibition and γ-irradiation or temozolomide in human U251 GBM cells. Upon irradiation, glioma cells predominantly arrest in the G2/M phase (Badie *et al.,* 1999) (Fig. 38A). Interestingly, combined TRF1 chemical inhibition together with γ-irradiation (6 Gys) synergistically increased the percentage of G2 arrested cells (Fig. 38A). These effects were also recapitulated in *Trf1* knocked-down cells (Fig. 38B).

Concomitantly with the cell cycle arrest, the present inventors also observed a further increase in the amounts of DNA damage as determined by γH2AX levels (Fig. 39A) when the TRF1 inhibitors were combined with IR. To assess whether the DNA damage was located at telomeres, the present inventors performed a double immunofluorescence of γH2AX and the telomeric protein RAP1. The percentage of TIFs was significantly increased combining the TRF1 chemical modulators with IR (Fig. 39B).

In addition, combined TRF1 inhibition and treatment with temozolomide at two different concentrations for three days, synergistically reduced cell viability in the U251 GBM cell line (Fig. 40A). In summary, TRF1 inhibition represents an effective therapeutic strategy for the treatment of glioblastoma alone or in combination with the current standard treatments of γ-irradiation and temozolomide.

### 2.3.3. Effect of TRF1 chemical modulator in patient-derived GSCs

Given the promising results the present inventors obtained with the TRF1 chemical modulators in the U251 GBM cell line, the present inventors next set to address the effects of these compounds in two independent patient-derived primary GSCs (h543 and h676) cultures. The present inventors tested the effect of the three independent compounds (ETP-47228, ETP-47037, ETP-50946) in the stem potential of the GSCs by a sphere formation assay and again, treatment of primary patient-derived GSCs with the three inhibitors revealed a significant drastic reduction in both the number of spheres and diameter of spheres, compared to the untreated controls (Fig. 41A and 41B).

Next, the present inventors set to address whether TRF1 chemical inhibition was able to block tumor growth *in vivo.* Out of the three TRF1 modulators, only ETP-47037 can be administrated *in vivo* and it has the limitation of not crossing the brain blood barrier. Thus, the two independent primary GSCs (h676 and h543) were injected subcutaneously into nude mice. One week after GSC injection, mice received oral administration of the vehicle (NMP 10%, PEG 90%) as placebo or the ETP-47037 TRF1 inhibitor 5 days/week, every week until human endpoint, and tumors were continuously followed-up by caliper measurements (Fig. 42A). Patient-derived xenografts form both GSCs showed a drastic reduction in the tumor areas upon treatment with ETP-47037 during all time points after treatment until the experiment had to be interrupted owing to mice in the placebo group reaching the human end-point (Fig. 42B and 42C).

In addition, post-mortem tumor analysis of patient-derived xenografts from h676 GSCs revealed a striking decrease in tumor size and tumor weight in the ETP-47037-treated tumors compared to those treated with the placebo (Fig. 43A), accompanied by a 80% decrease of TRF1 protein levels, as showed by immunofluorescence (Fig. 43B).

Together with the significant decrease of TRF1 protein levels, the present inventors observed a reduction of the proliferation marker Ki67 and a drastic increase in the γH2AX DNA damage marker (Fig. 44A and 44B).

Also, histological analysis of the tumors revealed that ETP-47037 treatment caused decreased cellularity in the tumors, together with necrotic areas, apoptotic bodies, compacted chromatin and fragmented DNA (Fig. 45A). Interestingly, the present inventors did not detect any signs of sickness or morbidity in the ETP-47037-treated cohorts compared to the placebo group (Table 1).

**Table1. Mice pathologies after long-term treatment with vehicle or ETP-47037**

| Vehicle | ETP-47037 |
|---|---|
| Mice 1: dermatitis, kidney calcification, lung adenoma | Mice 1: dermatitis, mild widening of lymphatic vessels in intestine |
| Mice 2: dermatitis, hyperplasia in spleen and lymph nodes | Mice 2: dermatitis |
| Mice 3: dermatitis | Mice 3: dermatitis |
| Mice 4: dermatitis | Mice 4: dermatitis |

In agreement with the above observation, histological analysis of skin intestine and bone marrow confirmed the absence of mayor pathologies in these organs (Fig. 45B).

### - Example 3. Screening and characterization of novel TRF1 modulators and effects of combinations of TRF1 modulators

### 3.1. Screening of novel TRF1 modulators in compounds approved by the FDA or in clinical trials

### 3.1.1. Initial screening in an ETP.antitumorals library

The present inventors also performed a screening with the ETP-CNIO collection of 114 antitumoral drugs approved by the FDA or in clinical trials (covering 20 of the 26 pathways included in Reactome data base: see Fig. 46A). The screening was performed in CHA-9.3 mouse lung cancer cell line (García-Beccaria *et al.,* 2015), by treatment with the compounds at 1 µM concentration during 24 h (Fig. 46A).

Preliminary results showed several FDA approved drugs that can inhibit TRF1 protein levels and which are modulators of particular signaling pathways and molecular targets, including those involved in the most deregulated pathways in cancer: RTH inhibitors (RTKi: Dasatinib), MEK inhibitors (MEKi), ERK inhibitors (ERKi), mTOR inhibitors (mTORi: KU-0063794), CDK inhibitors (CDKi: Flavopiridol), HSP90 inhibitors (HSP90i: Geldanamycin), Docetaxel, Gemcitabine, PLK inhibitors (PLKi: GSK461364) and Aurora inhibitors (Aurorai: Alisertib) (Fig. 46B and 46C). Some of them were already known as TRF1 inhibitors, but others can be considered novel in the field of TRF1 modulation.

It is worth to mention that the ETP.antitumorals library used includes inhibitors of PI3K and PLK1, known targets involved in TRF1 regulation, and said compounds were identified as TRF1 modulators (Fig. 46C), therefore validating the screening and the methodology followed by the present inventors.

Furthermore, the screening campaign has yielded other classes of antitumoral drugs able to modulate TRF1 binding to telomeres. The hits confirmed by confocal microscopy at 1 µM were selective inhibitors of ERK, MEK, Aurora and other multikinase inhibitors such as Flavopiridol (CDKs), Dasatinib, other compounds such as antimetabolite drug Gemcitabine and microtubule targeting agent Docetaxel. The compounds identified in the screening as novel TRF1 modulators are the following: ETP-50853 - HSP90i (Geldanamycin), ETP-45335 - Docetaxel, ETP-45337 - Gemcitabine, ETP-51634 - Aurorai (Alisertib), ETP-51801 - RTKi (Dasatinib), ETP-51799 - PLK1i (GSK461364), ETP-50537 - mTor1/2i (KU-0063794), ETP-50728 - ERKi (SCH772984), ETP-51667 - MEKi (Selumetinib), ETP-47306 - CDKi (Flavopiridol).

### 3.1.2. Validation of MEK/ERK, HSP90 and tubulin polymerization pathways as target for TRF1 modulation

To validate the MEK/ERK pathway as new signaling pathway that modulates TRF1, 4 structurally different MEK inhibitors (MEKi) (represented in Fig. 46D) and two structurally different ERK inhibitors (ERKi) (represented in Fig. 46E) were selected. All of them were tested at 1 µM during 24h in the phenotypic assay that measure TRF1 levels. All tested inhibitors showed a clear modulation of TRF1 levels with the exception of GDC-0944 (Fig. 46F), possibly due to the concentration used.

Following the same chemical biology approach, HSP90 inhibitors (HSP90i) (Fig. 46G and 46H) and tubulin agents (Fig. 46I and 46J) have been validated as pathways that modulate TRF1 levels.

From the five HSP90 inhibitors tested, Geldanamycin, identified in the screening as a TRF1 modulator for the first time, showed the lowest modulation but the other 4 inhibitors showed to be very potent modulators of TRF1 levels.

With regard to tubulin agents, both inhibitors of tubulin depolymerization (docetaxel and paclitaxel) and tubulin polymerization inhibitors (ABT-751) were chosen to validate tubulin agents as modulators of TRF1 levels. As it is shown in Fig. 46J, tubulin depolymerization agents modulates more strongly TRF1 levels.

### 3.2. Characterization of novel TRF1 inhibitors

### 3.2.1. TRF1 inhibition in GSCs

Given the striking results of TRF1 inhibition in GBM, the present inventors next characterize whether these novel compounds (HSP90i: Geldanamycin, Docetaxel,-Gemcitabine, Aurorai: Alisertib, RTKi: Dasatinib, PLK1i: GSK461364, mTor1/2i: KU-0063794, ERKi: SCH772984, MEKi: Selumetinib, CDKi: Flavopiridol) were able to downregulate TRF1 levels in the h676 GSCs. The present inventors treated GSCs for 24h at 1 µM concentration and assessed TRF1 protein levels by western blot. Based on the degree of TRF1 inhibition, the present inventors divided the compounds in three different groups: (1) potent TRF1 modulators, including RTKi, MEKi and ERKi; (2) medium TRF1 modulators, including mTORi, HSP90i, CDKi, Gemcitabine and Docetaxel; and (3) compounds with no ability to downregulate TRF1, including PLKi and Aurorai (Fig. 46K).

### 3.2.2. Connections with the PI3K/AKT pathway

The present inventors have confirmed the modulation of TRF1 through the PI3K-AKT pathway (Mendez-Pertuz *et al.,* 2017). In order to check whether the novel compounds act independently of this pathway, the present inventors treated the cells for 24h at 1 µM concentration and checked p-AKT activation. They also included in this analysis some compounds previously identified by the same research group: namely ETP-47037 (PI3Ki) (García-Beccaria *et al.,* 2015), and ETP-50946 (Fig. 47, right graph of the lower part).

Out of the 10 compounds, the present inventors identified that ERKi, MEKi, Gemcitabine, Docetaxel, Aurorai and CDKi act independently of p-AKT, while PLK1 i, RTKi, mTORi and HSP90i were p-AKT dependent (Fig. 47).

### 3.2.3. Checking of effects of TRF1 inhibition: blocking of spheres formation and DN damage

Next, the present inventors set to address whether the different compounds were able to block sphere formation in the h676 and h543 GSCs. Dose-response analysis revealed that all the compounds had the ability to impair sphere formation at various concentrations in the two independent cells lines (Fig. 48A). HSP90i, Docetaxel and Gemcitabine were identified as the most potent compounds, as they were active at very low concentrations (Fig. 48A).

The present inventors further demonstrated that those compounds showing high and medium TRF1 inhibition were able to induce the upregulation of the γH2AX DNA damage marker (Fig. 49A).

### - Example 4. Synergic effects of different TRF1 modulators

It is known that the bad prognosis of glioblastoma is mainly due to the existence of a group of cells with stem like properties, also known as glioma-stem like cells (GSCs) (Singh *et al.,* 2004). These cells develop resistance to the treatments and are able to recapitulate the whole tumor, causing a strong recurrence (Bao *et al.,* 2006). Thus, the present inventors next performed drug combination studies in GSCs in order to design new combinatory treatments based on TRF1 inhibition, which could effectively block resistance of individual drugs.

To assess possible synergic effects between the PI3Ki and the different compounds identified in 3.2.1 as high or medium TRF1 modulators, the present inventors first calculate the concentration that produces 50% of the inhibition (EC50) in the growth GSCs. Using this concentration as a reference point, the present inventors designed a combinatorial matrix using different concentrations of the compounds (e.g. 2x GI50, GI50 and ½ GI50) to study all the possible combinations. Combination index is calculated to establish if the combination is synergistic, additive or antagonistic (Fig. 50A).

Using this experimental set up, the present inventors demonstrated that the PI3Ki (ETP-47037) showed a significant synergic effect with RTKi, ERKi, MEKi, HSP90i, Gemcitabine and Docetaxel (Fig. 51A). These results open up promising opportunities to further apply these combinations in human patients, not only for the treatment of GBM or other brain tumors, but also to use such combinations in the treatment of other cancer types.

### References

Aldape, K., Zadeh, G., Mansouri, S. et al. A. N. (2015) 129: 829. doi:10. 1007/s0040.-015-1432-1 (2015) 'Glioblastoma: pathology , molecular mechanisms and markers', Acta Neuropathologica, 129(6), pp. 829-848. doi: 10.1007/s00401-015-1432-1.
Allsopp, R. C., Morin, G. B., DePinho, R., Harley, C. B. and Weissman, I. L. (2003) 'Telomerase is required to slow telomere shortening and extend replicative lifespan of HSCs during serial transplantation', Blood, 102(2), pp. 517-520. doi: 10.1182/blood-2002-07-2334.
Alt, F. W., Wong, K. K., Chang, S., Weiler, S. R., Ganesan, S., Chaudhuri, J., Zhu, C., Artandi, S. E., Rudolph, K. L., Gottlieb, G. J., Chin, L. and DePinho, R. A. (2000) 'Telomere dysfunction impairs DNA repair and enhances sensitivity to ionizing radiation.', Nature genetics, 26(1), pp. 85-8. doi: 10.1038/79232.
Arita, H., Narita, Y., Fukushima, S., Tateishi, K., Matsushita, Y., Yoshida, A., Miyakita, Y., Ohno, M., Collins, V. P., Kawahara, N., Shibui, S. and Ichimura, K. (2013) 'Upregulating mutations in the TERT promoter commonly occur in adult malignant gliomas and are strongly associated with total 1p19q loss', Acta Neuropathologica, 126(2), pp. 267-276. doi: 10.1007/s00401-013-1141-6.
Armanios, M. and Blackburn, E. H. (2012) 'The telomere syndromes.', Nature reviews. Genetics, pp. 693-704. doi: 10.1038/nrg3246.
Armanios, M. Y., Chen, J. J.-L., Cogan, J. D., Alder, J. K., Ingersoll, R. G., Markin, C., Lawson, W. E., Xie, M., Vulto, I., Phillips, J. A., Lansdorp, P. M., Greider, C. W. and Loyd, J. E. (2007) 'Telomerase Mutations in Families with Idiopathic Pulmonary Fibrosis', New England Journal of Medicine, 356(13), pp. 1317-1326. doi: 10.1056/NEJMoa066157.
Artandi, S. E. and Attardi, L. D. (2005) 'Pathways connecting telomeres and p53 in senescence, apoptosis, and cancer', Biochemical and Biophysical Research Communications, pp. 881-890. doi: 10.1016/j.bbrc.2005.03.211.
Badie, B., Goh, C. S., Klaver, J., Herweijer, H. and Boothman, D. a (1999) 'Combined radiation and p53 gene therapy of malignant glioma cells.', Cancer gene therapy, 6(2), pp. 155-62. doi: 10.1038/sj.cgt.7700009.
Baerlocher, G. M., Oppliger Leibundgut, E., Ottmann, O. G., Spitzer, G., Odenike, O., McDevitt, M. a., Röth, A., Daskalakis, M., Burington, B., Stuart, M. and Snyder, D. S. (2015) 'Telomerase Inhibitor Imetelstat in Patients with Essential Thrombocythemia.', The New England journal of medicine, 373(10), pp. 920-8. doi: 10.1056/NEJMoa1503479.
Bainbridge, M. N., Armstrong, G. N., Gramatges, M. M., Bertuch, A. A., Jhangiani, S. N., Doddapaneni, H., Lewis, L., Tombrello, J., Tsavachidis, S., Liu, Y., Jalali, A., Plon, S. E., Lau, C. C., Parsons, D. W., Claus, E. B., Barnholtz-Sloan, J., Il'yasova, D., Schildkraut, J., Ali-Osman, F., Sadetzki, S., Johansen, C., Houlston, R. S., Jenkins, R. B., Lachance, D., Olson, S. H., Bernstein, J. L., Merrell, R. T., Wrensch, M. R., Walsh, K. M., Davis, F. G., Lai, R., Shete, S., Aldape, K., Amos, C. I., Thompson, P. A., Muzny, D. M., Gibbs, R. A., Melin, B. S. and Bondy, M. L. (2015) 'Germline mutations in shelterin complex genes are associated with familial glioma', J Natl Cancer Inst, 107(1), p. 384. doi: 10.1093/jnci/dju384.
Bao, S., Wu, Q., McLendon, R. E., Hao, Y., Shi, Q., Hjelmeland, A. B., Dewhirst, M. W., Bigner, D. D. and Rich, J. N. (2006) 'Glioma stem cells promote radioresistance by preferential activation of the DNA damage response', Nature, 444(7120), pp. 756-60.
Barrientos, K. S., Kendellen, M. F., Freibaum, B. D., Armbruster, B. N., Etheridge, K. T. and Counter, C. M. (2008) 'Distinct Functions of POT1 at Telomeres', Molecular and Cellular Biology, 28(17), pp. 5251-5264. doi: 10.1128/MCB.00048-08.
Barthel, F. P., Wei, W., Tang, M., Martinez-Ledesma, E., Hu, X., Amin, S. B., Akdemir, K. C., Seth, S., Song, X., Wang, Q., Lichtenberg, T., Hu, J., Zhang, J., Zheng, S. and Verhaak, R. G. W. (2017) 'Systematic analysis of telomere length and somatic alterations in 31 cancer types', Nature Genetics. Nature Research. doi: 10.1038/ng.3781.
Baumann, P. and Cech, T. R. (2001) 'Pot1, the putative telomere end-binding protein in fission yeast and humans', Science, 292(5519), pp. 1171-1175. doi: 10.1126/science.1060036.
Beier, F., Foronda, M., Martinez, P. and Blasco, M. A. (2012) 'Conditional TRF1 knockout in the hematopoietic compartment leads to bone marrow failure and recapitulates clinical features of dyskeratosis congenita', Blood, 120(15), pp. 2990-3000. doi: 10.1182/blood-2012-03-418038.
Bernardes de Jesus, B., Vera, E., Schneeberger, K., Tejera, A. M., Ayuso, E., Bosch, F. and Blasco, M. A. (2012) 'Telomerase gene therapy in adult and old mice delays aging and increases longevity without increasing cancer', EMBO Molecular Medicine, 4(8), pp. 691-704. doi: 10.1002/emmm.201200245.
Bhat, K. P. L., Balasubramaniyan, V., Vaillant, B., Ezhilarasan, R., Hummelink, K., Hollingsworth, F., Wani, K., Heathcock, L., James, J. D., Goodman, L. D., Conroy, S., Long, L., Lelic, N., Wang, S., Gumin, J., Raj, D., Kodama, Y., Raghunathan, A., Olar, A., Joshi, K., Pelloski, C. E., Heimberger, A., Kim, S. H., Cahill, D. P., Rao, G., DenDunnen, W. F. A., Boddeke, H. W. G. M., Phillips, H. S., Nakano, I., Lang, F. F., Colman, H., Sulman, E. P. and Aldape, K. (2013) 'Mesenchymal Differentiation Mediated by NF-??B Promotes Radiation Resistance in Glioblastoma', Cancer Cell, 24(3), pp. 331-346. doi: 10.1016/j.ccr.2013.08.001.
Bianchi, A., Smith, S., Chong, L., Elias, P. and De Lange, T. (1997) 'TRF1 is a dimer and bends telomeric DNA', EMBO Journal, 16(7), pp. 1785-1794. doi: 10.1093/emboj/16.7.1785.
Blackburn, E. H. and Gall, J. G. (1978) 'A tandemly repeated sequence at the termini of the extrachromosomal ribosomal RNA genes in Tetrahymena', Journal of Molecular Biology, 120(1), pp. 33-53. doi: 10.1016/0022-2836(78)90294-2.
Blasco, M. A., Lee, H. W., Hande, M. P., Samper, E., Lansdorp, P. M., DePinho, R. A. and Greider, C. W. (1997) 'Telomere shortening and tumor formation by mouse cells lacking telomerase RNA.', Cell, 91(1), pp. 25-34. doi: S0092-8674(01)80006-4 [pii].
Bodnar, A. G., Ouellette, M., Frolkis, M., Holt, S. E., Chiu, C. P., Morin, G. B., Harley, C. B., Shay, J. W., Lichtsteiner, S. and Wright, W. E. (1998) 'Extension of life-span by introduction of telomerase into normal human cells', Science, 279(5349), pp. 349-352. doi: 10.1126/science.279.5349.349.
Boldrini, L., Pistolesi, S., Gisfredi, S., Ursino, S., Alì, G., Pieracci, N., Basolo, F., Parenti, G. and Fontanini, G. (2006) 'Telomerase activity and hTERT mRNA expression in glial tumors', International Journal of Oncology, 28(6), pp. 1555-1560.
Boué, S., Paramonov, I., Barrero, M. J. and Belmonte, J. C. I. (2010) 'Analysis of human and mouse reprogramming of somatic cells to induced pluripotent stem cells. what is in the plate?', PLoS ONE, 5(9), pp. 1-14. doi: 10.1371/journal.pone.0012664.
Bowman, R. L., Wang, Q., Carro, A., Verhaak, R. G. W. and Squatrito, M. (2017) 'GlioVis data portal for visualization and analysis of brain tumor expression datasets', Neuro-Oncology, 19(1), pp. 139-141. doi: 10.1093/neuonc/now247.
Brennan, C., Momota, H., Hambardzumyan, D., Ozawa, T., Tandon, A., Pedraza, A. and Holland, E. (2009) 'Glioblastoma subclasses can be defined by activity among signal transduction pathways and associated genomic alterations', PLoS ONE, 4(11). doi: 10.1371/journal.pone.0007752.
Brennan, C. W., Verhaak, R. G. W., McKenna, A., Campos, B., Noushmehr, H., Salama, S. R., Zheng, S., Chakravarty, D., Sanborn, J. Z., Berman, S. H., Beroukhim, R., Bernard, B., Wu, C. J., Genovese, G., Shmulevich, I., Barnholtz-Sloan, J., Zou, L., Vegesna, R., Shukla, S. A., Ciriello, G., Yung, W. K., Zhang, W., Sougnez, C., Mikkelsen, T., Aldape, K., Bigner, D. D., Van Meir, E. G., Prados, M., Sloan, A., Black, K. L., Eschbacher, J., Finocchiaro, G., Friedman, W., Andrews, D. W., Guha, A., lacocca, M., O'Neill, B. P., Foltz, G., Myers, J., Weisenberger, D. J., Penny, R., Kucherlapati, R., Perou, C. M., Hayes, D. N., Gibbs, R., Marra, M., Mills, G. B., Lander, E., Spellman, P., Wilson, R., Sander, C., Weinstein, J., Meyerson, M., Gabriel, S., Laird, P. W., Haussler, D., Getz, G., Chin, L., Benz, C., Barrett, W., Ostrom, Q., Wolinsky, Y., Bose, B., Boulos, P. T., Boulos, M., Brown, J., Czerinski, C., Eppley, M., Kempista, T., Kitko, T., Koyfman, Y., Rabeno, B., Rastogi, P., Sugarman, M., Swanson, P., Yalamanchii, K., Otey, I. P., Liu, Y. S., Xiao, Y., Auman, J. T., Chen, P. C., Hadjipanayis, A., Lee, E., Lee, S., Park, P. J., Seidman, J., Yang, L., Kalkanis, S., Poisson, L. M., Raghunathan, A., Scarpace, L., Bressler, R., Eakin, A., lype, L., Kreisberg, R. B., Leinonen, K., Reynolds, S., Rovira, H., Thorsson, V., Annala, M. J., Paulauskis, J., Curley, E., Hatfield, M., Mallery, D., Morris, S., Shelton, T., Shelton, C., Sherman, M., Yena, P., Cuppini, L., DiMeco, F., Eoli, M., Maderna, E., Pollo, B., Saini, M., Balu, S., Hoadley, K. A., Li, L., Miller, C. R., Shi, Y., Topal, M. D., Wu, J., Dunn, G., Giannini, C., Aksoy, B. A., Antipin, Y., Borsu, L., Cerami, E., Gao, J., Gross, B., Jacobsen, A., Ladanyi, M., Lash, A., Liang, Y., Reva, B., Schultz, N., Shen, R., Socci, N. D., Viale, A., Ferguson, M. L., Chen, Q. R., Demchok, J. A., Dillon, L. A. L., Mills Shaw, K. R., Sheth, M., Tarnuzzer, R., Wang, Z., Yang, L., Davidsen, T., Guyer, M. S., Ozenberger, B. A., Sofia, H. J., Bergsten, J., Eckman, J., Harr, J., Smith, C., Tucker, K., Winemiller, C., Zach, L. A., Ljubimova, J. Y., Eley, G., Ayala, B., Jensen, M. A., Kahn, A., Pihl, T. D., Pot, D. A., Wan, Y., Hansen, N., Hothi, P., Lin, B., Shah, N., Yoon, J. G., Lau, C., Berens, M., Ardlie, K., Carter, S. L., Cherniack, A. D., Noble, M., Cho, J., Cibulskis, K., DiCara, D., Frazer, S., Gabriel, S. B., Gehlenborg, N., Gentry, J., Heiman, D., Kim, J., Jing, R., Lander, E. S., Lawrence, M., Lin, P., Mallard, W., Onofrio, R. C., Saksena, G., Schumacher, S., Stojanov, P., Tabak, B., Voet, D., Zhang, H., Dees, N. N., Ding, L., Fulton, L. L., Fulton, R. S., Kanchi, K. L., Mardis, E. R., Wilson, R. K., Baylin, S. B., Harshyne, L., Cohen, M. L., Devine, K., Sloan, A. E., Van Den Berg, S. R., Berger, M. S., Carlin, D., Craft, B., Ellrott, K., Goldman, M., Goldstein, T., Grifford, M., Ma, S., Ng, S., Stuart, J., Swatloski, T., Waltman, P., Zhu, J., Foss, R., Frentzen, B., McTiernan, R., Yachnis, A., Mao, Y., Akbani, R., Bogler, O., Fuller, G. N., Liu, W., Liu, Y., Lu, Y., Protopopov, A., Ren, X., Sun, Y., Yung, W. K. A., Zhang, J., Chen, K., Weinstein, J. N., Bootwalla, M. S., Lai, P. H., Triche, T. J., Van Den Berg, D. J., Gutmann, D. H., Lehman, N. L., Brat, D., Olson, J. J., Mastrogianakis, G. M., Devi, N. S., Zhang, Z., Lipp, E. and McLendon, R. (2013) 'The somatic genomic landscape of glioblastoma', Cell, 155(2). doi: 10.1016/j.cell.2013.09.034.
Broccoli, D., Smogorzewska, A., Chong, L. and de Lange, T. (1997) 'Human telomeres contain two distinct Myb-related proteins, TRF1 and TRF2', Nature Genetics, p. 235. doi: 10.1038/ng1097-231.
Bryan, T. M., Englezou, A., Dalla-Pozza, L., Dunham, M. A. and Reddel, R. R. (1997) 'Evidence for an alternative mechanism for maintaining telomere length in human tumors and tumor-derived cell lines', Nat Med, 3(11), pp. 1271-1274. doi: 10.1038/nm1197-1271.
Bryan, T. M., Englezou, A., Gupta, J., Bacchetti, S. and Reddel, R. R. (1995) 'Telomere elongation in immortal human cells without detectable telomerase activity', EMBO J, 14(17), pp. 4240-4248. doi: papers3://publication/uuid/E553EAC0-7D77-471F-A996-D41412778FFB.
Calado, R. T., Regal, J. A., Kleiner, D. E., Schrump, D. S., Peterson, N. R., Pons, V., Chanock, S. J., Lansdorp, P. M. and Young, N. S. (2009) 'A spectrum of severe familial liver disorders associate with telomerase mutations', PLoS ONE, 4(11). doi: 10.1371/journal.pone.0007926.
Calsou, P., Delteil, C., Frit, P., Drouet, J. and Salles, B. (2003) 'Coordinated assembly of Ku and p460 subunits of the DNA-dependent protein kinase on DNA ends is necessary for XRCC4-ligase IV recruitment', Journal of Molecular Biology, 326(1), pp. 93-103. doi: 10.1016/S0022-2836(02)01328-1.
Calvete, O., Martinez, P., Garcia-Pavia, P., Benitez-Buelga, C., Paumard-Hernández, B., Fernandez, V., Dominguez, F., Salas, C., Romero-Laorden, N., Garcia-Donas, J., Carrillo, J., Perona, R., Triviño, J. C., Andrés, R., Cano, J. M., Rivera, B., Alonso-Pulpon, L., Setien, F., Esteller, M., Rodriguez-Perales, S., Bougeard, G., Frebourg, T., Urioste, M., Blasco, M. A. and Benítez, J. (2015) 'A mutation in the POT1 gene is responsible for cardiac angiosarcoma in TP53-negative Li-Fraumeni-like families.', Nature communications, 6, p. 8383. doi: 10.1038/ncomms9383.
Canela, A., Klatt, P. and Blasco, M. A. (2007) 'Telomere length analysis', Methods in Molecular Biology, 371, pp. 45-72. doi: 10.1385/1-59745-361-7:45.
Carro, A., Perez-Martinez, M., Soriano, J., Pisano, D. G. and Megias, D. (2015) 'iMSRC: converting a standard automated microscope into an intelligent screening platform', Scientific Reports, 5(1), p. 10502. doi: 10.1038/srep10502.
Celli, G. B. and de Lange, T. (2005) 'DNA processing is not required for ATM-mediated telomere damage response after TRF2 deletion', Nature Cell Biology, 7(7), pp. 712-718. doi: 10.1038/ncb1275.
Chen, J., McKay, R. M. and Parada, L. F. (2012) 'Malignant glioma: Lessons from genomics, mouse models, and stem cells', Cell, pp. 36-47. doi: 10.1016/j.cell.2012.03.009.
Chin, L., Artandi, S. E., Shen, Q., Tam, A., Lee, S. L., Gottlieb, G. J., Greider, C. W. and DePinho, R. A. (1999) 'p53 deficiency rescues the adverse effects of telomere loss and cooperates with telomere dysfunction to accelerate carcinogenesis', Cell, 97(4), pp. 527-538. doi: 10.1016/S0092-8674(00)80762-X.
Cimprich, K. A. and Cortez, D. (2008) 'ATR: An essential regulator of genome integrity', Nature Reviews Molecular Cell Biology, pp. 616-627. doi: 10.1038/nrm2450.
Cohen, S. B., Graham, M. E., Lovrecz, G. O., Bache, N., Robinson, P. J. and Reddel, R. R. (2007) 'Protein composition of catalytically active human telomerase from immortal cells.', Science (New York, N.Y.), 315(5820), pp. 1850-3. doi: 10.1126/science.1138596.
Daniel El Fassi, Bjørn, M., Nielsen, C. and Hasselbalch, H. (2015) 'Telomerase Inhibitor Imetelstat in Essential Thrombocythemia and Myelofibrosis', New England Journal of Medicine, 373(26), pp. 2579-2581. doi: 10.1056/NEJMc1512663.
DeFazio, L. G., Stansel, R. M., Griffith, J. D. and Chu, G. (2002) 'Synapsis of DNA ends by DNA-dependent protein kinase', EMBO Journal, 21(12), pp. 3192-3200. doi: 10.1093/emboj/cdf299.
Donate, L. E. and Blasco, M. A. (2011) 'Telomeres in cancer and ageing', Philosophical Transactions of the Royal Society B: Biological Sciences, 366(1561), pp. 76-84. doi: 10.1098/rstb.201 0.0291.
Dunham, M. A., Neumann, A. A., Fasching, C. L. and Reddel, R. R. (2000) 'Telomere maintenance by recombination in human cells', Nature Genetics, 26(4), pp. 447-450. doi: 10.1038/82586.
Espejel, S., Franco, S., Rodr??guez-Perales, S., Bouffler, S. D., Cigudosa, J. C. and Blasco, M. A. (2002) 'Mammalian Ku86 mediates chromosomal fusions and apoptosis caused by critically short telomeres', EMBO Journal, 21(9), pp. 2207-2219. doi: 10.1093/emboj/21.9.2207.
Espejel, S., Franco, S., Sgura, A., Gae, D., Bailey, S. M., Taccioli, G. E. and Blasco, M. A. (2002) 'Functional interaction between DNA-PKcs and telomerase in telomere length maintenance', EMBO Journal, 21(22), pp. 6275-6287. doi: 10.1093/emboj/cdf593.
Falchetti, M. L., Fiorenzo, P., Mongiardi, M. P., Petrucci, G., Montano, N., Maira, G., Pierconti, F., Larocca, L. M., Levi, A. and Pallini, R. (2006) 'Telomerase inhibition impairs tumor growth in glioblastoma xenografts', NEUROLOGICAL RESEARCH, 28(5), pp. 532-537. doi: 10.1179/016164106X116818.
Fang, X., Zhou, W., Wu, Q., Huang, Z., Shi, Y., Yang, K., Chen, C., Xie, Q., Mack, S. C., Wang, X., Carcaboso, A. M., Sloan, A. E., Ouyang, G., McLendon, R. E., Bian, X., Rich, J. N. and Bao, S. (2016) 'Deubiquitinase USP13 maintains glioblastoma stem cells by antagonizing FBXL14-mediated Myc ubiquitination', Journal of Experimental Medicine, pp. 245-267. doi: 10.1084/jem.20151673.
Flores, I., Canela, A., Vera, E., Tejera, A., Cotsarelis, G. and Blasco, M. A. (2008) 'The longest telomeres: A general signature of adult stem cell compartments', Genes and Development, 22(5), pp. 654-667. doi: 10.1101/gad.451008.
Franco, S., Alsheimer, M., Herrera, E., Benavente, R. and Blasco, M. A. (2002) 'Mammalian meiotic telomeres: Composition and ultrastructure in telomerase-deficient mice', European Journal of Cell Biology, 81(6), pp. 335-340. doi: 10.1078/0171-9335-00259.
Furnari, F. B., Fenton, T., Bachoo, R. M., Mukasa, A., Stommel, J. M., Stegh, A., Hahn, W. C., Ligon, K. L., Louis, D. N., Brennan, C., Chin, L., DePinho, R. A. and Cavenee, W. K. (2007) 'Malignant astrocytic glioma: Genetics, biology, and paths to treatment', Genes and Development, pp. 2683-2710. doi: 10.1101/gad.1596707.
Gajjar, A., Bowers, D. C., Karajannis, M. A., Leary, S., Witt, H. and Gottardo, N. G. (2015) 'Pediatric brain tumors: Innovative genomic information is transforming the diagnostic and clinical landscape', Journal of Clinical Oncology, pp. 2986-2998. doi: 10.1200/JCO.2014.59.9217.
García-Beccaria, M., Martinez, P., Mendez-Pertuz, M., Martinez, S., Blanco-Aparicio, C., Cañamero, M., Mulero, F., Ambrogio, C., Flores, J. M., Megias, D., Barbacid, M., Pastor, J. and Blasco, M. a (2015) 'Therapeutic inhibition of TRF1 impairs the growth of p53-deficient K-RasG12V-induced lung cancer by induction of telomeric DNA damage.', EMBO molecular medicine, 7(7), pp. 930-49. doi: 10.15252/emmm.201404497.
Gomez, M. (2006) 'PARP1 Is a TRF2-associated Poly(ADP-Ribose)Polymerase and Protects Eroded Telomeres', Molecular Biology of the Cell, 17(4), pp. 1686-1696. doi: 10.1091/mbc.E05-07-0672.
Gonzalez-Suarez, E., Samper, E., Flores, J. M. and Blasco, M. A. (2000) 'Telomerase-deficient mice with short telomeres are resistant to skin tumorigenesis.', Nature genetics, 26(1), pp. 114-117. doi: 10.1038/79089.
Goytisolo, F. A., Samper, E., Martín-Caballero, J., Finnon, P., Herrera, E., Flores, J. M., Bouffler, S. D. and Blasco, M. A. (2000) 'Short telomeres result in organismal hypersensitivity to ionizing radiation in mammals.', The Journal of experimental medicine, 192(11), pp. 1625-36. doi: 10.1084/jem.192.11.1625.
Greenberg, R. A., Chin, L., Femino, A., Kee-Ho, L., Gottlieb, G. J., Singer, R. H., Greider, C. W. and DePinho, R. A. (1999) 'Short dysfunctional telomeres impair tumorigenesis in the INK4a(??2/3) cancer-prone mouse', Cell, 97(4), pp. 515-525. doi: 10.1016/S0092-8674(00)80761-8.
Greenberg, R. a, Allsopp, R. C., Chin, L., Morin, G. B. and DePinho, R. a (1998) 'Expression of mouse telomerase reverse transcriptase during development, differentiation and proliferation.', Oncogene, 16(13), pp. 1723-1730. doi: 10.1038/sj.onc.1201933.
Greider, C. W. (1999) 'Telomeres do D-loop-T-loop', Cell, pp. 419-422. doi: 10.1016/S0092-8674(00)80750-3.
Greider, C. W. and Blackburn, E. H. (1985) 'Identification of a specific telomere terminal transferase activity in tetrahymena extracts', Cell, 43(2 PART 1), pp. 405-413. doi: 10.1016/0092-8674(85)90170-9.
Griffith, J. D., Comeau, L., Rosenfield, S., Stansel, R. M., Bianchi, A., Moss, H. and De Lange, T. (1999) 'Mammalian telomeres end in a large duplex loop', Cell, 97(4), pp. 503-514. doi: 10.1016/S0092-8674(00)80760-6.
Hambardzumyan, D., Amankulor, N. M., Helmy, K. Y., Becher, O. J. and Holland, E. C. (2009) 'Modeling Adult Gliomas Using RCAS/t-va Technology', Transl Oncol, 2(2), pp. 89-95. doi: 10.1593/tlo.09100.
Hambardzumyan, D., Parada, L. F., Holland, E. C. and Charest, A. (2011) 'Genetic modeling of gliomas in mice: New tools to tackle old problems', GLIA, 59(8), pp. 1155-1168. doi: 10.1002/glia.21142.
Hanahan, D. and Weinberg, R. A. (2011) 'Hallmarks of cancer: The next generation', Cell, pp. 646-674. doi: 10.1016/j.cell.2011.02.013.
Harley, C. B., Futcher, A. B. and Greider, C. W. (1990) 'Telomeres shorten during ageing of human fibroblasts', Nature, 345(6274), pp. 458-460. doi: 10.1038/345458a0.
Harley, C. B., Futcher, A. B. and Greider, C. W. (1990) 'Telomeres shorten during ageing of human fibroblasts.', Nature, 345(6274), pp. 458-60. doi: 10.1038/345458a0.
Hasegawa, D., Okabe, S., Okamoto, K., Nakano, I., Shin-Ya, K. and Seimiya, H. (2016) 'G-quadruplex ligand-induced DNA damage response coupled with telomere dysfunction and replication stress in glioma stem cells', Biochemical and Biophysical Research Communications, 471(1), pp. 75-81. doi: 10.1016/j.bbrc.2016.01.176.
Hastie, N. D., Dempster, M., Dunlop, M. G., Thompson, A. M., Green, D. K. and Allshire, R. C. (1990) 'Telomere reduction in human colorectal carcinoma and with ageing.', Nature, 346(6287), pp. 866-868. doi: 10.1038/346866a0.
Hayflick, L. and Moorhead, P. S. (1961) 'The serial cultivation of human diploid cell strains', Experimental Cell Research, 25(3), pp. 585-621. doi: 10.1016/0014-4827(61)90192-6.
Heaphy, C. M., de Wilde, R. F., Jiao, Y., Klein, A. P., Edil, B. H., Shi, C., Bettegowda, C., Rodriguez, F. J., Eberhart, C. G., Hebbar, S., Offerhaus, G. J., McLendon, R., Rasheed, B. A., He, Y., Yan, H., Bigner, D. D., Oba-Shinjo, S. M., Marie, S. K., Riggins, G. J., Kinzler, K. W., Vogelstein, B., Hruban, R. H., Maitra, A., Papadopoulos, N. and Meeker, A. K. (2011) 'Altered telomeres in tumors with ATRX and DAXX mutations', Science, 333(6041), p. 425. doi: 10.1126/science. 1207313.
Hegi, M. E., Diserens, A.-C., Gorlia, T., Hamou, M.-F., de Tribolet, N., Weller, M., Kros, J. M., Hainfellner, J. A., Mason, W., Mariani, L., Bromberg, J. E. C., Hau, P., Mirimanoff, R.O., Cairncross, J. G., Janzer, R. C. and Stupp, R. (2005) 'MGMT gene silencing and benefit from temozolomide in glioblastoma', The New England journal of medicine, 352(10), pp. 997-1003. doi: 10.1056/NEJMoa043331.
Hemann, M. T. and Greider, C. W. (2000) 'Wild-derived inbred mouse strains have short telomeres.', Nucleic acids research, 28(22), pp. 4474-8. doi: 10.1093/nar/28.22.4474.
Herrera, E. (1999) 'Telomere shortening in mTR-/- embryos is associated with failure to close the neural tube', The EMBO Journal, 18(5), pp. 1172-1181. doi: 10.1093/emboj/18.5.1172.
Herrera, E., Martínez-A, C. and Blasco, M. a (2000) 'Impaired germinal center reaction in mice with short telomeres.', The EMBO journal, 19(3), pp. 472-481. doi: 10.1093/emboj/19.3.472.
Herrera, E., Samper, E., Martín-Caballero, J., Flores, J. M., Lee, H. W. and Blasco, M. A. (1999) 'Disease states associated with telomerase deficiency appear earlier in mice with short telomeres', EMBO Journal, 18(11), pp. 2950-2960. doi: 10.1093/emboj/18.11.2950.
Hertwig, F., Peifer, M. and Fischer, M. (2016) 'Telomere maintenance is pivotal for high-risk neuroblastoma', Cell Cycle, pp. 311-312. doi: 10.1080/15384101.2015.1125243.
Hockemeyer, D., Daniels, J. P., Takai, H. and de Lange, T. (2006) 'Recent Expansion of the Telomeric Complex in Rodents: Two Distinct POT1 Proteins Protect Mouse Telomeres', Cell, 126(1), pp. 63-77. doi: 10.1016/j.cell.2006.04.044.
Hoffmeyer, K., Raggioli, A., Rudloff, S., Anton, R., Hierholzer, A., Del Valle, I., Hein, K., Vogt, R. and Kemler, R. (2012) 'Wnt/ -Catenin Signaling Regulates Telomerase in Stem Cells and Cancer Cells', Science, 336(6088), pp. 1549-1554. doi: 10.1126/science.1218370.
Holland, E. C., Hively, W. P., DePinho, R. A. and Varmus, H. E. (1998) 'A constitutively active epidermal growth factor receptor cooperates with disruption of G1 cell-cycle arrest pathways to induce glioma-like lesions in mice', Genes and Development, 12(23), pp. 3675-3685. doi: 10.1101/gad.12.23.3675.
Houghtaling, B. R., Cuttonaro, L., Chang, W. and Smith, S. (2004) 'A dynamic molecular link between the telomere length regulator TRF1 and the chromosome end protector TRF2', Current Biology, 14(18), pp. 1621-1631. doi: 10.1016/j.cub.2004.08.052.
Hu, H., Zhang, Y., Zou, M., Yang, S. and Liang, X. Q. (2010) 'Expression of TRF1, TRF2, TIN2, TERT, KU70, and BRCA1 proteins is associated with telomere shortening and may contribute to multistage carcinogenesis of gastric cancer', Journal of Cancer Research and Clinical Oncology, 136(9), pp. 1407-1414. doi: 10.1007/s00432-010-0795-x.
Huffman, K. E., Levene, S. D., Tesmer, V. M., Shay, J. W. and Wright, W. E. (2000) 'Telomere shortening is proportional to the size of the G-rich telomeric 3'-overhang', Journal of Biological Chemistry, 275(26), pp. 19719-19722. doi: 10.1074/jbc.M002843200.
Ivancich, M., Schrank, Z., Wojdyla, L., Leviskas, B., Kuckovic, A., Sanjali, A. and Puri, N. (2017) 'Treating Cancer by Targeting Telomeres and Telomerase', Antioxidants, 6(1), p. 15. doi: 10.3390/antiox6010015.
De Jager, M., Van Noort, J., Van Gent, D. C., Dekker, C., Kanaar, R. and Wyman, C. (2001) 'Human Rad50/Mre11 is a flexible complex that can tether DNA ends', Molecular Cell, 8(5), pp. 1129-1135. doi: 10.1016/S1097-2765(01)00381-1.
Joseph, I., Tressler, R., Bassett, E., Harley, C., Buseman, C. M., Pattamatta, P., Wright, W. E., Shay, J. W. and Go, N. F. (2010) 'The telomerase inhibitor imetelstat depletes cancer stem cells in breast and pancreatic cancer cell lines', Cancer Research, 70(22), pp. 9494-9504. doi: 10.1158/0008-5472.CAN-10-0233.
Kanzawa, T., Germano, I. M., Kondo, Y., Ito, H., Kyo, S. and Kondo, S. (2003) 'Inhibition of telomerase activity in malignant glioma cells correlates with their sensitivity to temozolomide.', British journal of cancer, 89(5), pp. 922-929. doi: 10.1038/sj.bjc.6601193.
Khadka, P., Lee, J. H., Baek, S. H., Oh, S. Y. and Chung, I. K. (2014) 'DNA-PKcs-interacting protein KIP binding to TRF2 is required for the maintenance of functional telomeres.', The Biochemical journal, 463(1), pp. 19-30. doi: 10.1042/BJ20131395.
Kibe, T., Osawa, G. A., Keegan, C. E. and de Lange, T. (2010) 'Telomere Protection by TPP1 Is Mediated by POT1a and POT1b', Molecular and Cellular Biology, 30(4), pp. 1059-1066. doi: 10.1128/MCB.01498-09.
Kim, N., Piatyszek, M., Prowse, K., Harley, C., West, M., Ho, P., Coviello, G., Wright, W., Weinrich, S. and Shay, J. (1994) 'Specific association of human telomerase activity with immortal cells and cancer', Science, 266(5193), pp. 2011-2015. doi: 10.1126/science.7605428.
Kim, S. H., Beausejour, C., Davalos, A. R., Kaminker, P., Heo, S. J. and Campisi, J. (2004) 'TIN2 mediates functions of TRF2 at human telomeres', Journal of Biological Chemistry, 279(42), pp. 43799-43804. doi: 10.1074/jbc.M408650200.
Kim, S. H., Kaminker, P. and Campisi, J. (1999) 'TIN2, a new regulator of telomere length in human cells', Nature Genetics, 23(4), pp. 405-412. doi: 10.1038/70508. Klobutcher, L. A., Swanton, M. T., Donini, P. and Prescott, D. M. (1981) 'All gene-sized DNA molecules in four species of hypotrichs have the same terminal sequence and an unusual 3' terminus.', Proceedings of the National Academy of Sciences, 78(5), pp. 3015-3019. doi: 10.1073/pnas.78.5.3015.
Koelsche, C., Sahm, F., Capper, D., Reuss, D., Sturm, D., Jones, D. T. W., Kool, M., Northcott, P. A., Wiestler, B., Bömer, K., Meyer, J., Mawrin, C., Hartmann, C., Mittelbronn, M., Platten, M., Brokinkel, B., Seiz, M., Herold-Mende, C., Unterberg, A., Schittenhelm, J., Weller, M., Pfister, S., Wick, W., Korshunov, A. and Von Deimling, A. (2013) 'Distribution of TERT promoter mutations in pediatric and adult tumors of the nervous system', Acta Neuropathologica, 126(6), pp. 907-915. doi: 10.1007/s00401-013-1195-5.
De Lange, T. (2002) 'Protection of mammalian telomeres', Oncogene, pp. 532-540. doi: 10.1038/sj/onc/1205080.
De Lange, T. (2005) 'Shelterin: The protein complex that shapes and safeguards human telomeres', Genes and Development, pp. 2100-2110. doi: 10.1101/gad.1346005.
De Lange, T. and DePinho, R. A. (1999) 'Unlimited mileage from tetomerase?', Science, pp. 947-948. doi: 10.1126/science.283.5404.947.
de Lange, T., Shiue, L., Myers, R. M., Cox, D. R., Naylor, S. L., Killery, a M. and Varmus, H. E. (1990) 'Structure and variability of human chromosome ends.', Molecular and cellular biology, 10(2), pp. 518-527. doi: 10.1128/MCB.10.2.518.Updated.
Lansdorp, P. M., Verwoerd, N. P., Van De Rijke, F. M., Dragowska, V., Little, M. T., Dirks, R. W., Raap, A. K. and Tanke, H. J. (1996) 'Heterogeneity in telomere length of human chromosomes', Human Molecular Genetics, 5(5), pp. 685-691. doi: 10.1093/hmg/5.5.685.
Lathia, J. D., Mack, S. C., Mulkearns-Hubert, E. E., Valentim, C. L. L. and Rich, J. N. (2015) 'Cancer stem cells in glioblastoma.', Genes & Development, 29(12), pp. 1203-1217. doi: 10.1101/gad.261982.115.
Lee, H. W., Blasco, M. A., Gottlieb, G. J., Horner, J. W., Greider, C. W. and DePinho, R. A. (1998) 'Essential role of mouse telomerase in highly proliferative organs', Nature, 392(6676), pp. 569-574. doi: 10.1038/33345.
Lee, J.-H. and Paull, T. T. (2007) 'Activation and regulation of ATM kinase activity in response to DNA double-strand breaks', Oncogene, 26(56), pp. 7741-7748. doi: 10.1038/sj.0nc.1210872.
Lenain, C., Bauwens, S., Amiard, S., Brunori, M., Giraud-Panis, M. J. and Gilson, E. (2006) 'The Apollo 5' Exonuclease Functions Together with TRF2 to Protect Telomeres from DNA Repair', Current Biology, 16(13), pp. 1303-1310. doi: 10.1016/j.cub.2006.05.021.
Li, B. (2003) 'Rap1 Affects the Length and Heterogeneity of Human Telomeres', Molecular Biology of the Cell, 14(12), pp. 5060-5068. doi: 10.1091/mbc.E03-06-0403.
Li, B., Oestreich, S. and De Lange, T. (2000) 'Identification of human Rap1: Implications for telomere evolution', Cell, 101(5), pp. 471-483. doi: 10.1016/S0092-8674(00)80858-2.
Li, L., Zhang, L., Fan, J., Greenberg, K., Desiderio, S., Rassool, F. V. and Small, D. (2011) 'Defective nonhomologous end joining blocks B-cell development in FLT3/ITD mice', Blood, 117(11), pp. 3131-3139. doi: 10.1182/blood-2010-05-286070.
Lipps, H. J. and Rhodes, D. (2009) 'G-quadruplex structures: in vivo evidence and function', Trends in Cell Biology, pp. 414-422. doi: 10.1016/j.tcb.2009.05.002.
Liu, D., O'Connor, M. S., Qin, J. and Songyang, Z. (2004) 'Telosome, a mammalian telomere-associated complex formed by multiple telomeric proteins', Journal of Biological Chemistry, 279(49), pp. 51338-51342. doi: 10.1074/jbc.M409293200.
Loayza, D. and De Lange, T. (2003) 'POT1 as a terminal transducer of TRF1 telomere length control', Nature, 423(6943), pp. 1013-1018. doi: 10.1038/nature01688.
Lobanova, A., She, R., Pieraut, S., Clapp, C., Maximov, A. and Denchi, E. L. (2017) 'Different requirements of functional telomeres in neural stem cells and terminally differentiated neurons', Genes & Development, 31(7), pp. 639-647. doi: 10.1101/gad.295402.116.
Longhese, M. P., Bonetti, D., Manfrini, N. and Clerici, M. (2010) 'Mechanisms and regulation of DNA end resection', The EMBO Journal, 29(17), pp. 2864-2874. doi: 10.1038/emboj.2010.165.
Louis, D. N., Ohgaki, H., Wiestler, O. D., Cavenee, W. K., Burger, P. C., Jouvet, A., Scheithauer, B. W. and Kleihues, P. (2007) 'The 2007 WHO classification of tumours of the central nervous system (vol 114, pg 97, 2007)', Acta Neuropathologica, 114, p. 547. doi: 10.1007/s00401-007-0243-4.
Louis, D. N., Perry, A., Reifenberger, G., von Deimling, A., Figarella-Branger, D., Cavenee, W. K., Ohgaki, H., Wiestler, O. D., Kleihues, P. and Ellison, D. W. (2016) 'The 2016 World Health Organization Classification of Tumors of the Central Nervous System: a summary', Acta Neuropathologica, pp. 803-820. doi: 10.1007/s00401-016-1545-1.
Marcand, S., Brevet, V., Mann, C. and Gilson, E. (2000) 'Cell cycle restriction of telomere elongation', Current Biology, 10(8), pp. 487-490. doi: 10.1016/S0960-9822(00)00450-4.
Marcand, S., Gilson, E. and Shore, D. (1997) 'A protein-counting mechanism for telomere length regulation in yeast', Science, 275(5302), pp. 986-990. doi: 10.1126/science.275.5302.986.
Marian, C. O., Cho, S. K., Mcellin, B. M., Maher, E. A., Hatanpaa, K. J., Madden, C. J., Mickey, B. E., Wright, W. E., Shay, J. W. and Bachoo, R. M. (2010) 'The telomerase antagonist, imetelstat, efficiently targets glioblastoma tumor-initiating cells leading to decreased proliferation and tumor growth', Clinical Cancer Research, 16(1), pp. 154-163. doi: 10.1158/1078-0432.CCR-09-2850.
Marion, R. M., Strati, K., Li, H., Tejera, A., Schoeftner, S., Ortega, S., Serrano, M. and Blasco, M. A. (2009) 'Telomeres Acquire Embryonic Stem Cell Characteristics in Induced Pluripotent Stem Cells', Cell Stem Cell, 4(2), pp. 141-154. doi: 10.1016/j.stem.2008.12.010.
Martinez, P. and Blasco, M. A. (2011) 'Telomeric and extra-telomeric roles for telomerase and the telomere-binding proteins', Nature Reviews Cancer, 11(3), pp. 161-176. doi: 10.1038/nrc3025.
Martinez, P., Thanasoula, M., Carlos, A. R., Gómez-López, G., Tejera, A. M., Schoeftner, S., Dominguez, O., Pisano, D. G., Tarsounas, M. and Blasco, M. A. (2010) 'Mammalian Rap1 controls telomere function and gene expression through binding to telomeric and extratelomeric sites', Nature Cell Biology, 12(8), pp. 768-780. doi: 10.1038/ncb2081.
Martinez, P., Thanasoula, M., Muñoz, P., Liao, C., Tejera, A., McNees, C., Flores, J. M., Fernandez-Capetillo, O., Tarsounas, M. and Blasco, M. A. (2009) 'Increased telomere fragility and fusions resulting from TRF1 deficiency lead to degenerative pathologies and increased cancer in mice', Genes and Development, 23(17), pp. 2060-2075. doi: 10.1101/gad.543509.
McClintock, B. (1941) 'The Stability of Broken Ends of Chromosomes in Zea Mays.', Genetics, 26(2), pp. 234-282. doi: 10.1038/378739a0.
Mendez-Pertuz, M., Martinez, P., Blanco-Aparicio, C., Gomez-Casero, E., Belen Garcia, A., Martínez-Torrecuadrada, J., Palafox, M., Cortés, J., Serra, V., Pastor, J. and Blasco, M. A. (2017) 'Modulation of telomere protection by the PI3K/AKT pathway', Nature Communications, 8(1). doi: 10.1038/s41467-017-01329-2.
Merle, P., Gueugneau, M., Teulade-Fichou, M.-P., Müller-Barthélémy, M., Amiard, S., Chautard, E., Guetta, C., Dedieu, V., Communal, Y., Mergny, J.-L., Gallego, M., White, C., Verrelle, P. and Tchirkov, A. (2015) 'Highly efficient radiosensitization of human glioblastoma and lung cancer cells by a G-quadruplex DNA binding compound.', Scientific reports, 5, p. 16255. doi: 10.1038/srep16255.
Meyne, J., Ratliff, R. L. and Moyzis, R. K. (1989) 'Conservation of the human telomere sequence (TTAGGG)n among vertebrates.', Proceedings of the National Academy of Sciences of the United States of America, 86(18), pp. 7049-53. doi: 10.1073/pnas.86.18.7049.
Middleton, G., Silcocks, P., Cox, T., Valle, J., Wadsley, J., Propper, D., Coxon, F., Ross, P., Madhusudan, S., Roques, T., Cunningham, D., Falk, S., Wadd, N., Harrison, M., Corrie, P., Iveson, T., Robinson, A., McAdam, K., Eatock, M., Evans, J., Archer, C., Hickish, T., Garcia-Alonso, A., Nicolson, M., Steward, W., Anthoney, A., Greenhalf, W., Shaw, V., Costello, E., Naisbitt, D., Rawcliffe, C., Nanson, G. and Neoptolemos, J. (2014) 'Gemcitabine and capecitabine with or without telomerase peptide vaccine GV1001 in patients with locally advanced or metastatic pancreatic cancer (TeloVac): An open-label, randomised, phase 3 trial', The Lancet Oncology, 15(8), pp. 829-840. doi: 10.1016/S1470-2045(14)70236-0.
Mignone, J. L., Kukekov, V., Chiang, A. S., Steindler, D. and Enikolopov, G. (2004) 'Neural stem and progenitor cells in nestin-GFP transgenic mice', Journal of Comparative Neurology, 469(3), pp. 311-324. doi: 10.1002/cne. 10964.
Mitchell, J. R., Wood, E. and Collins, K. (1999) 'A telomerase component is defective in the human disease dyskeratosis congenita', Nature, 402(6761), pp. 551-555. doi: 10.1038/990141.
Molina, J. R., Hayashi, Y., Stephens, C. and Georgescu, M.-M. M.-M. (2010) 'Invasive glioblastoma cells acquire stemness and increased Akt activation.', Neoplasia (New York, N.Y.), 12(6), pp. 453-63. doi: 10.1593/neo.10126.
Montgomery, R. K., Carlone, D. L., Richmond, C. A., Farilla, L., Kranendonk, M. E. G., Henderson, D. E., Baffour-Awuah, N. Y., Ambruzs, D. M., Fogli, L. K., Algra, S. and Breault, D. T. (2011) 'Mouse telomerase reverse transcriptase (mTert) expression marks slowly cycling intestinal stem cells', Proceedings of the National Academy of Sciences, 108(1), pp. 179-184. doi: 10.1073/pnas.1013004108.
Müller H (1938) The remaking of chromosomes. Collecting NET(13): 181-198
Muntoni, A. and Reddel, R. R. (2005) 'The first molecular details of ALT in human tumor cells', Human Molecular Genetics, 14(SUPPL. 2). doi: 10.1093/hmg/ddi266.
Newey, P. J., Nesbit, M. A., Rimmer, A. J., Attar, M., Head, R. T., Christie, P. T., Gorvin, C. M., Stechman, M., Gregory, L., Mihai, R., Sadler, G., McVean, G., Buck, D. and Thakker, R. V. (2012) 'Whole-exome sequencing studies of nonhereditary (sporadic) parathyroid adenomas', Journal of Clinical Endocrinology and Metabolism, 97(10). doi: 10.1210/jc.2012-2303.
Nonoguchi, N., Ohta, T., Oh, J. E., Kim, Y. H., Kleihues, P. and Ohgaki, H. (2013) 'TERT promoter mutations in primary and secondary glioblastomas', Acta Neuropathologica, 126(6), pp. 931-937. doi: 10.1007/s00401-013-1163-0.
Nussenzweig, A. and Nussenzweig, M. C. (2007) 'A Backup DNA Repair Pathway Moves to the Forefront', Cell, pp. 223-225. doi: 10.1016/j.cell.2007.10.005.
Ohgaki, H. and Kleihues, P. (2009) 'Genetic alterations and signaling pathways in the evolution of gliomas', Cancer Science, pp. 2235-2241. doi: 10.1111/j.1349-7006.2009.01308.x.
Ohki R, Tsurimoto T, Ishikawa F (2001) In vitro reconstitution of the end replication problem. Mol Cell Biol 21(17): 5753-5766
Ohmura, H., Tahara, H., Suzuki, M., Ide, T., Shimizu, M., Yoshida, M. A., Tahara, E., Shay, J. W., Barrett, J. C. and Oshimura, M. (1995) 'Restoration of the Cellular Senescence Program and Repression of Telomerase by Human Chromosome 3', Japanese Journal of Cancer Research, 86(10), pp. 899-904. doi: 10.1111/j.1349-7006.1995.tb02998.x.
Okazaki R, Okazaki T, Sakabe K, Sugimoto K (1967) Mechanism of DNA replication possible discontinuity of DNA chain growth. Jpn J Med Sci Biol 20(3): 255-260
Olovnikov, A. M. (1973) 'A theory of marginotomy. The incomplete copying of template margin in enzymic synthesis of polynucleotides and biological significance of the phenomenon', Journal of Theoretical Biology, 41(1), pp. 181-190. doi: 10.1016/0022-5193(73)90198-7.
Opresko, P. L., Von Kobbe, C., Laine, J. P., Harrigan, J., Hickson, I. D. and Bohr, V. A. (2002) 'Telomere-binding protein TRF2 binds to and stimulates the Werner and Bloom syndrome helicases', Journal of Biological Chemistry, 277(43), pp. 41110-41119. doi: 10.1074/jbc.M205396200.
Ozawa, T., Riester, M., Cheng, Y. K., Huse, J., Squatrito, M., Helmy, K., Charles, N., Michor, F. and Holland, E. C. (2014) 'Most human non-GCIMP glioblastoma subtypes evolve from a common proneural-like precursor glioma', Cancer Cell, 26(2), pp. 288-300. doi: 10.1016/j.ccr.2014.06.005.
Pal, D., Sharma, U., Singh, S. K., Kakkar, N. and Prasad, R. (2015) 'Over-expression of telomere binding factors (TRF1 & TRF2) in renal cell carcinoma and their inhibition by using SiRNA induce apoptosis, reduce cell proliferation and migration invitro', PLoS ONE, 10(3). doi: 10.1371/journal.pone.0115651.
Parkhurst, M. R., Riley, J. P., Igarashi, T., Li, Y., Robbins, P. F. and Rosenberg, S. A. (2004) 'Immunization of patients with the hTERT:540-548 peptide induces peptide-reactive T lymphocytes that do not recognize tumors endogenously expressing telomerase', Clinical Cancer Research, 10(14), pp. 4688-4698. doi: 10.1158/1078-0432.CCR-04-0325.
Perera, S. A., Maser, R. S., Xia, H., McNamara, K., Protopopov, A., Chen, L., F.hezel, A., Kim, C. F., Bronson, R. T., Castrillon, D. H., Chin, L., Bardeesy, N., DePinho, R. A. and Wong, K. K. (2008) 'Telomere dysfunction promotes genome instability and metastatic potential in a K-ras p53 mouse model of lung cancer', Carcinogenesis, 29(4), pp. 747-753. doi: 10.1093/carcin/bgn050.
Ramsay, A. J., Quesada, V., Foronda, M., Conde, L., Martínez-Trillos, A., Villamor, N., Rodriguez, D., Kwarciak, A., Garabaya, C., Gallardo, M., López-Guerra, M., López-Guillermo, A., Puente, X. S., Blasco, M. A., Campo, E. and López-Otín, C. (2013) 'POT1 mutations cause telomere dysfunction in chronic lymphocytic leukemia', Nature genetics, 45(5), pp. 526-530. doi: 10.1038/ng.2584.
Riballo, E., Kühne, M., Rief, N., Doherty, A., Smith, G. C. M., Recio, M. J., Reis, C., Dahm, K., Fricke, A., Krempler, A., Parker, A. R., Jackson, S. P., Gennery, A., Jeggo, P. A. and Löbrich, M. (2004) 'A pathway of double-strand break rejoining dependent upon ATM, Artemis, and proteins locating to γ-H2AX foci', Molecular Cell, 16(5), pp. 715-724. doi: 10.1016/j.molcel.2004.10.029.
Robles-Espinoza, C. D., Harland, M., Ramsay, A. J., Aoude, L. G., Quesada, V., Ding, Z., Pooley, K. A., Pritchard, A. L., Tiffen, J. C., Petljak, M., Palmer, J. M., Symmons, J., Johansson, P., Stark, M. S., Gartside, M. G., Snowden, H., Montgomery, G. W., Martin, N. G., Liu, J. Z., Choi, J., Makowski, M., Brown, K. M., Dunning, A. M., Keane, T. M., Lopez-Otin, C., Gruis, N. A., Hayward, N. K., Bishop, D. T., Newton-Bishop, J. A. and Adams, D. J. (2014) 'POT1 loss-of-function variants predispose to familial melanoma', Nat Genet, 46(5), pp. 478-481. doi: 10.1038/ng.2947.
Rudolph, K. L., Chang, S., Lee, H. W., Blasco, M., Gottlieb, G. J., Greider, C. and DePinho, R. A. (1999) 'Longevity, stress response, and cancer in aging telomerase-deficient mice', Cell, 96(5), pp. 701-712. doi: 10.1016/S0092-8674(00)80580-2.
Ruzankina, Y., Pinzon-Guzman, C., Asare, A., Ong, T., Pontano, L., Cotsarelis, G., Zediak, V. P., Velez, M., Bhandoola, A. and Brown, E. J. (2007) 'Deletion of the Developmentally Essential Gene ATR in Adult Mice Leads to Age-Related Phenotypes and Stem Cell Loss', Cell Stem Cell, 1(1), pp. 113-126. doi: 10.1016/j.stem.2007.03.002.
Sallmyr, A., Tomkinson, A. E. and Rassool, F. V. (2008) 'Up-regulation of WRN and DNA ligase III in chronic myeloid leukemia: consequences for the repair of DNA double-strand breaks', Blood, 112(4), pp. 1413-1423. doi: 10.1182/blood-2007-07-104257.
Samper, E., Fernandez, P., Eguía, R., Martin-Rivera, L., Bernad, A., Blasco, M. A. and Aracil, M. (2002) 'Long-term repopulating ability of telomerase-deficient murine hematopoietic stem cells', Blood, 99(8), pp. 2767-2775. doi: 10.1182/blood.V99.8.2767.
Samper, E., Flores, J. M. and Blasco, M. A. (2001) 'Restoration of telomerase activity rescues chromosomal instability and premature aging in Terc-/- mice with short telomeres', EMBO Reports, 2(9), pp. 800-807. doi: 10.1093/embo-reports/kve174.
Schepers, A. G., Vries, R., Van Den Born, M., Van De Wetering, M. and Clevers, H. (2011) 'Lgr5 intestinal stem cells have high telomerase activity and randomly segregate their chromosomes', EMBO Journal, 30(6), pp. 1104-1109. doi: 10.1038/emboj.2011.26.
Schneider, R. P., Garrobo, I., Foronda, M., Palacios, J. a, Marión, R. M., Flores, I., Ortega, S. and Blasco, M. a (2013) 'TRF1 is a stem cell marker and is essential for the generation of induced pluripotent stem cells.', Nature communications, 4, p. 1946. doi: 10.1038/ncomms2946.
Schwartzentruber, J., Korshunov, A., Liu, X.-Y., Jones, D. T. W., Pfaff, E., Jacob, K., Sturm, D., Fontebasso, A. M., Quang, D.-A. K., Tönjes, M., Hovestadt, V., Albrecht, S., Kool, M., Nantel, A., Konermann, C., Lindroth, A., Jager, N., Rausch, T., Ryzhova, M., Korbel, J. O., Hielscher, T., Hauser, P., Garami, M., Klekner, A., Bognar, L., Ebinger, M., Schuhmann, M. U., Scheurlen, W., Pekrun, A., Frühwald, M. C., Roggendorf, W., Kramm, C., Dürken, M., Atkinson, J., Lepage, P., Montpetit, A., Zakrzewska, M., Zakrzewski, K., Liberski, P. P., Dong, Z., Siegel, P., Kulozik, A. E., Zapatka, M., Guha, A., Malkin, D., Felsberg, J., Reifenberger, G., von Deimling, A., Ichimura, K., Collins, V. P., Witt, H., Milde, T., Witt, O., Zhang, C., Castelo-Branco, P., Lichter, P., Faury, D., Tabori, U., Plass, C., Majewski, J., Pfister, S. M. and Jabado, N. (2012) 'Corrigendum: Driver mutations in histone H3.3 and chromatin remodelling genes in paediatric glioblastoma', Nature, 484(7392), pp. 130-130. doi: 10.1038/nature11026.
Segerman, A., Niklasson, M., Haglund, C., Bergström, T., Jarvius, M., Xie, Y., Westermark, A., Sönmez, D., Hermansson, A., Kastemar, M., Naimaie-Ali, Z., Nyberg, F., Berglund, M., Sundström, M., Hesselager, G., Uhrbom, L., Gustafsson, M., Larsson, R., Fryknäs, M., Segerman, B. and Westermark, B. (2016) 'Clonal Variation in Drug and Radiation Response among Glioma-Initiating Cells Is Linked to Proneural-Mesenchymal Transition', Cell Reports. Elsevier, 17(11), pp. 2994-3009. doi: 10.1016/j.celrep.2016.11.056.
Serrano, M., Lee, H. W., Chin, L., Cordon-Cardo, C., Beach, D. and DePinho, R. A. (1996) 'Role of the INK4a locus in tumor suppression and cell mortality', Cell, 85(1), pp. 27-37. doi: 10.1016/S0092-8674(00)81079-X.
Sfeir, A., Kabir, S., Van Overbeek, M., Celli, G. B. and De Lange, T. (2010) 'Loss of Rap1 induces telomere recombination in the absence of NHEJ or a DNA damage signal', Science, 327(5973), pp. 1657-1661. doi: 10.1126/science.1185100.
Sfeir, A., Kosiyatrakul, S. T., Hockemeyer, D., MacRae, S. L., Karlseder, J., Schildkraut, C. L. and de Lange, T. (2009) 'Mammalian Telomeres Resemble Fragile Sites and Require TRF1 for Efficient Replication', Cell, 138(1), pp. 90-103. doi: 10.1016/j.cell.2009.06.021.
Shay, J. W. and Bacchetti, S. (1997) 'A survey of telomerase activity in human cancer.', European journal of cancer (Oxford, England: 1990), 33(5), pp. 787-91. doi: 10.1016/S0959-8049(97)00062-2.
Shi, J., Yang, X. R., Ballew, B., Rotunno, M., Calista, D., Fargnoli, M. C., Ghiorzo, P., Bressac-de Paillerets, B., Nagore, E., Avril, M. F., Caporaso, N. E., McMaster, M. L., Cullen, M., Wang, Z., Zhang, X., Group, N. D. C. S. W., Laboratory, N. D. C. G. R., French Familial Melanoma Study, G., Bruno, W., Pastorino, L., Queirolo, P., Banuls-Roca, J., Garcia-Casado, Z., Vaysse, A., Mohamdi, H., Riazalhosseini, Y., Foglio, M., Jouenne, F., Hua, X., Hyland, P. L., Yin, J., Vallabhaneni, H., Chai, W., Minghetti, P., Pellegrini, C., Ravichandran, S., Eggermont, A., Lathrop, M., Peris, K., Scarra, G. B., Landi, G., Savage, S. A., Sampson, J. N., He, J., Yeager, M., Goldin, L. R., Demenais, F., Chanock, S. J., Tucker, M. A., Goldstein, A. M., Liu, Y. and Landi, M. T. (2014) 'Rare missense variants in POT1 predispose to familial cutaneous malignant melanoma', Nat Genet, 46(5), pp. 482-486. doi: 10.1038/ng.2941.
Singh, S. K., Hawkins, C., Clarke, I. D., Squire, J. A., Bayani, J., Hide, T., Henkelman, R. M., Cusimano, M. D. and Dirks, P. B. (2004) 'Identification of human brain tumour initiating cells', Nature, 432(7015), pp. 396-401. doi: 10.1038/nature03128.
Smith, S. (1998) 'Tankyrase, a Poly(ADP-Ribose) Polymerase at Human Telomeres', Science, 282(5393), pp. 1484-1487. doi: 10.1126/science.282.5393.1484.
Smogorzewska, A., Karlseder, J., Holtgreve-Grez, H., Jauch, A. and De Lange, T. (2002) 'DNA ligase IV-dependent NHEJ of deprotected mammalian telomeres in G1 and G2', Current Biology, 12(19), pp. 1635-1644. doi: 10.1016/S0960-9822(02)01179-X.
Smogorzewska, A. and de Lange, T. (2002) 'Different telomere damage signaling pathways in human and mouse cells.', The EMBO journal, 21(16), pp. 4338-48. doi: 10.1093/emboj/cdf433.
Smogorzewska, A., van Steensel, B., Bianchi, A., Oelmann, S., Schaefer, M. R., Schnapp, G. and de Lange, T. (2000) 'Control of Human Telomere Length by TRF1 and TRF2', Molecular and Cellular Biology, 20(5), pp. 1659-1668. doi: 10.1128/MCB.20.5.1659-1668.2000.
Soeda, A., Hara, A., Kunisada, T., Yoshimura, S., Iwama, T. and Park, D. M. (2015) 'The evidence of glioblastoma heterogeneity', Sci Rep, 5, p. 7979. doi: 10.1038/srep07979.
Sottoriva, A., Spiteri, I., Piccirillo, S. G. M., Touloumis, A., Collins, V. P., Marioni, J. C., Curtis, C., Watts, C. and Tavaré, S. (2013) 'Intratumor heterogeneity in human glioblastoma reflects cancer evolutionary dynamics.', Proceedings of the National Academy of Sciences of the United States of America, 110(10), pp. 4009-14. doi: 10.1073/pnas.1219747110.
Sundquist, W. I. and Klug, A. (1989) 'Telomeric DNA dimerizes by formation of guanine tetrads between hairpin loops', Nature, 342(6251), pp. 825-829. doi: 10.1038/342825a0.
Tabori, U., Ma, J., Carter, M., Zielenska, M., Rutka, J., Bouffet, E., Bartels, U., Malkin, D. and Hawkins, C. (2006) 'Human telomere reverse transcriptase expression predicts progression and survival in pediatric intracranial ependymoma', Journal of Clinical Oncology, 24(10), pp. 1522-1528. doi: 10.1200/JCO.2005.04.2127.
Tang, J., Kan, Z. Y., Yao, Y., Wang, Q., Hao, Y. H. and Tan, Z. (2008) 'G-quadruplex preferentially forms at the very 3' end of vertebrate telomeric DNA', Nucleic Acids Research, 36(4), pp. 1200-1208. doi: 10.1093/nar/gkm1137.
Tefferi, A., Lasho, T. L., Begna, K. H., Patnaik, M. M., Zblewski, D. L., Finke, C. M., Laborde, R. R., Wassie, E., Schimek, L., Hanson, C. a., Gangat, N., Wang, X. and Pardanani, A. (2015) 'A Pilot Study of the Telomerase Inhibitor Imetelstat for Myelofibrosis.', New England Journal of Medicine, 373(10), pp. 908-919. doi: 10.1056/NEJMoa1310523.
Tejera, A. M., Stagno d'Alcontres, M., Thanasoula, M., Marion, R. M., Martinez, P., Liao, C., Flores, J. M., Tarsounas, M. and Blasco, M. A. (2010) 'TPP1 is required for TERT recruitment, telomere elongation during nuclear reprogramming, and normal skin development in mice', Developmental Cell, 18(5), pp. 775-789. doi: 10.1016/j.devcel.2010.03.011.
Tomás-Loba, A., Flores, I., Fernandez-Marcos, P. J., Cayuela, M. L., Maraver, A., Tejera, A., Borrás, C., Matheu, A., Klatt, P., Flores, J. M., Viña, J., Serrano, M. and Blasco, M. A. (2008) 'Telomerase Reverse Transcriptase Delays Aging in Cancer-Resistant Mice', Cell, 135(4), pp. 609-622. doi: 10.1016/j.cell.2008.09.034.
Tsakiri, K. D., Cronkhite, J. T., Kuan, P. J., Xing, C., Raghu, G., Weissler, J. C., Rosenblatt, R. L., Shay, J. W. and Garcia, C. K. (2007) 'Adult-onset pulmonary fibrosis caused by mutations in telomerase', Proceedings of the National Academy of Sciences, 104(18), pp. 7552-7557. doi: 10.1073/pnas.0701009104.
Varela, E., Schneider, R. P., Ortega, S. and Blasco, M. A. (2011) 'Different telomere-length dynamics at the inner cell mass versus established embryonic stem (ES) cells', Proceedings of the National Academy of Sciences, 108(37), pp. 15207-15212. doi: 10.1073/pnas.1105414108.
Vaziri, H. and Benchimol, S. (1998) 'Reconstitution of telomerase activity in normal human cells leads to elongation of telomeres and extended replicative life span', Current Biology, 8(5), pp. 279-282. doi: 10.1016/S0960-9822(98)70109-5.
Verhaak, R. G. W., Hoadley, K. A., Purdom, E., Wang, V., Qi, Y., Wilkerson, M. D., Miller, C. R., Ding, L., Golub, T., Mesirov, J. P., Alexe, G., Lawrence, M., O'Kelly, M., Tamayo, P., Weir, B. A., Gabriel, S., Winckler, W., Gupta, S., Jakkula, L., Feiler, H. S., Hodgson, J. G., James, C. D., Sarkaria, J. N., Brennan, C., Kahn, A., Spellman, P. T., Wilson, R. K., Speed, T. P., Gray, J. W., Meyerson, M., Getz, G., Perou, C. M. and Hayes, D. N. (2010) 'Integrated Genomic Analysis Identifies Clinically Relevant Subtypes of Glioblastoma Characterized by Abnormalities in PDGFRA, IDH1, EGFR, and NF1', Cancer Cell, 17(1), pp. 98-110. doi: 10.1016/j.ccr.2009.12.020.
Wang, M., Wu, W., Wu, W., Rosidi, B., Zhang, L., Wang, H. and Iliakis, G. (2006) 'PARP-1 and Ku compete for repair of DNA double strand breaks by distinct NHEJ pathways', Nucleic Acids Research, 34(21), pp. 6170-6182. doi: 10.1093/nar/gkl840.
Wang, Q., Liu, J. Q., Chen, Z., Zheng, K. W., Chen, C. Y., Hao, Y. H. and Tan, Z. (2011) 'G-quadruplex formation at the 3' end of telomere DNA inhibits its extension by telomerase, polymerase and unwinding by helicase', Nucleic Acids Research, 39(14), pp. 6229-6237. doi: 10.1093/nar/gkr164.
Wen, P. Y. and Kesari, S. (2008) 'Malignant gliomas in .', The New England journal of medicine, 359(5), pp. 492-507. doi: 10.1056/NEJMra0708126.
Williamson, J. R., Raghuraman, M. K. and Cech, T. R. (1989) 'Monovalent cation-induced structure of telomeric DNA: The G-quartet model', Cell, 59(5), pp. 871-880. doi: 10.1016/0092-8674(89)90610-7.
Wu, P., Takai, H. and De Lange, T. (2012) 'Telomeric 3' overhangs derive from resection by Exo1 and apollo and fill-in by POT1b-associated CST', Cell, 150(1), pp. 39-52. doi: 10.1016/j.cell.2012.05.026.
Yamaguchi, H., Calado, R. T., Ly, H., Kajigaya, S., Baerlocher, G. M., Chanock, S. J., Lansdorp, P. M. and Young, N. S. (2005) 'Mutations in TERT, the gene for telomerase reverse transcriptase, in aplastic anemia.', The New England journal of medicine, 352(14), pp. 1413-24. doi: 10.1056/NEJMoa042980.
Yang, J., Chang, E., Cherry, A. M., Bangs, C. D., Oei, Y., Bodnar, A., Bronstein, A., Chiu, C. P. and Herron, G. S. (1999) 'Human endothelial cell life extension by telomerase expression.', The Journal of biological chemistry, 274(37), pp. 26141-8. doi: 10.1074/jbc.274.37.26141.
Yang, M. and Crawley, J. N. (2009) 'Simple behavioral assessment of mouse olfaction', Current Protocols in Neuroscience, (SUPPL. 48). doi: 10.1002/0471142301.ns0824s48.
Ye, J. Z. S., Donigian, J. R., Van Overbeek, M., Loayza, D., Luo, Y., Krutchinsky, A. N., Chait, B. T. and De Lange, T. (2004) 'TIN2 binds TRF1 and TRF2 simultaneously and stabilizes the TRF2 complex on telomeres', Journal of Biological Chemistry, 279(45), pp. 47264-47271. doi: 10.1074/jbc.M409047200.
Ye, J. Z. S., Hockemeyer, D., Krutchinsky, A. N., Loayza, D., Hooper, S. M., Chait, B. T. and De Lange, T. (2004) 'POT1-interaction protein PIP1: A telomere length regulator that recruits POT1 to the TIN2/TRF1 complex', Genes and Development, 18(14), pp. 1649-1654. doi: 10.1101/gad.1215404.
Zhang, J., Jima, D., Moffitt, A. B., Liu, Q., Czader, M., Hsi, E. D., Fedoriw, Y., Dunphy, C. H., Richards, K. L., Gill, J. I., Sun, Z., Love, C., Scotland, P., Lock, E., Levy, S., Hsu, D. S., Dunson, D. and Dave, S. S. (2014) 'The genomic landscape of mantle cell lymphoma is related to the epigenetically determined chromatin state of normal B cells', Blood, 123(19), pp. 2988-2996. doi: 10.1182/blood-2013-07-517177.
Zhou, X. Z. and Lu, K. P. (2001) 'The Pin2/TRF1-interacting protein PinX1 is a potent telomerase inhibitor', Cell, 107(3), pp. 347-359. doi: 10.1016/S0092-8674(01)00538-4.
Zhu, X. D., Küster, B., Mann, M., Petrini, J. H. J. and De Lange, T. (2000) 'Cell-cycle-regulated association of RAD50/MRE11/NBS1 with TRF2 and human telomeres', Nature Genetics, 25(3), pp. 347-352. doi: 10.1038/77139.
Zhu, X. D., Niedernhofer, L., Kuster, B., Mann, M., Hoeijmakers, J. H. J. and De Lange, T. (2003) 'ERCC1/XPF Removes the 3' Overhang from Uncapped Telomeres and Represses Formation of Telomeric DNA-Containing Double Minute Chromosomes', Molecular Cell, 12(6), pp. 1489-1498. doi: 10.1016/S1097-2765(03)00478-7.
Zijlmans, J. M., Martens, U. M., Poon, S. S., Raap, A. K., Tanke, H. J., Ward, R. K. and Lansdorp, P. M. (1997) 'Telomeres in the mouse have large inter-chromosomal variations in the number of T2AG3 repeats.', Proceedings of the National Academy of Sciences of the United States of America, 94(14), pp. 7423-8. doi: 10.1073/pnas.94.14.7423.

## Claims

1. A composition which comprises at least a TRF1 inhibitor for use in the treatment or prevention of glioblastoma multiforme or another brain tumor and, optionally, a pharmaceutically acceptable excipient, diluent or carrier.

2. A composition which comprises at least a TRF1 inhibitor for use according to claim 1, wherein the composition comprises at least a first and a second TRF1 inhibitor and at least one of the TRF1 inhibitor is an inhibitor of TRF1 which decreases TRF1 protein levels.

3. A composition which comprises at least a TRF1 inhibitor for use according to claim 1 or 2, wherein at least a TRF1 inhibitor is selected of the group of:
a) a compound which is an inhibitor of PI3K, or
b) a compound of Formula I:

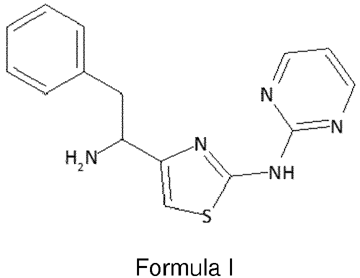

4. A composition which comprises at least a TRF1 inhibitor for use according to claim 3, wherein at least a TRF1 inhibitor is an inhibitor of PI3K which is selected from the group of:
a)
i) a compound of Formula II 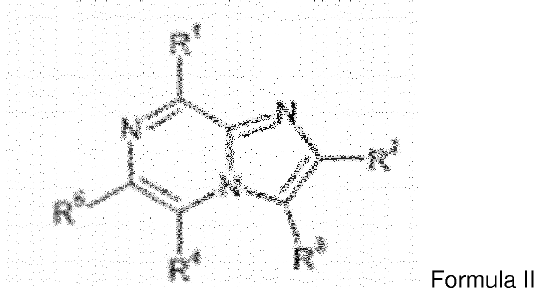
wherein:
R¹ represents:
(a) -N(R^{1a})R^{1b}, in which R^{1a} and R^{1b} are linked together to form, together with the nitrogen atom to which they are necessarily attached, a 5- to 7-membered ring optionally containing a further one or two heteroatoms, optionally containing one or two double bonds, and which ring is optionally substituted by one or more substituents selected from =O and B¹;
(b) a heterocycloalkyl group (attached to the requisite imidazopyrazine via a carbon atom), optionally substituted by one or more substituents selected from =O and B²;
(c) a monocyclic heteroaryl group optionally substituted by one or more substituents selected from B³;
R² and R³ independently represent:
(i) hydrogen;
(ii) Q¹;
(iii) C₁₋-_{I2} alkyl optionally substituted by one or more substituents selected from =O, =S, =N(R^{10a}) and Q²; or
R² or R³ may represent a fragment of Formula IIR 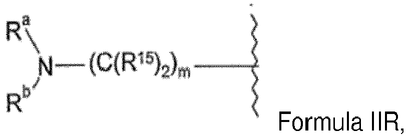 wherein
m represents 0, 1, 2, 3, 4, 5 or 6;
each R¹⁵ represents hydrogen, halo or C₁₋₆ alkyl optionally substituted by one or more substituents selected from E¹; or
the two R¹⁵ groups may linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E²;
R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
(a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring;
(b) comprises a linker group -(C(R^{X})₂)_{P}- and/or -(C(R^{x})₂)r-O-(C(R^{x})₂)s- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
(c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
all of which cyclic groups, defined by the linkage of R^{a} and R^{b}, are optionally substituted by one or more substituents selected from =O and E³;
R⁴ represents hydrogen or a substituent selected from halo, -CN, -OR^{10b}, -N(R^{10b})R^{11b}, - C(O)N(R^{10b})R^{11b}, -C(O)R^{10b}, C₁₋₆ alkyl and heterocycloalkyl, which latter two groups are optionally substituted by one or more substituents selected from E⁴ and =O;
but wherein at least one of R², R³ and R⁴ represents a substituent other than hydrogen;
R⁵ represents aryl or heteroaryl (both of which are optionally substituted by one or more substituents selected from E⁵);
each Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -OC(=Y)-R^{10a}, -OC(=Y)-OR^{10a}, -OC(=Y)N(R^{10a})R^{11a}, -OS(O)₂OR¹⁰³, -OP(=Y)(OR^{10a})(OR^{11a}), -OP(OR^{10a})(OR^{11a}), -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R¹⁰³, -SR^{10a}, -S(O)R^{10a}, C_{1-I2} alkyl, heterocycloalkyl (which latter two groups are optionally substituted by one or more substituents selected from =O, =S, =N(R^{10a}) and E⁶), aryl or heteroaryl (which latter two groups are optionally substituted by one or more substituents selected from E⁷);
each B¹, B² and B³ independently represent halo, -NO₂, -CN, -N(R^{10a})R^{11a}, -OR^{10a}, -C(=Y)-R^{10a}, -C(=Y)-OR^{10a}, -C(=Y)N(R^{10a})R^{11a}, -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -SC(=Y)OR^{10a}, -S(O)₂R^{10a}, C₁₋₁₂ alkyl, heterocycloalkyl (which latter two groups are optionally substituted by one or more substituents selected from =O and E⁸), aryl or heteroaryl (which latter two groups are optionally substituted by one or more substituents selected from E⁹);
or, any two B¹ substituents, when attached to the same carbon atom (thereby forming a spiro-cycle), may be linked together to form, a 3- to 12- membered ring, optionally containing one or more heteroatoms, which ring optionally contains one or more double bonds, and which ring is itself optionally substituted by one or more substituents selected from halo, =O and C₁₋₃ alkyl optionally substituted by one or more fluoro atoms;
each R^{10a}, R^{11a}, R^{12a}, R^{10b} and R^{11b} independently represent, on each occasion when used herein, hydrogen, C^₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a}, R^{11a} and R^{12a} and/or any pair of R^{10b} and R^{11b} may be linked together to form a 3- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁶, E⁷, E⁸, E⁹, E¹⁰, E¹¹ and E¹² independently represents, on each occasion when used herein:
(i) Q⁴;
(ii) C_{1-I2} alkyl optionally substituted by one or more substituents selected from =O and Q⁵;
or
any two E¹, E², E³, E⁶, E⁷, E⁸, E⁹, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), -OP(OR²⁰XOR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or any relevant pair of R²⁰, R²¹ and R²², may be linked together to form a 3- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: -CN, halo, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂R⁵⁰, -SR⁵⁰, -S(O)R⁵⁰ or C₁₋₆ alkyl optionally substituted by one or more fluoro atoms;
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms;
or a pharmaceutically acceptable ester, amide, solvate or salt thereof;
a) ii) a compound of Formula III 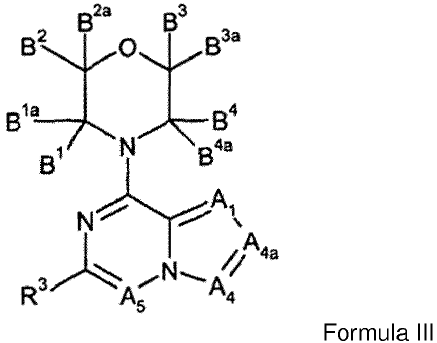
wherein
A₁ represents N or C(R¹);
A₄ represents N or C(R^{1a});
A4a represents N or C(R^{1b});
wherein at least one of A₄ and A₄ₐ does not represent N;
A5 represents N or C(R²);
each B¹, B^{1a}, B², B^{2a}, B³, B^{3a}, B⁴ and B^{4a} independently represent hydrogen or a substituent selected from halo, -C(=Y)-R^{10a}, -C(=Y)-OR^{10a}, -C(=Y)N(R^{10a})R^{11a}, -S(O)₂N(R^{10a})R^{11a}, C₁₋₁₂ alkyl, heterocycloalkyl (which latter two groups are optionally substituted by one or more substituents selected from =O and E¹), aryl and/or heteroaryl (which latter two groups are optionally substituted by one or more substituents selected from E²); or
any two B¹, B^{1a}, B², B^{2a}, B³, B^{3a}, B⁴ and B^{4a} substituents that are attached to the same carbon atom (i.e. B¹ and B^{1a}; B² and B^{2a}; B³ and B^{3a}; and/or B⁴ and B^{4a}) may together form a =O group;
or, any two B¹, B^{1a}, B², B^{2a}, B³, B^{3a}, B⁴ and B^{4a} substituents may be linked together to form a further 3- to 12- membered ring, optionally containing (in addition to the atom(s) of the morpholine ring) one or more heteroatom(s), which ring optionally contains one or more double bonds, and which ring is itself optionally substituted by one or more substituents selected from halo, =O and C₁₋₃ alkyl optionally substituted by one or more fluoro atoms; R¹ and R² independently represents hydrogen or a substituent selected from halo, -CN,-OR^{10b}, -N(R^{10b})R^{11b}, -C(O)N(R^{10b})R^{11b}, C₁₋₁₂ (e.g. C₁₋₆) alkyl and heterocycloalkyl, which latter two groups are optionally substituted by one or more substituents selected from E³ and =O;
R^{1b} (when present) represents:
(i) C₁₋₁₂ alkyl optionally substituted by one or more substituents selected from Q^{1a};
(ii) heterocycloalkyl (linked via a carbon atom) optionally substituted by one or more substituents selected from =O and Q^{1b}; or
(iii) a fragment of formula IIIR;
R^{1a} (when present) represents:
(i) hydrogen;
(ii) Q¹;
(iii) C₁₋₁₂ alkyl optionally substituted by one or more substituents selected from =O, =S, =N(R^{10a}) and Q²; or
(iv) a fragment of formula IIIR;
the fragment of formula IIIR represents: 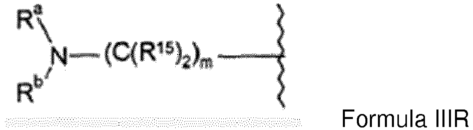 wherein:
m represents 1, 2, 3, 4, 5 or 6;
each R¹⁵ represents hydrogen, halo or C₁₋₆ alkyl optionally substituted by more substituents selected from E⁴; or
the two R¹⁵ groups may be linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E⁵;
R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
(a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring (in which the heteroatoms are preferably selected from sulfur and, especially, nitrogen and oxygen);
(b) comprises a linker group -(C(R^{x})₂)ₚ- and/or -(C(R^{x})₂)ᵣ-O-(C(R^{x})2)ₛ- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
(c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
all of which cyclic groups, defined by the linkage of R^{a} and R^{b}, are optionally substituted by one or more substituents selected from =O, =NOR^{10a} and E⁶;
R³ represents aryl or heteroaryl (both of which are optionally substituted by one or more substituents selected from E⁷);
each Q^{1a}, Q^{1b}, Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a},-C(=Y)N(R^{10a})-OR^{11c}, -OC(=Y)-R^{10a}, -OC(=Y)-OR^{10a}, -OC(=Y)N(R^{10a})R^{11a}, -OS(O)₂OR^{10a},-OP(=Y)(OR^{10a})(OR^{11a}), -OP(OR^{10a})(OR^{11a}), -N(R^{12a})C(=Y)R^{11a}, -N(R^{12a})C(=Y)OR^{11a},-N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a},-SC(=Y)R^{10a}, -S(O)₂R^{10a}, -SR^{10a}, -S(O)R^{10a}, C₁₋₁₂ alkyl, heterocycloalkyl (which latter two groups are optionally substituted by one or more substituents selected from =O, =S, =N(R^{10a}) and E⁸), aryl or heteroaryl (which latter two groups are optionally substituted by one or more substituents selected from E⁹);
each R^{11c} independently represents C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹);
each R^{10a}, R^{11a}, R^{10b}, R^{11b} and R^{12a} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a} and R^{11a} or R^{10b} and R^{11b} may be linked together to form a 4- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁴, E⁵, E⁶, E⁷, E⁸, E¹⁰, E¹¹ and E¹² independently represents, on each occasion when used herein:
(i) Q⁴;
(ii) C₁₋₁₂ alkyl optionally substituted by one or more substituents selected from =O and Q⁵;
or
any two E¹, E², E³, E⁴, E⁵, E⁶, E⁷, E⁸, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -C(=Y)N(R²⁰)-O-R^{21a}, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), - OP(OR²⁰)(OR²¹), -N(R²²)C(=Y)R²¹, -N(R²²)C(=Y)OR²¹, -N(R²²)C(=Y)N(R²⁰)R²¹, - NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, - S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R^{21a} represents C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹ and R²², may be linked together to form a 4- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: halo, -CN, -N(R⁵⁰ R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂N(R⁵⁰)R⁵¹, -N(R⁵²)-C(=Y^{a})-N(R⁵⁰)R⁵¹, -S(O)₂R⁵⁰, -SR⁵⁰, -S(O)R⁵⁰, C₁₋₆ alkyl (optionally substituted by one or more fluoro atoms), heterocycloalkyl, aryl or heteroaryl (which latter three groups are optionally substituted by one or more substituents selected from halo, -OR⁶⁰ and -N(R⁶¹)R⁶²);
each Y³ independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
each R⁶⁰, R⁶¹ and R⁶² independently represent hydrogen or C₁₋₆ alkyl optionally substituted by one or more fluoro atoms,
or a pharmaceutically acceptable ester, amide, solvate or salt thereof.

5. A composition which comprises at least a TRF1 inhibitor for use according to any one of claims 2 to 4, wherein at least a second TRF1 inhibitor is present, which second TRF1 inhibitor is selected of the group of: an Aurora inhibitor, an RTK inhibitor, a MEK inhibitor, an ERK inhibitor, an mTOR inhibitor, a CDK inhibitor, an HSP90 inhibitor, docetaxel and gemcitabine.

6. A composition which comprises at least a TRF1 inhibitor for use according to any one of claims 4 or 5, wherein the composition comprises at least a first and a second TRF1 inhibitor and
the first TRF1 inhibitor is the PI3K inhibitor of the following formula 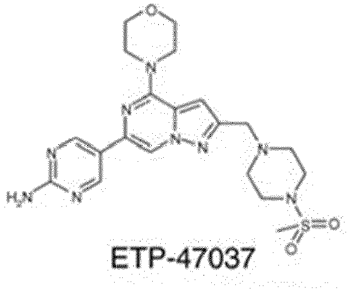 and
the second TRF1 the inhibitor of TRF1 which decreases TRF1 protein levels is selected from the group of: an RTK inhibitor, a MEK inhibitor, an ERK inhibitor, an HSP90 inhibitor, docetaxel and gemcitabine.

7. A composition which comprises at least a TRF1 inhibitor for use according to any one of claims 4 or 5, wherein the composition comprises at least a first and a second TRF1 inhibitor and
the first TRF1 inhibitor is a PI3K inhibitor, and
the second TRF1 inhibitor is selected from the group of: Geldanamycin, Docetaxel, Gemcitabine, Alisertib, Dasatinib, GSK461364, KU-0063794, SCH772984, Selumetinib, Flavopiridol.

8. A composition which comprises at least a TRF1 inhibitor for use according to any one of claims 1, 3 or 4, which additionally comprises another antitumoral compound.

9. A composition which comprises at least a TRF1 inhibitor for use according to claim 8, which additionally comprises an antitumoral compound which is used for glioblastoma multiforme treatment.

10. A composition which comprises at least a TRF1 inhibitor for use according to claim 9, which additionally comprises temozolomide.

11. A compound which is a TRF1 inhibitor for use in the treatment or prevention of a brain tumor.

12. A compound which is a TRF1 inhibitor for use according to claim 11, for use in blocking, diminishing or slowing the progression of a glioblastoma tumor.

13. A compound which is TRF1 inhibitor for use according to claim 11 or 12, wherein the compound is a compound which decreases TRF1 protein levels in a cell.

14. A compound which is a TRF1 inhibitor for use according to any one of claims 11 to 13, wherein the compound is the compound of Formula I:

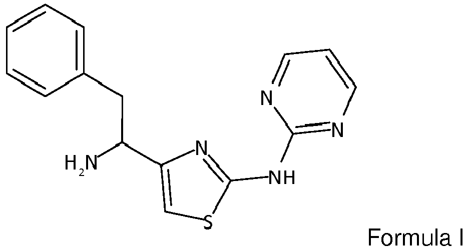

15. A compound which is a TRF1 inhibitor for use according to any one of claims 11 to 13, wherein the compound is an inhibitor of PI3K.

16. A compound which is a TRF1 inhibitor for use according to claim 15, wherein the compound is a compound of Formula II 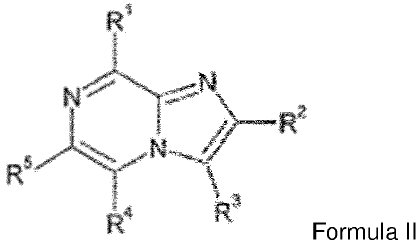 wherein:
R¹ represents:
(a) -N(R^{1a})R^{1b}, in which R^{1a} and R^{1b} are linked together to form, together with the nitrogen atom to which they are necessarily attached, a 5- to 7-membered ring optionally containing a further one or two heteroatoms, optionally containing one or two double bonds, and which ring is optionally substituted by one or more substituents selected from =O and B¹;
(b) a heterocycloalkyl group (attached to the requisite imidazopyrazine via a carbon atom), optionally substituted by one or more substituents selected from =O and B²;
(c) a monocyclic heteroaryl group optionally substituted by one or more substituents selected from B³;
R² and R³ independently represent:
(i) hydrogen;
(ii) Q¹;
(iii) C₁₋-_{I2} alkyl optionally substituted by one or more substituents selected from =O, =S, =N(R^{10a}) and Q²; or
R² or R³ may represent a fragment of Formula IIR 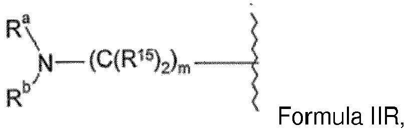
wherein
m represents 0, 1, 2, 3, 4, 5 or 6;
each R¹⁵ represents hydrogen, halo or C₁₋₆ alkyl optionally substituted by one or more substituents selected from E¹; or
the two R¹⁵ groups may linked together to form (along with the requisite carbon atom to which those R¹⁵ groups are necessarily attached) a 3- to 6-membered (spiro-cyclic) ring,
which ring optionally contains one or more double bonds, and optionally contains a further heteroatom selected from nitrogen, sulfur and oxygen, and which ring is optionally substituted by one or more substituents selected from E²;
R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
(a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring;
(b) comprises a linker group -(C(R^{X})₂)_{P}- and/or -(C(R^{x})₂)r-O-(C(R^{x})₂)s- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
(c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
all of which cyclic groups, defined by the linkage of R^{a} and R^{b}, are optionally substituted by one or more substituents selected from =O and E³;
R⁴ represents hydrogen or a substituent selected from halo, -CN, -OR^{10b}, -N(R^{10b})R^{11b}, - C(O)N(R^{10b})R^{11b}, -C(O)R^{10b}, C₁₋₆ alkyl and heterocycloalkyl, which latter two groups are optionally substituted by one or more substituents selected from E⁴ and =O;
but wherein at least one of R², R³ and R⁴ represents a substituent other than hydrogen;
R⁵ represents aryl or heteroaryl (both of which are optionally substituted by one or more substituents selected from E⁵);
each Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -OC(=Y)-R^{10a}, -OC(=Y)-OR^{10a}, -OC(=Y)N(R^{10a})R^{11a}, -OS(O)₂OR¹⁰³, -OP(=Y)(OR^{10a})(OR^{11a}), -OP(OR^{10a})(OR^{11a}), -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R¹⁰³, -SR^{10a}, -S(O)R^{10a}, C_{1-I2} alkyl, heterocycloalkyl (which latter two groups are optionally substituted by one or more substituents selected from =O, =S, =N(R^{10a}) and E⁶), aryl or heteroaryl (which latter two groups are optionally substituted by one or more substituents selected from E⁷);
each B¹, B² and B³ independently represent halo, -NO₂, -CN, -N(R^{10a})R^{11a}, -OR^{10a}, -C(=Y)-R^{10a}, -C(=Y)-OR^{10a}, -C(=Y)N(R^{10a})R^{11a}, -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -SC(=Y)OR^{10a}, -S(O)₂R^{10a}, C₁₋₁₂ alkyl, heterocycloalkyl (which latter two groups are optionally substituted by one or more substituents selected from =O and E⁸), aryl or heteroaryl (which latter two groups are optionally substituted by one or more substituents selected from E⁹);
or, any two B¹ substituents, when attached to the same carbon atom (thereby forming a spiro-cycle), may be linked together to form, a 3- to 12- membered ring, optionally containing one or more heteroatoms, which ring optionally contains one or more double bonds, and which ring is itself optionally substituted by one or more substituents selected from halo, =O and C₁₋₃ alkyl optionally substituted by one or more fluoro atoms;
each R^{10a}, R^{11a}, R^{12a}, R^{10b} and R^{11b} independently represent, on each occasion when used herein, hydrogen, C^₂ alkyl, heterocycloalkyl (which latter two groups are optionally substituted by one or more substituents selected from =O, =S, =N(R²⁰) and E¹⁰), aryl or
heteroaryl (which latter two groups are optionally substituted by one or more substituents selected from E¹¹); or
any relevant pair of R^{10a}, R^{11a} and R^{12a} and/or any pair of R^{10b} and R^{11b} may be linked together to form a 3- to 20-membered ring, optionally containing one or more heteroatoms,
optionally containing one or more unsaturations, and which ring is optionally substituted by one or more substituents selected from =O, =S, =N(R²⁰) and E¹²;
each E¹, E², E³, E⁶, E⁷, E⁸, E⁹, E¹⁰, E¹¹ and E¹² independently represents, on each occasion when used herein:
(i) Q⁴;
(ii) C_{1-I2} alkyl optionally substituted by one or more substituents selected from =O and Q⁵;
or
any two E¹, E², E³, E⁶, E⁷, E⁸, E⁹, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), -OP(OR²⁰XOR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)2N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or any relevant pair of R²⁰, R²¹ and R²², may be linked together to form a 3- to 20-membered ring,
optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: -CN, halo, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂R⁵⁰, -SR⁵⁰, -S(O)R⁵⁰ or C₁₋₆ alkyl optionally substituted by one or more fluoro atoms;
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms;
or a pharmaceutically acceptable ester, amide, solvate or salt thereof.

17. A compound which is a TRF1 inhibitor for use according to claim 16, wherein the compound is a compound of Formula Ila 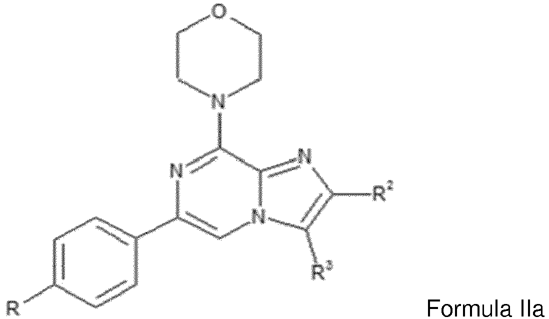 wherein,
R represents NH-CO-NH-Ph, where the later Ph (phenyl) group is substituted in the p-position with a group CO-piperazinyl-additionally substituted in the 4-piperazinyl N with methyl;
R² and R³ independently represent:
(i) hydrogen;
(ii) Q¹;
(iii) C₁₋₁₂ alkyl optionally substituted by one or more substituents selected from =O, =S, =N(R^{10a}) and Q²; or
R² or R³ may represent a fragment of Formula IIR 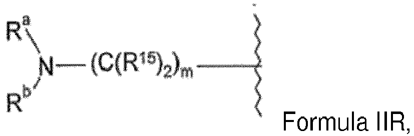 wherein
m represents 0, 1, 2, 3, 4, 5 or 6;
each R¹⁵ represents hydrogen, halo or C₁₋₆ alkyl optionally substituted by one or more substituents selected from E¹; or the two R¹⁵ groups may linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E²_{;}
R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
(a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring;
(b) comprises a linker group -(C(R^{X})₂)_{P}- and/or -(C(R^{x})₂)r-O-(C(R^{x})₂)s- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
(c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle), all of which cyclic groups, defined by the linkage of R^{a} and R^{b}, are optionally substituted by one or more substituents selected from =O and E³;
each Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -OC(=Y)-R^{10a}, -OC(=Y)-OR^{10a}, -OC(=Y)N(R^{10a})R^{11a}, -OS(O)₂OR¹⁰³, -OP(=Y)(OR^{10a})(OR^{11a}), -OP(OR^{10a})(OR^{11a}), -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂N(R^{10a}), -SR^{10a}, -S(O)R^{10a}, C₁₋₁₂ alkyl, heterocycloalkyl (which latter two groups are optionally substituted by one or more substituents selected from =O, =S, =N(R^{10a}) and E⁶), aryl or heteroaryl (which latter two groups are optionally substituted by one or more substituents selected from E⁷);
each R^{10a}, R^{11a}, R^{12a}, R^{10b} and R^{11b} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a}, R^{11a} and R^{12a} and/or any pair of R^{10b} and R^{11b} may be linked together to form a 3- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁶, E⁷, E⁸, E⁹, E¹⁰, E¹¹ and E¹² independently represents, on each occasion when used herein:
(i) Q⁴;
(ii) C₁₋₁₂ alkyl optionally substituted by one or more substituents selected from =O and Q⁵;
or
any two E¹, E², E³, E⁶, E⁷, E⁸, E⁹, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰,-OC(=Y)-OR²⁰, -OC(=Y)N(R²⁰)R²¹, -OS(O)₂OR²⁰, -OP(=Y)(OR²⁰)(OR²¹), -OP(OR²⁰XOR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹, and R²², may be linked together to form a 3- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: -CN, halo, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂R⁵⁰, -SR⁵⁰, -S(O)R⁵⁰ or C₁₋₆ alkyl optionally substituted by one or more fluoro atoms;
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms;
or a pharmaceutically acceptable ester, amide, solvate or salt thereof.

18. A compound which is a TRF1 inhibitor for use according to claim 16 or 17, wherein the compound is the compound of the following formula:

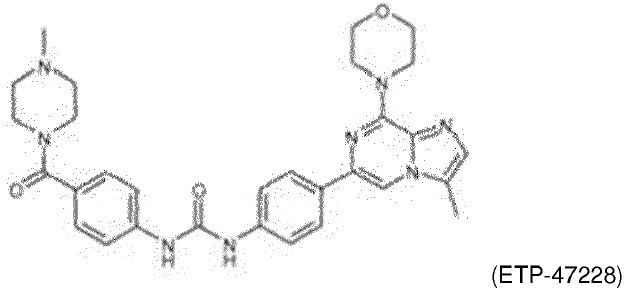

19. A compound which is a TRF1 inhibitor for use according to claim 15, wherein the compound is a compound of Formula III 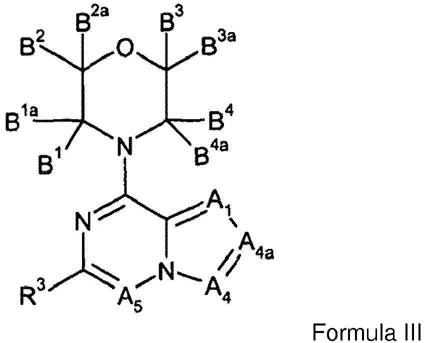 wherein
A₁ represents N or C(R¹);
A₄ represents N or C(R^{1a});
A₄ₐ represents N or C(R^{1b});
wherein at least one of A₄ and A₄ₐ does not represent N;
A₅ represents N or C(R²);
each B¹, B¹a, B², B^{2a}, B³, B^{3a}, B⁴ and B^{4a} independently represent hydrogen or a substituent selected from halo, -C(=Y)-R^{10a}, -C(=Y)-OR^{10a}, -C(=Y)N(R^{10a})R^{11a}, -S(O)₂N(R^{10a})R^{11a}, C₁₋₁₂ alkyl, heterocycloalkyl (which latter two groups are optionally substituted by one or more substituents selected from =O and E¹), aryl and/or heteroaryl (which latter two groups are optionally substituted by one or more substituents selected from E²); or
any two B¹, B¹a, B², B^{2a}, B³, B^{3a}, B⁴ and B^{4a} substituents that are attached to the same carbon atom (i.e. B¹ and B^{1a}; B² and B^{2a}; B³ and B^{3a}; and/or B⁴ and B^{4a}) may together form a =O group; or, any two B¹, B¹a, B², B^{2a}, B³, B^{3a}, B⁴ and B^{4a} substituents may be linked together to form a further 3- to 12- membered ring, optionally containing (in addition to the atom(s) of the morpholine ring) one or more heteroatom(s), which ring optionally contains one or more double bonds, and which ring is itself optionally substituted by one or more substituents selected from halo, =O and C₁₋₃ alkyl optionally substituted by one or more fluoro atoms;
R¹ and R² independently represents hydrogen or a substituent selected from halo, -CN,-OR^{10b}, -N(R)¹⁰R^{11b}, -C(O)N(R^{10b})R^{11b}, C₁₋₁₂ (e.g. C₁₋₆) alkyl and heterocycloalkyl, which latter two groups are optionally substituted by one or more substituents selected from E³ and =O;
R^{1b} (when present) represents:
(i) C₁₋₁₂ alkyl optionally substituted by one or more substituents selected from Q^{1a};
(ii) heterocycloalkyl (linked via a carbon atom) optionally substituted by one or more substituents selected from =O and Q^{1b}; or
(iii) a fragment of formula IIIR;
R^{1a} (when present) represents:
(i) hydrogen;
(ii) Q¹;
(iii) C₁₋₁₂ alkyl optionally substituted by one or more substituents selected from =O, =S, =N(R^{10a}) and Q²; or
(iv) a fragment of formula IIIR;
the fragment of formula IIIR represents: 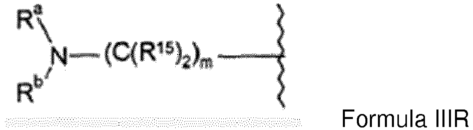 wherein:
m represents 1, 2, 3, 4, 5 or 6;
each R¹⁵ represents hydrogen, halo or C₁₋₆ alkyl optionally substituted by more substituents selected from E⁴; or
the two R¹⁵ groups may be linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E⁵;
R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
(a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring (in which the heteroatoms are preferably selected from sulfur and, especially, nitrogen and oxygen);
(b) comprises a linker group -(C(R^{x})₂)ₚ- and/or -(C(R^{x})₂)ᵣO-(C(R^{x})2)ₛ- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
(c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
all of which cyclic groups, defined by the linkage of R^{a} and R^{b}, are optionally substituted by one or more substituents selected from =O, =NOR^{10a} and E⁶;
R³ represents aryl or heteroaryl (both of which are optionally substituted by one or more substituents selected from E⁷);
each Q^{1a}, Q^{1b}, Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a},-C(=Y)N(R^{10a})-OR^{11c}, OC(=Y)-R^{10a}, -OC(=Y)-OR^{10a}, -OC(=Y)N(R^{10a})R^{11a}, -OS(O)₂OR^{10a},-OP(=Y)(OR^{10a})(OR^{11a}), -OP(OR^{10a})(OR^{11a}), -N(R^{12a})C(=Y)R^{11a}, -N(R^{12a})C(=Y)OR^{11a},-N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a},-SC(=Y)R^{10a}, -S(O)₂R^{10a}, -SR^{10a}, -S(O)R^{10a}, C₁₋₁₂ alkyl, heterocycloalkyl (which latter two groups are optionally substituted by one or more substituents selected from =O, =S, =N(R^{10a}) and E⁸), aryl or heteroaryl (which latter two groups are optionally substituted by one or more substituents selected from E⁹);
each R^{11c} independently represents C₁-₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹);
each R^{10a}, R^{11a}, R^{10b}, R^{11b} and R^{12a} independently represent, on each occasion when used herein, hydrogen, C₁-₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a} and R^{11a} or R^{10b} and R^{11b} may be linked together to form a 4- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁴, E⁵, E⁶, E⁷, E⁸, E¹⁰, E¹¹ and E¹² independently represents, on each occasion when used herein:
(i) Q⁴;
(ii) C₁₋₁₂ alkyl optionally substituted by one or more substituents selected from =O and Q⁵;
or
any two E¹, E², E³, E⁴, E⁵, E⁶, E⁷, E⁸, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -C(=Y)N(R²⁰)-O-R^{21a}, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹),-OP(OR²⁰)(OR²¹), -N(R²²)C(=Y)R²¹, -N(R²²)C(=Y)OR²¹, -N(R²²)C(=Y)N(R²⁰)R²¹,-NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰,-S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R^{21a} represents C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹, and R²², may be linked together to form a 4- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: halo, -CN, -N(R⁵⁰R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂N(R⁵⁰)R⁵¹, -N(R⁵²)-C(=Y^{a})-N(R⁵⁰)R⁵¹, -S(O)₂R⁵⁰, -SR⁵⁰, -S(O)R⁵⁰, C₁₋₆ alkyl (optionally substituted by one or more fluoro atoms), heterocycloalkyl, aryl or heteroaryl (which latter three groups are optionally substituted by one or more substituents selected from halo, -OR⁶⁰ and -N(R⁶¹)R⁶²);
each Y³ independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
each R⁶⁰, R⁶¹ and R⁶² independently represent hydrogen or C₁₋₆ alkyl optionally substituted by one or more fluoro atoms,
or a pharmaceutically acceptable ester, amide, solvate or salt thereof.

20. A compound which is a TRF1 inhibitor for use according to claim 18, wherein the compound is a compound of Formula IIIa 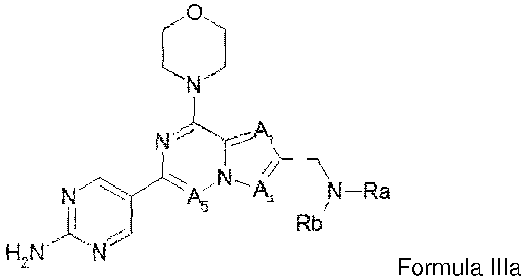 wherein
A₁ represents N or C(R¹);
A₄ represents N or C(R^{1a});
wherein at least one of A₄ and A₄ₐ does not represent N;
A5 represents N or C(R²);
R¹ and R² independently represents hydrogen or a substituent selected from halo, -CN,-OR^{10b}, -N(R^{10b})R^{11b}, -C(O)N(R^{10b})R^{11b}, C₁₋₁₂ (e.g. C₁₋₆) alkyl and heterocycloalkyl, which latter two groups are optionally substituted by one or more substituents selected from E³ and =O;
R^{1a} (when present) represents:
(i) hydrogen;
(ii) Q¹;
(iii) C₁-₁₂ alkyl optionally substituted by one or more substituents selected from =O, =S, =N(R^{10a}) and Q²; or
(iv) a fragment of formula IIIR;
the fragment of formula IIIR represents: 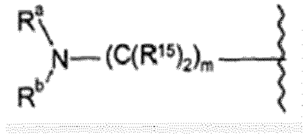
wherein:
m represents 1, 2, 3, 4, 5 or 6;
each R¹⁵ represents hydrogen, halo or C₁₋₆ alkyl optionally substituted by more substituents selected from E⁴; or
the two R¹⁵ groups may be linked together to form (along with the requisite carbon atom to which those R¹⁵ groups are necessarily attached) a 3- to 6-membered (spiro-cyclic) ring, which ring optionally contains one or more double bonds, and optionally contains a further heteroatom selected from nitrogen, sulfur and oxygen, and which ring is optionally substituted by one or more substituents selected from E⁵;
R^{a} and R^{b} are linked together, along with the requisite nitrogen atom to which they are necessarily attached, to form a first 3- to 7-membered cyclic group, optionally containing one further heteroatom selected from nitrogen, sulfur and oxygen, and which ring:
(a) is fused to a second ring that is either a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring (in which the heteroatoms are preferably selected from sulfur and, especially, nitrogen and oxygen);
(b) comprises a linker group -(C(R^{x})₂)ₚ- and/or -(C(R^{x})₂)ᵣ-O-(C(R^{x})2)ₛ- (wherein p is 1 or 2; r is 0 or 1; s is 0 or 1; and each R^{x} independently represents hydrogen or C₁₋₆ alkyl), linking together any two non-adjacent atoms of the first 3- to 7-membered ring (i.e. forming a bridged structure); or
(c) comprises a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring via a single carbon atom common to both rings (i.e. forming a spiro-cycle),
all of which cyclic groups, defined by the linkage of R^{a} and R^{b}, are optionally substituted by one or more substituents selected from =O, =NOR^{10a} and E⁶;
each Q¹ and Q² independently represents, on each occasion when used herein: halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -C(=Y)N(R^{10a})-OR^{11c}, -OC(=Y)-R^{10a}, -OC(=Y)-OR^{10a}, -OC(=Y)N(R^{10a})R^{11a}, -OS(O)₂OR^{10a}, -OP(=Y)OR^{10a})(OP(OR^{11a}), -OP(OR^{10a})(OR^{11a}), -N(R^{12a})C(=Y)R^{11a}, -N(R^{12a})C=(Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R^{10a}, -SR^{10a}, -S(O)R^{10a}, C₁₋₁₂ alkyl, heterocycloalkyl (which latter two groups are optionally substituted by one or more substituents selected from =O, =S, =N(R^{10a}) and E⁸), aryl or heteroaryl (which latter two groups are optionally substituted by one or more substituents selected from E⁹);
each R^{11c} independently represents C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹);
each R^{10a}, R^{11a}, R^{10b}, R^{11b} and R^{12a} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a} and R^{11a} or R^{10b} and R^{11b} may be linked together to form a 4- to 20-membered ring, optionally containing one or more heteroatoms, optionally containing one or more unsaturations, and which ring is optionally substituted by one or more substituents selected from =O, =S, =N(R²⁰) and E¹²;
each E³, E⁴, E⁵, E⁶, E⁷, E⁸, E¹⁰, E¹¹ and E¹² independently represents, on each occasion when used herein:
(i) Q⁴;
(ii) C₁₋₁₂ alkyl optionally substituted by one or more substituents selected from =O and Q⁵;
or
any two E¹, E², E³, E⁴, E⁵, E⁶, E⁷, E⁸, E¹⁰, E¹¹ or E¹² groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -C(=Y)N(R²⁰)-O-R^{21a}, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹),-OP(OR²⁰)(OR²¹), -N(R²²)C(=Y)R²¹, -N(R²²)C(=Y)OR²¹, -N(R²²)C(=Y)N(R²⁰)R²¹,-NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰,-S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R^{21a} represents C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵);
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹, and R²², may be linked together to form a 4- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: halo,-CN, -N(R⁵⁰R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹,-NR⁵²S(O)₂R⁵⁰, -S(O)₂N(R⁵⁰)R⁵¹, -N(R⁵²)-C(=Y^{a})-N(R⁵⁰)R⁵¹, -S(O)₂R⁵⁰, -SR⁵⁰, -S(O)R⁵⁰, C₁₋₆ alkyl (optionally substituted by one or more fluoro atoms), heterocycloalkyl, aryl or heteroaryl (which latter three groups are optionally substituted by one or more substituents selected from halo, -OR⁶⁰ and -N(R⁶¹)R⁶²);
each Y³ independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
each R⁶⁰, R⁶¹ and R⁶² independently represent hydrogen or C₁₋₆ alkyl optionally substituted by one or more fluoro atoms,
or a pharmaceutically acceptable ester, amide, solvate or salt thereof

21. A compound which is a TRF1 inhibitor for use according to claim 20, wherein the compound is the compound of the following formula:

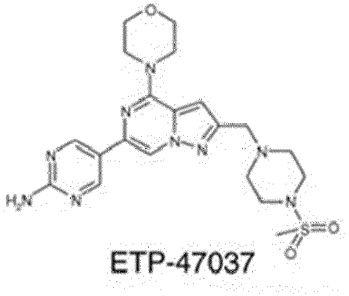

22. A method for identifying a compound as a candidate for use in the prevention or treatment of glioblastoma, which comprises a step wherein it is determined that the compound inhibits or decreases TRF1 activity.

23. The method according to claim 22, wherein it is determined that the compound inhibits or decreases TRF1 activity by verifying that the compound downregulates TRF1 protein levels.

24. The method according to claim 23, where it is verified that the compound downregulates TRF1 protein levels by:
a. adding the compound to a culture of cells,
b. quantifying protein levels in the cells subjected to the effect of the compound by a method which is selected from the group of:
i. determining total TRF1 protein levels,
ii. quantifying TRF1 foci by immunoassays, or
iii. combinations thereof;
c. comparing the data obtained with the cells subjected to the effect of the compound with the data obtained with control cells not having contacted the compound, and
d. concluding that the compound downregulates TRF1 protein levels after verifying that the TRF1 protein levels obtained with the cells subjected to the effect of the compound with the data obtained with control cells not having contacted the compound.

25. The method according to claim 23 or 24, wherein it is determined that the compound inhibits or decreases TRF1 activity by verifying that the compound downregulates TRF1 protein levels in a culture of cells of a previously established glioblastoma cell line or cells extracted from a glioblastoma patient.

26. The method according to claim 24 or 25, wherein it is additionally assessed that the compound reduces proliferation of the cells having contacted the compound.

27. The method according to any one of claims 24 to 26, wherein it is additionally assessed that the compound induces DNA damage.

28. The method according to any one of claims 24 to 27, wherein it is additionally verified that the compound has the ability to reduce stemness in a in a culture of cells of a previously established glioblastoma cell line or cells extracted from a glioblastoma patient.
